# EUROPEAN PATENT APPLICATION

(11) **EP 4 772 500 A1**
(43) Date of publication of application: **08.07.2026**
(21) Application number: 24860165.0
(22) Date of filing: 11.07.2024
(51) Int. Cl.: C07D 261/08, C07D 413/04, C07D 249/06, C07D 231/12, A61K 31/42, A61K 31/4192, A61K 31/415, A61P 1/16

(54) **NOVEL ISOXAZOLE COMPOUND AS TM4SF5-SPECIFIC INHIBITOR AND USE THEREOF**

(30) Priority: 31.08.2023 KR 20230115873
(71) Applicant: Seoul National University R&DB Foundation, Seoul 08826 (KR); Cha University Industry-Academic Cooperation Foundation, Pocheon-si, Gyeonggi-do 11160 (KR)
(72) Inventor: LEE, Jung Weon, Hanam-si, Gyeonggi-do 13010 (KR); SUH, Young-Ger, Seoul 06731 (KR); KIM, Hyun Su, Seongnam-si, Gyeonggi-do 13590 (KR); KIM, Soyeon, Seoul 08750 (KR); KIM, Ji Eon, Seoul, 05698, Republic of Korea (KR); SHIN, Eun-Ae, Suwon-si, Gyeonggi-do 16235 (KR); LEE, Haesong, Gwangmyeong-si, Gyeonggi-do 14278 (KR); KIM, Wonsik, Gwangju-si, Gyeonggi-do 12789 (KR); KIM, Eunmi, Seoul 06272 (KR); LEE, Eunhae, Incheon, 22127, Republic of Korea (KR); KIM, Taewoo, Seoul 01821 (KR)
(74) Representative: Dehns
(86) International application number: PCT/KR2024/009937
(87) International publication number: WO 2025/048243

(57) **Abstract**

The present invention relates to a novel isoxazole compound as a TM4SF5-specific inhibitor and a use thereof and, more specifically, to: a novel isoxazole compound, a pharmaceutically acceptable salt thereof, or a pharmaceutical composition comprising the compound, wherein the isoxazole compound inhibits immune checkpoint functions by downregulating the expression of TM4SF5 protein, suppressing protein-protein binding, inhibiting signal transduction activation, or activating the killing activity of T and NK immune cells or controlling the distribution of the immune cells. Specifically, with the ability to inhibit immune checkpoints of NK cells, the TM4SF5-specific isoxazole compound of the present invention can be advantageously used as an inhibitor against liver diseases or liver cancer.

## Description

### TECHNICAL FIELD

The present invention relates to novel isoxazole compounds as TM4SF5-specific inhibitors and uses thereof, and more specifically to novel isoxazole compounds having efficacy in regulating expression of the TM4SF5 protein, protein-protein interactions, signal transduction activation, or the activation and distribution of T and NK immune cells, pharmaceutically acceptable salts thereof, or pharmaceutical compositions comprising the same.

### BACKGROUND ART

Inflammation occurring in the human liver may be regarded as both a cause and a characteristic of various chronic liver diseases. Inflammatory responses accompanying chronic injury and cell death of hepatocytes caused by alcohol, viral infection, toxin accumulation, and abnormal metabolic function play an important role in the additional induction and aggravation of liver diseases. In a normal liver, non-alcoholic fatty liver resulting from abnormalities in metabolism is accompanied by inflammation due to cellular injury and cell death, progresses to steatohepatitis, and may further progress to fibrosis and cirrhosis characterized by accumulation of extracellular matrix and abnormal cellular proliferation. Furthermore, depending on changes in and interactions within the metabolic-inflammatory environment resulting from interactions among epithelial cells and immune cells including macrophages, T cells, and NK (natural killer) cells in the liver, carcinogenesis of fibrotic/cirrhotic liver tissue may be suppressed or promoted.

Chronic liver diseases and liver cancer, ranging from non-alcoholic fatty liver (NAFL), non-alcoholic steatohepatitis (NASH), liver fibrosis, liver cirrhosis, to liver cancer, are induced and aggravated by abnormalities in metabolic activity and the inflammatory immune system. Patients with non-alcoholic fatty liver disease (NAFLD), which affects up to 30% of the global population, lack clearly identified diagnostic biomarkers and approved therapeutic agents, and when progression to liver cancer occurs, the disease is often diagnosed at an advanced stage. Moreover, therapeutic agents for liver cancer have not yet been sufficiently developed, and it has been confirmed that immune checkpoint inhibitors, which show limited efficacy in solid tumors, may paradoxically exert adverse effects in liver cancer (developed via NASH) (Nature, 2021, Vol. 592:450). Recently, combination therapies of immune checkpoint inhibitors with angiogenesis inhibitors have been attempted; however, their clinical efficacy remains uncertain. Accordingly, there is a need to identify novel paradigm targets that play critical roles in the progression from NASH to liver cancer and to develop inhibitors thereof, and the importance of T- or NK-cell immune checkpoint inhibitors has been increasingly emphasized. In this context, the development of novel TM4SF5-specific inhibitors is considered important.

Transmembrane 4 L six family member 5 (TM4SF5) is a tetraspanin that spans the cell membrane four times, and its expression has been confirmed to be increased in hepatocytes and macrophages by the action of various cytokines/chemokines associated with chronic hepatocellular injury. Accordingly, interactions among hepatocytes, macrophages, T cells, and NK cells can remodel the inflammatory response and immune environment of the liver in a TM4SF5-expression-dependent manner, thereby inducing fibrosis/cirrhosis beyond steatohepatitis, and have been confirmed to enhance tumorigenesis by regulating NK cell function. Ultimately, at the stage when carcinogenesis occurs, TM4SF5 expression in hepatocytes enables immune escape processes that suppress the anticancer activity of immune cells including T cells and NK (natural killer) cells, thereby increasing effective carcinogenesis or cancer incidence.

Therefore, if small molecule synthetic compounds or antibodies are used as inhibitors that suppress expression of TM4SF5 or regulate various functions of TM4SF5, it would be possible to reduce the onset of liver diseases /liver cancer through abnormal proliferation/survival/migration functions of hepatocytes, to suppress and regulate progression to steatohepatitis, fibrosis/cirrhosis by inhibiting interactions between hepatocytes and immune cells in liver tissue, and ultimately to suppress and eliminate immune evasion of anticancer immunity mediated by T and NK cells, thereby enabling immune checkpoint inhibition for liver cancer.

The inventors of the present invention confirmed that novel isoxazole compounds can be utilized as TM4SF5-specific inhibitors and completed the present invention based thereon.

### DISCLOSURE

### TECHNICAL PROBLEM

An object of the present invention is to provide novel isoxazole compounds that inhibit efficacy in regulating the expression of the TM4SF5 protein, protein-protein interactions, signal transduction activation, or the activation and distribution of T and NK immune cells.

Another object of the present invention is to provide a pharmaceutical composition for preventing or treating a liver disease, comprising the compound or the pharmaceutically acceptable salt thereof.

Another object of the present invention is to provide a pharmaceutical formulation comprising the pharmaceutical composition.

Another object of the present invention is to provide a method for inhibiting expression, protein-protein binding, or signal transduction activation of TM4SF5 protein, or for inhibiting immune checkpoint function by activating a killing activity or controlling the distribution of T and NK immune cells in a subject or a cell, comprising administering to the subject a pharmaceutically effective amount of the compound.

Another object of the present invention is to provide use of the compound or a pharmaceutically acceptable salt thereof for preventing or treating liver disease.

### TECHNICAL SOLUTION

The present invention provides a compound represented by Chemical Formula (1) below, a pharmaceutically acceptable salt thereof, a hydrate thereof or a solvate thereof:

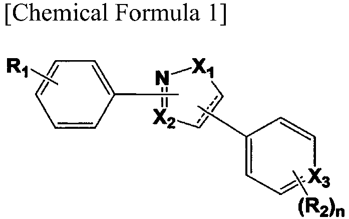

wherein in the Chemical Formula 1,
X₁ is O or N;
X₂ is C or N;
X₃ is C or N;
- - - - - - is single bond or double bond;
R₁ is hydrogen, RₐNH- or RₐO-;
Rₐ is hydrogen, C₁₋₅ alkyl, R_{b}SO₂- or R_{b}CO-;
each R_{b} is independently hydrogen, C₁₋₅ alkyl, C₂₋₅ alkenyl or C₆₋₁₀ aryl unsubstituted or substituted with C₁₋₅ alkyl;
R₂ is hydrogen, halogen, C₁₋₅ alkyl, C₁₋₆ haloalkyl, R_{c}NH- or R_{c}O-;
R_{c} is hydrogen, C₁₋₅ alkyl, RaSO₂- or R_{d}CO-;
each Rₐ is independently hydrogen, C₁₋₅ alkyl or C₆₋₁₀ aryl unsubstituted or substituted with C₁₋₅ alkyl; and
n is 1 or 2.

(When X₁ and X₂ in Chemical Formula 1 are N, the compound of Chemical Formula 1 is represented by Chemical Formula 3.)

The present invention also provides a pharmaceutical composition for preventing or treating liver disease, comprising the compound or the pharmaceutically acceptable salt thereof.

The present invention also provides a pharmaceutical composition for preventing or treating liver disease, comprising the compound or the pharmaceutically acceptable salt thereof.

The present invention also provides a method for inhibiting expression, protein-protein binding, or signal transduction activation of TM4SF5 protein, or for inhibiting immune checkpoint function by activating a killing activity or controlling the distribution of T and NK immune cells in a subject or a cell, comprising administering to the subject a pharmaceutically effective amount of the compound.

The present invention also provides use of the compound or a pharmaceutically acceptable salt thereof for preventing or treating liver disease.

### ADVANTAGEOUS EFFECTS

The isoxazole compound of Chemical Formula 1 in the present invention exhibits excellent efficacy in regulating expression of the TM4SF5 protein, inhibiting protein-protein interactions, inhibiting signal transduction activation, or controlling activation and distribution of T and NK immune cells, and thus can be usefully employed as a therapeutic agent for liver diseases.

### DESCRIPTION OF THE DRAWINGS

FIG. 1 shows a synthetic scheme of Example 32, a representative compound among the isoxazole compounds.
FIGS. 2a and 2b show results of inhibition of TM4SF5-expression dependent phosphorylation of signaling molecules by the isoxazole compounds.
FIGS. 3a to 3c show results of inhibition of TM4SF5-expression dependent two-dimensional and three-dimensional cell growth/proliferation by the isoxazole compounds.
FIG. 4 shows results of inhibition of binding between TM4SF5 and various cell membrane receptors by treatment with the isoxazole compounds.
FIGS. 5a to 5c show results of inhibition of TM4SF5-expression dependent phosphorylation of signaling molecules and three-dimensional sphere proliferation by the isoxazole compounds.
FIG. 6 shows results of inhibition of binding between TM4SF5 and various cell membrane receptors and signaling molecules by treatment with the isoxazole compounds.
FIG. 7 shows results of inhibition of TM4SF5-expression dependent cell migration by the isoxazole compounds.
FIGS. 8a to 8i show results of inhibition of TM4SF5-expression dependent NASH induction by the isoxazole compounds.
FIGS. 9a to 9c show results of inhibition of TM4SF5-expression dependent phosphorylation of signaling molecules by the isoxazole compounds.
FIGS. 10a to 10c show results of inhibition of TM4SF5-expression dependent three-dimensional cell growth/proliferation by the isoxazole compounds.
FIGS. 11a to 11e show results of inhibition of TM4SF5-expression dependent xenograft liver cancer formation by the isoxazole compounds.
FIGS. 12a to 12d show results of inhibition of TM4SF5-expression dependent phosphorylation of signaling molecules and three-dimensional cell growth/proliferation by the isoxazole compounds.
FIGS. 13a to 13j show results of reduced expression of ligands associated with natural cytotoxic function of NK cell and inhibition of binding to TM4SF5 in a TM4SF5 expression dependent manner by the isoxazole compounds.
FIGS. 14a to 14d show results demonstrating reduced expression of Slamf7s associated with natural cytotoxic function of NK cell in a TM4SF5 expression dependent manner and the importance of N-glycosylation of both proteins in binding to TM4SF5.
FIGS. 15a to 15h show results of reduced expression, degradation, and intracellular localization regulation of ligands associated with natural cytotoxic function of NK cell in a TM4SF5 expression dependent manner by the isoxazole compounds.
FIGS. 16a to 16g show results of reduction of natural cytotoxic function of NK cell induced by TM4SF5 expression.
FIGS. 17a to 17d show results of inhibition of expression and binding of PD-L1, which induces immune evasion of T and NK cells in a TM4SF5 expression dependent manner, by the isoxazole compounds.
FIGS. 18a and 18b show results of inhibition of PD-1 expression and suppression of T-cell activation in a TM4SF5 expression dependent manner by the isoxazole compounds.
FIGS. 19a to 19e show results of inhibition of cholesterol-binding ability of TM4SF5 and PD-L1 by binding of the isoxazole compounds to TM4SF5 and treatment with the isoxazole.
FIGS. 20a to 20d show results of inhibition of liver cancer formation and expression of liver cancer markers in a liver-orthotopic xenograft model using TM4SF5 expressing cell lines by isoxazole compounds.
FIGS. 21a to 21e show results of inhibition of liver cancer formation and expression of liver cancer markers in a TM4SF5-expressing PDX model by the isoxazole compounds.
FIGS. 22a to 22e show results of inhibition of liver cancer formation, regulation of expression of ligands associated with natural cytotoxic function of NK cell, and confirmation of NK cell infiltration into tumor tissue in a liver-orthotopic xenograft model using TM4SF5-expressing cell lines by the isoxazole compounds.
FIGS. 23a to 23c show results of inhibition of liver cancer formation induced by DEN treatment and regulation of expression of ligands associated with natural cytotoxic function of NK cell in TM4SF5 genetically modified animals by the isoxazole compounds.
FIGS. 24a to 24d show results of inhibition of liver cancer formation and regulation of expression of ligands associated with natural cytotoxic function of NK cell in cancer cells in a subcutaneous xenograft liver cancer model using severe combined immunodeficient animals (NOD-SCID mouse) by the isoxazole compounds.

### MODES OF THE INVENTION

The inventors of the present invention confirmed that, in TM4SF5 expression-dependent cell line models and animal models, signal transduction activity and binding functions with membrane receptors increased due to TM4SF5 expression, TM4SF5 expression-dependent cell survival/proliferation and migration in two-dimensional and three-dimensional environments, and the development of liver diseases including steatohepatitis (NASH-fibrosis) and liver cancer were inhibited (in particular, two compounds, ST-5-001 and ST-5-002, exhibited effects at concentrations of 1 µM or less or at doses of 2.5 mpk or less). In particular, the inventors previously reported that TM4SF5 can reduce the cytotoxic function of NK immune cells (Korean Patent Application No. 10-2021-0171614 and PCT Application No. PCT/KR2021/018197), and the novel isoxazole compounds of the present invention exhibited a mechanism of action capable of inhibiting such functional impairment of NK cells caused by TM4SF5. In addition, PD-L1 immune checkpoints that induce immune evasion during liver cancer formation are expressed and function not only in NK cells but also in T cells, and thus it can be interpreted that the novel isoxazole compounds of the present invention exhibit a mechanism of action capable of inhibiting functional impairment of T cells caused by TM4SF5. Furthermore, TM4SF5 expressed in liver cancer cells binds to ligands such as SLAMF7 that induce activation of NK cells, thereby inducing translocation to lysosome and degradation of the ligands and suppressing NK cell activation and natural cytotoxic efficacy, whereas isoxazole compounds acting as TM4SF5-specific inhibitors inhibit such degradative functions, thereby enhancing NK cell activation and natural cytotoxic efficacy. In addition, the isoxazole compounds are capable of reversing both (i) an increase in PD-L1 expression and stabilization through direct binding mediated by TM4SF5 and (ii) induction of PD-1 expression in T cells, which suppresses T-cell activation and cancer cell killing functions.

Accordingly, the present invention provides novel isoxazole compounds acting as TM4SF5-specific inhibitors, which are capable of inhibiting the onset of liver diseases and liver cancer and interrupting progression among said diseases, and which have potential utility as anti-liver cancer agents based on efficacy as immune checkpoint inhibitors for T cells and NK cells present in the liver.

In the present invention, the term "TM4SF5 protein" refers to Transmembrane 4 L six family member 5 (also referred to as Four Transmembrane L6 Superfamily member 5 or "L6H"), which is a member of a group of membrane receptors that span the cell membrane four times, including tetraspanin, tetraspan, or Transmembrane 4 Superfamily (TM4SF). The TM4SF (Transmembrane 4 Superfamily) proteins share similar structural features in that they each span the cell membrane four times and biochemically comprise four hydrophobic regions presumed to be transmembrane domains. Among these, the Four-Transmembrane L6 Superfamily (including L6, TM4SF5, L6D, and IL-TMP), which includes TM4SF5, has biochemical functions that have not been well characterized among tetraspanins.

The TM4SF5 is a hydrophobic protein having four transmembrane domains that pass through the cell membrane, two extracellular loop structures, and two cytoplasmic terminal structures, and is known to be highly expressed in various cancer cells, including pancreatic cancer, lung cancer, gastric cancer, prostate cancer, breast cancer, esophageal cancer, colorectal cancer, and liver cancer (Muller-Pillasch, F., et al., Gene 208:25, 1998; Pascual-Le Tallec, L. et al., J. Clin. Endocrinol. Metab. 87:501, 2002).

In the present invention, the term "liver disease" refers to any disease that causes impairment of liver function, and may be induced, for example, by viruses (e.g., hepatitis A, B, C, D, or E viruses), alcohol, aflatoxin, drugs (such as antituberculosis agents, aspirin, antibiotics, anesthetics, antihypertensive agents, oral contraceptives, and the like), or congenital metabolic abnormalities. Specific examples of liver diseases include chronic liver injury, non-alcoholic or alcoholic liver disease or fatty liver disease, hepatitis, liver fibrosis, liver cirrhosis, and liver cancer. The liver disease is preferably liver cancer, fatty liver, hepatitis, liver fibrosis, or liver cirrhosis, but is not limited thereto.

In the present invention, the term "halogen" refers to fluorine, chlorine, bromine, or iodine, unless otherwise stated, and specifically refers to fluorine, but is not limited thereto.

In the present invention, the term "alkyl" refers to a saturated, linear or branched monovalent hydrocarbon radical, unless otherwise stated.

In the present invention, the term "alkenyl" refers to a monovalent hydrocarbon radical containing at least one carbon-carbon double bond, unless otherwise stated, and each double bond may have an E- or Z-stereochemical configuration.

Such alkyl and alkenyl groups may be linear, that is, straight-chain, or branched. According to each definition, the number of carbon atoms in the alkyl group may be 1, 2, 3, 4, 5, or 6, or may be 1, 2, 3, or 4. Examples of alkyl groups include methyl, ethyl, propyl including n-propyl and isopropyl, butyl including n-butyl, sec-butyl, isobutyl, and tert-butyl, pentyl including n-pentyl, 1-methylbutyl, isopentyl, neopentyl, and tert-pentyl, and hexyl including n-hexyl, 3,3-dimethylbutyl, and isohexyl. The double bond of the alkenyl group may be present at any position.

In the present invention, the term "aryl" refers to an aromatic group that may be substituted or unsubstituted, unless otherwise stated, and includes, without limitation, phenyl, biphenyl, naphthyl, toluyl, naphthalenyl, anthracenyl, or all possible isomers thereof.

In the present invention, a numerical range expressed using the term "to" refers to a range that includes the numerical values stated before and after the term "to" as the lower limit and the upper limit, respectively.

In one embodiment, the compound of Formula 1 may exist in a solvate form. As used herein, the term "solvate" refers to a molecular complex comprising the compound and one or more pharmaceutically acceptable solvent molecules, for example, ethanol or water. A complex in which the solvent molecule is water is also referred to as a "hydrate."

In one embodiment, the compound of Formula 1 and solvates thereof may exist in the form of a pharmaceutically acceptable salt.

In the present invention, the term "pharmaceutically acceptable salt" refers to a salt that has low toxicity to the human body and does not adversely affect the biological activity or physicochemical properties of the parent compound. Such pharmaceutically acceptable salts include, but are not limited to, acid addition salts formed from pharmaceutically acceptable free acids and a basic compound of Formula 1; alkali metal salts (e.g., sodium salts) and alkaline earth metal salts (e.g., calcium salts); organic base addition salts formed between an organic base and a carboxylic acid moiety of Formula 1; and amino acid addition salts.

Preferred forms of the salts of the compound in the present invention include salts with inorganic acids or organic acids. Examples of inorganic acids include hydrochloric acid, sulfuric acid, nitric acid, phosphoric acid, perchloric acid, and hydrobromic acid. Examples of organic acids include acetic acid, methanesulfonic acid, ethanesulfonic acid, p-toluenesulfonic acid, fumaric acid, maleic acid, malonic acid, phthalic acid, succinic acid, lactic acid, citric acid, citrate, gluconic acid, tartaric acid, salicylic acid, malic acid, oxalic acid, benzoic acid, embonic acid, aspartic acid, and glutamic acid. Examples of organic bases that may be used to prepare organic base addition salts include tris(hydroxymethyl)methylamine and dicyclohexylamine. Examples of amino acids that may be used to prepare amino acid addition salts include natural amino acids such as alanine and glycine. It will be apparent to those skilled in the art that acids or bases other than the inorganic acids, organic acids, organic bases, and amino acids exemplified above may also be used.

The salts may be prepared by conventional methods. For example, the compound of Formula 1 may be dissolved in a water-miscible solvent such as methanol, ethanol, acetone, or 1,4-dioxane, followed by addition of a free acid or a free base and subsequent crystallization to obtain the salt.

The details of the method for prevention or treatment described herein may be applied to the above description of the pharmaceutical composition according to one embodiment of the present invention.

In the present invention, the term "treatment" refers to treatment, improvement, amelioration, or management of a disease.

In the present invention, the term "prevention" refers to preventing a disease, for example, preventing a disease, condition, or disorder in a subject who may be predisposed to the disease, condition, or disorder but has not yet experienced or exhibited the pathology or symptoms thereof.

In the present invention, the term "administration" refers to providing a predetermined amount of a composition of the present invention to a subject by any suitable method.

In the present invention, the term "subject" or "patient" refers to any animal, including mammals such as Caenorhabditis elegans, Drosophila melanogaster, zebrafish, mice, rats, other rodents, rabbits, dogs, cats, pigs, cattle, sheep, horses, primates, and humans.

Hereinafter, the present invention will be described in detail.

In one embodiment, the novel isoxazole compound may be represented by Chemical Formula 1 below.

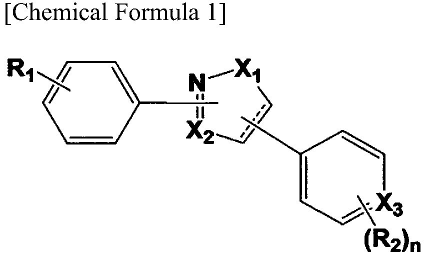

A compound represented by Chemical Formula 1 below, a pharmaceutically acceptable salt thereof, a hydrate thereof or a solvate thereof:
wherein in the Chemical Formula 1,
X₁ is O or N;
X₂ is C or N;
X₃ is C or N;
- - - - - - is single bond or double bond, preferably;
R₁ is hydrogen, RₐNH- or RₐO-, preferably an amine, acetamide, methanesulfonamide, methylbenzenesulfonamide, methylbenzamide, acrylamide, methoxy, or hydroxy;
Rₐ is hydrogen, C₁₋₅ alkyl, R₈SO₂- or R_{b}CO-;
each R_{b} is independently hydrogen, C₁₋₅ alkyl, C₂₋₅ alkenyl or C₆₋₁₀ aryl unsubstituted or substituted with C₁₋₅ alkyl;
R₂ is hydrogen, halogen, C₁₋₅ alkyl, C₁₋₆ haloalkyl, R_{c}NH- or R_{c}O-, preferably methoxy, propoxy, t-butyl, fluoro, trifluoromethyl, hydroxy, methylbenzenesulfonate, acetamide, or methylbenzamide.;
R_{c} is hydrogen, C₁₋₅ alkyl, RaSO₂- or R₄CO-;
each Rₐ is independently hydrogen, C₁₋₅ alkyl or C₆₋₁₀ aryl unsubstituted or substituted with C₁₋₅ alkyl; and
n is 1 or 2.

(When X₁ and X₂ in Chemical Formula 1 are N, the compound of Chemical Formula 1 is represented by Chemical Formula 3.)

In another embodiment, the compound represented by Chemical Formula 1 is represented by any one of Chemical Formulas 2 to 4 below:

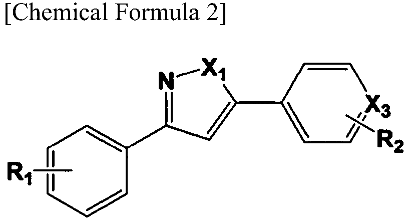

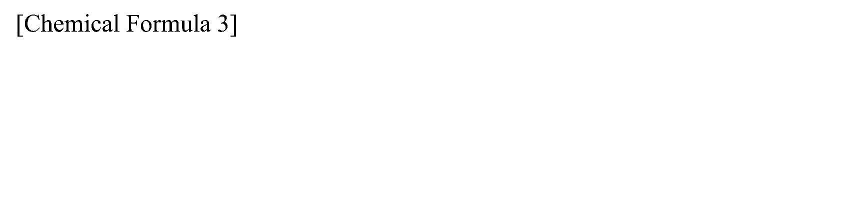

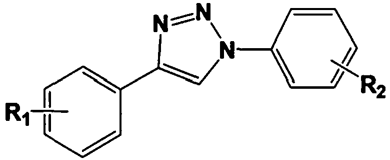

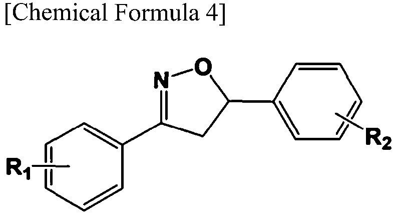

wherein in the Chemical Formulas 2 to 4,
X₁, X₃, R₁, Rₐ, R_{b}, R₂, R_{c}, R_{d} and n are as defined in Chemical Formula 1 of claim 1.

In addition, preferred examples of the compound of Chemical Formula 1 according to the present invention are as follows, but are not limited thereto.
4-(5-(4-methoxyphenyl)isoxazole-3-yl)aniline;
4-(5-(3-methoxyphenyl)isoxazole-3-yl)aniline;
4-(5-(2-methoxyphenyl)isoxazole-3-yl)aniline;
4-(5-(3,4-dimethoxyphenyl)isoxazole-3-yl)aniline;
4-(5-(4-(tert-butyl)phenyl)isoxazole-3-yl)aniline;
4-(5-(4-fluorophenyl)isoxazole-3-yl)aniline;
4-(5-(4-(trifluoromethyl)phenyl)isoxazole-3-yl)aniline;
4-(5-(4-propoxyphenyl)isoxazole-3-yl)aniline;
3-(5-(4-methoxyphenyl)isoxazole-3-yl)aniline;
*N*-(4-(5-(4-methoxyphenyl)isoxazole-3-yl)phenyl)acetamide;
*N*-(4-(5-(4-methoxyphenyl)isoxazole-3-yl)phenyl)methanesulfonamide;
*N*-(4-(5-(4-methoxyphenyl)isoxazole-3-yl)phenyl)-4-methylbenzenesulfonamide;
*N*-(4-(5-(4-hydroxyphenyl)isoxazole-3-yl)phenyl)-4-methylbenzenesulfonamide;
*N*-(4-(5-(4-methoxyphenyl)isoxazole-3-yl)phenyl)-4-methylbenzamide;
*N*-(4-(5-(4-methoxyphenyl)isoxazole-3-yl)phenyl)acrylamide;
*N*-(4-(5-(3-methoxyphenyl)isoxazole-3-yl)phenyl)acetamide;
*N*-(4-(5-(2-methoxyphenyl)isoxazole-3-yl)phenyl)acetamide;
*N*-(4-(5-(3,4-dimethoxyphenyl)isoxazole-3-yl)phenyl)acetamide;
*N*-(4-(5-(4-propoxyphenyl)isoxazole-3-yl)phenyl)acetamide;
*N*-(4-(5-(4-(trifluoromethyl)phenyl)isoxazole-3-yl)phenyl)acetamide;
*N*-(4-(5-(4-fluorophenyl)isoxazole-3-yl)phenyl)acetamide;
*N*-(4-(5-(4-(tert-butyl)phenyl)isoxazole-3-yl)phenyl)acetamide;
*N*-(4-(5-(pyridine-4-yl)isoxazole-3-yl)phenyl)acetamide;
*N*-(3-(5-(4-methoxyphenyl)isoxazole-3-yl)phenyl)acetamide;
*N*-(3-(5-(4-methoxyphenyl)isoxazole-3-yl)phenyl)-4-methylbenzamide;
*N*-(4-(5-(4-hydroxyphenyl)isoxazole-3-yl)phenyl)acetamide;
4-(3-(4-acetamidophenyl)isoxazole-5-yl)phenyl 4-methylbenzenesulfonate;
*N*-(4-(3-(4-methoxyphenyl)isoxazole-5-yl)phenyl)acetamide;
*N*-(4-(3-(4-methoxyphenyl)isoxazole-5-yl)phenyl)-4-methylbenzenesulfonamide;
*N*-(4-(3-(4-hydroxyphenyl)isoxazole-5-yl)phenyl)-4-methylbenzenesulfonamide;
*N*-(4-(3-(4-hydroxyphenyl)isoxazole-5-yl)phenyl)acetamide;
*N*-(4-(3-(4-hydroxyphenyl)isoxazole-5-yl)phenyl)-4-methylbenzamide;
*N*-(4-(1-(4-hydroxyphenyl)-1H-1,2,3-triazole-4-yl)phenyl)-4-methylbenzenesulfonamide;
*N*-(4-(1-(4-methoxyphenyl)-1H-1,2,3-triazole-4-yl)phenyl)acetamide;
*N*-(4-(5-(4-hydroxyphenyl)-1H-pyrazol-3-yl)phenyl)-4-methylbenzenesulfonamide;
*N*-(4-(5-(4-hydroxyphenyl)-4,5-dihydroisoxazole-3-yl)phenyl)-4-methylbenzenesulfonamide; and
*N*-(4-(5-(4-methoxyphenyl)-4,5-dihydroisoxazole-3-yl)phenyl)acetamide.

In addition, preferred examples of the compound of Chemical Formula 1 according to the present invention are listed in Table 1 below.

**[Table 1]**

| example | Chemical Formula | Reference number |
|---|---|---|
| 1 | 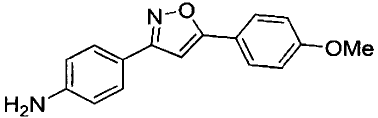 | ST-1-025 |
| | | 4-(5-(4-methoxyphenyl)isoxazole-3-yl)aniline |
| 2 | 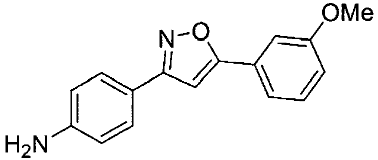 | ST-2-004 |
| | | 4-(5-(3-methoxyphenyl)isoxazole-3-yl)aniline |
| 3 | 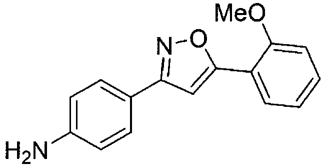 | ST-2-005 |
| | | 4-(5-(2-methoxyphenyl)isoxazole-3-yl)aniline |
| 4 | 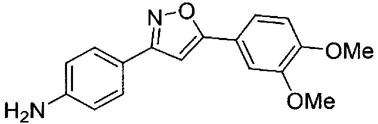 | ST-2-006 |
| | | 4-(5-(3,4-dimethoxyphenyl)isoxazole-3-yl)aniline |
| 5 | 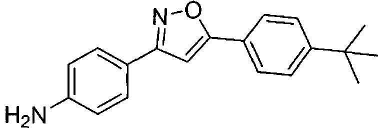 | ST-2-007 |
| | | 4-(5-(4-(tert-butyl)phenyl)isoxazole-3-yl)aniline |
| 6 | 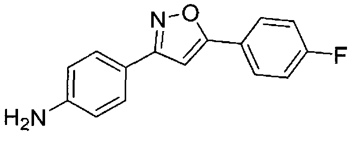 | ST-2-008 |
| | | 4-(5-(4-fluorophenyl)isoxazole-3-yl)aniline |
| 7 | 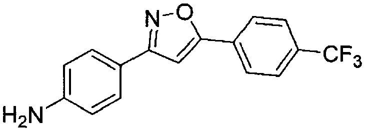 | ST-2-009 |
| | | 4-(5-(4-(trifluoromethyl)phenyl)isoxazole-3-yl)aniline |
| 8 | 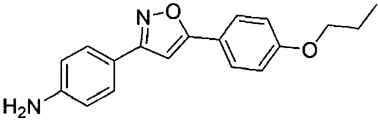 | ST-2-011 |
| | | 4-(5-(4-propoxyphenyl)isoxazole-3-yl)aniline |
| 9 | 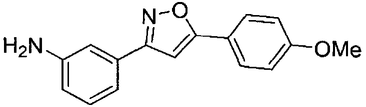 | ST-2-010 |
| | | 3-(5-(4-methoxyphenyl)isoxazole-3-yl)aniline |
| 10 | 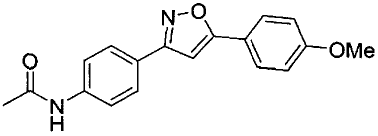 | ST-2-001 |
| | | *N*-(4-(5-(4-methoxyphenyl)isoxazole-3-yl)phenyl)acetamide |
| 11 | 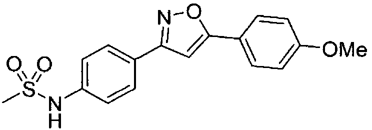 | ST-2-002 |
| | | *N*-(4-(5-(4-methoxyphenyl)isoxazole-3-yl)phenyl)methanesulfonamide |
| 12 | 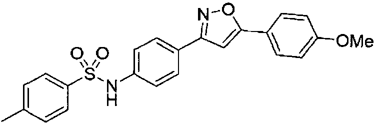 | ST-2-003 |
| | | *N*-(4-(5-(4-methoxyphenyl)isoxazole-3-yl)phenyl)-4-methylbenzenesulfonamide |
| 13 | 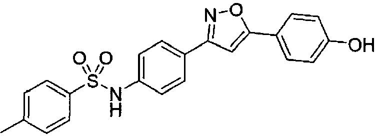 | ST-1-024 |
| | | *N*-(4-(5-(4-hydroxyphenyl)isoxazole-3-yl)phenyl)-4-methylbenzenesulfonamide |
| 14 | 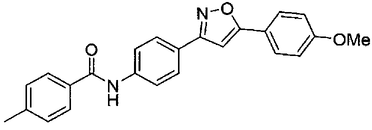 | ST-3-009 |
| | | *N*-(4-(5-(4-methoxyphenyl)isoxazole-3-yl)phenyl)-4-methylbenzamide |
| 15 | 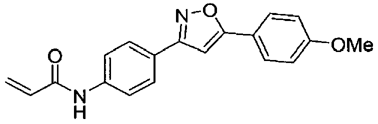 | ST-3-010 |
| | | *N*-(4-(5-(4-methoxyphenyl)isoxazole-3-yl)phenyl)acrylamide |
| 16 | 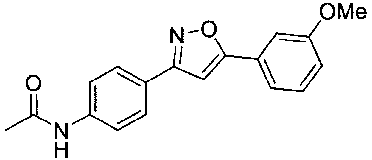 | ST-3-001 |
| | | *N*-(4-(5-(3-methoxyphenyl)isoxazole-3-yl)phenyl)acetamide |
| 17 | 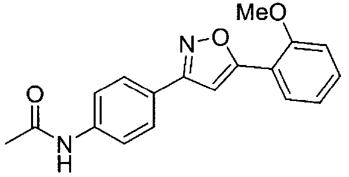 | ST-3-002 |
| | | *N*-(4-(5-(2-methoxyphenyl)isoxazole-3-yl)phenyl)acetamide |
| 18 | 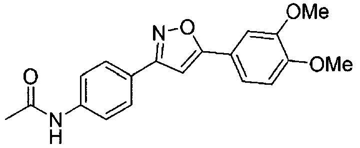 | ST-3-003 |
| | | *N*-(4-(5-(3,4-dimethoxyphenyl)isoxazole-3-yl)phenyl)acetamide |
| 19 | 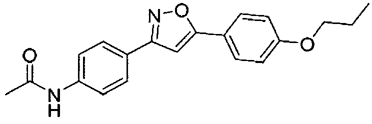 | ST-3-004 |
| | | *N*-(4-(5-(4-propoxyphenyl)isoxazole-3-yl)phenyl)acetamide |
| 20 | 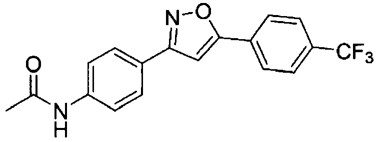 | ST-3-005 |
| | | *N*-(4-(5-(4-(trifluoromethyl)phenyl)isoxazole-3-yl)phenyl)acetamide |
| 21 | 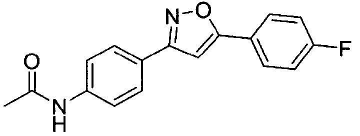 | ST-3-006 |
| | | *N*-(4-(5-(4-fluorophenyl)isoxazole-3-yl)phenyl)acetamide |
| 22 | 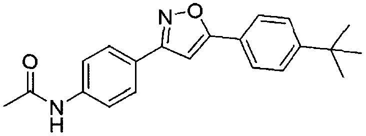 | ST-3-007 |
| | | *N*-(4-(5-(4-(tert-butyl)phenyl)isoxazole-3-yl)phenyl)acetamide |
| 23 | 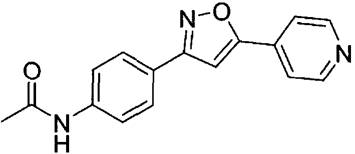 | ST-3-008 |
| | | *N*-(4-(5-(pyridine-4-yl)isoxazole-3-yl)phenyl)acetamide |
| 24 | 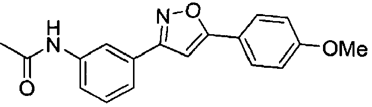 | ST-3-011 |
| | | *N*-(3-(5-(4-methoxyphenyl)isoxazole-3-yl)phenyl)acetamide |
| 25 | 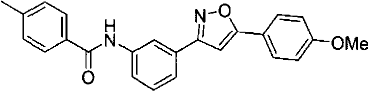 | ST-3-012 |
| | | *N*-(3-(5-(4-methoxyphenyl)isoxazole-3-yl)phenyl)-4-methylbenzamide |
| 26 | 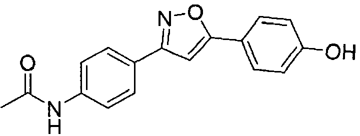 | ST-4-001 |
| | | *N*-(4-(5-(4-hydroxyphenyl)isoxazole-3-yl)phenyl)acetamide |
| 27 | 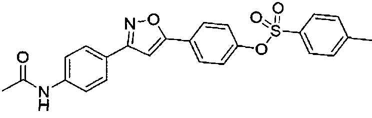 | ST-4-002 |
| | | 4-(3-(4-acetamidophenyl)isoxazole-5-yl)phenyl 4-methylbenzenesulfonate |
| 28 | 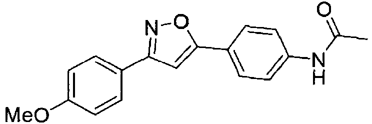 | ST-4-003 |
| | | *N*-(4-(3-(4-methoxyphenyl)isoxazole-5-yl)phenyl)acetamide |
| 29 | 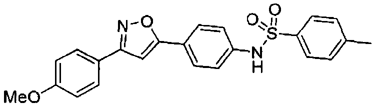 | ST-4-004 |
| | | *N*-(4-(3-(4-methoxyphenyl)isoxazole-5-yl)phenyl)-4-methylbenzenesulfonamide |
| 30 | 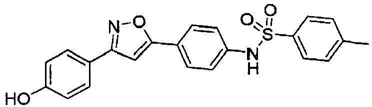 | ST-4-005 |
| | | *N*-(4-(3-(4-hydroxyphenyl)isoxazole-5-yl)phenyl)-4-methylbenzenesulfonamide |
| 31 | 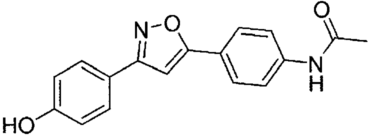 | ST-5-001 |
| | | *N*-(4-(3-(4-hydroxyphenyl)isoxazole-5-yl)phenyl)acetamide |
| 32 | 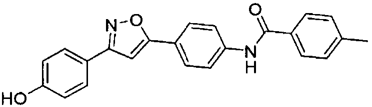 | ST-5-002 |
| | | *N*-(4-(3-(4-hydroxyphenyl)isoxazole-5-yl)phenyl)-4-methylbenzamide |
| 33 | 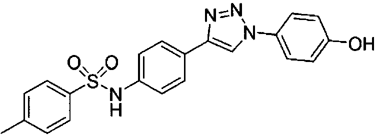 | ST-1-001 |
| | | *N*-(4-(1-(4-hydroxyphenyl)-1*H*-1,2,3-triazole-4-yl)phenyl)-4-methylbenzenesulfonamide |
| 34 | 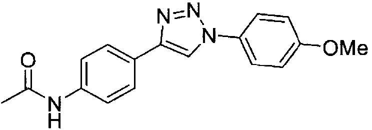 | ST-4-007 |
| | | *N*-(4-(1-(4-methoxyphenyl)-1*H*-1,2,3-triazole-4-yl)phenyl)acetamide |
| 35 | 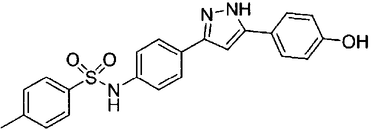 | ST-1-002 |
| | | *N*-(4-(5-(4-hydroxyphenyl)-1*H*-pyrazol-3-yl)phenyl)-4-methylbenzenesulfonamide |
| 36 | 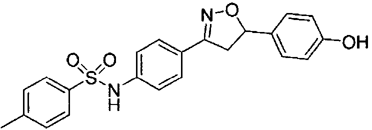 | ST-1-004 |
| | | *N*-(4-(5-(4-hydroxyphenyl)-4,5-dihydroisoxazole-3-yl)phenyl)-4-methylbenzenesulfonamide |
| 37 | 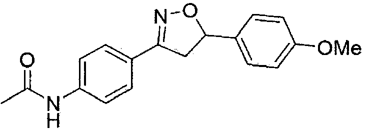 | ST-4-006 |
| | | *N*-(4-(5-(4-methoxyphenyl)-4,5-dihydroisoxazole-3-yl)phenyl)acetamide |

In another embodiment, the present invention provides a pharmaceutical composition for preventing or treating a liver disease, comprising the compound of Chemical Formula 1 and the pharmaceutically acceptable salt thereof.

The liver disease is liver cancer, chronic liver injury, non-alcoholic or alcoholic liver disease or fatty liver disease, hepatitis, liver fibrosis, or liver cirrhosis.

The pharmaceutical composition is for use in treating liver disease by inhibiting expression, protein-protein binding, or signal transduction activation of TM4SF5 protein, or by inhibiting immune checkpoint function by activating a killing activity or controlling the distribution of T and NK immune cells.

In another embodiment, the present invention provides a pharmaceutical formulation comprising the pharmaceutical composition.

The pharmaceutical formulation of the present invention is in the form of a patch, ointment, inhalant, nasal drop, tablet, pill, powder, capsule, syrup, or emulsion.

In addition, the pharmaceutical formulation may be formulated according to conventional methods by adding, in addition to the active ingredient, one or more commonly used, non-toxic, pharmaceutically acceptable additives selected from the group consisting of carriers, adjuvants, and excipients.

Excipients that may be used in the pharmaceutical formulation of the present invention include sweeteners, binders, solubilizers, solubilizing aids, wetting agents, emulsifiers, isotonic agents, adsorbents, disintegrants, antioxidants, preservatives, lubricants, fillers, and flavoring agents, but not limited thereto. For example, excipients include lactose, dextrose, sucrose, mannitol, sorbitol, cellulose, glycine, silica, magnesium aluminum silicate, starch, gelatin, tragacanth gum, alginic acid, sodium alginate, methylcellulose, sodium carboxymethylcellulose, water, ethanol, polyethylene glycol, polyvinylpyrrolidone, sodium chloride, calcium chloride, orange essence, strawberry essence, and vanilla flavor.

When the pharmaceutical formulation of the present invention is in an oral dosage form, examples of carriers include cellulose, calcium silicate, corn starch, lactose, sucrose, dextrose, calcium phosphate, stearic acid, magnesium stearate, calcium stearate, gelatin, and talc, but are not limited thereto.

When the pharmaceutical formulation of the present invention is in an injectable form, examples of carriers include water, saline, aqueous glucose solution, aqueous sugar solutions, alcohols, glycols, ethers, oils, fatty acids, fatty acid esters, and glycerides, but are not limited thereto.

For use of the compound according to the present invention as a medicament, the compound is formulated into a pharmaceutical formulation, which contains, in addition to the active ingredient for oral or parenteral administration, suitable pharmaceutically acceptable organic or inorganic inert carrier materials, such as water, gelatin, gum arabic, lactose, starch, vegetable oils, and polyalkylene glycols. The pharmaceutical formulation may be present in a solid form, such as tablets, sugar-coated tablets, suppositories, or capsules, or in a liquid form, such as solutions, suspensions, or emulsions. In addition, the pharmaceutical formulation may optionally contain auxiliary agents, such as preservatives, stabilizers, wetting agents, or emulsifiers; as well as salts or buffering agents for adjusting osmotic pressure.

For parenteral administration, injectable solutions or suspensions are preferred.

As a carrier system, surface-active auxiliary agents, such as bile salts or animal- or plant-derived phospholipids, as well as mixtures thereof, and liposomes or components thereof, may also be used.

For oral administration, tablets, sugar-coated tablets, or capsules containing, in particular, talc and/or a hydrocarbon vehicle or binders, such as lactose, corn starch, or potato starch, are suitable. In addition, liquid dosage forms, for example, administration in juice to which a sweetener has been added, may be used.

In another embodiment, the present invention provides a method for inhibiting expression, protein-protein binding, or signal transduction activation of TM4SF5 protein, or for inhibiting immune checkpoint function by activating a killing activity or controlling the distribution of T and NK immune cells in a subject or a cell, comprising administering to the subject a pharmaceutically effective amount of the compound of Chemical Formula 1.

In another embodiment, the present invention provides a method for preventing or treating liver disease in a subject, comprising administering to the subject a pharmaceutically effective amount of the compound of Chemical Formula 1. Preferably, the subject refers to individual or a patient, but is not limited thereto.

In another embodiment, the present invention provides use of the compound of Chemical Formula 1 or a pharmaceutically acceptable salt thereof for preventing or treating liver disease.

Hereinafter, the present invention will be described in more detail through preparation examples and experimental examples. However, the following preparation examples and experimental examples are intended to illustrate the present invention, and the scope of the present invention is not limited thereto.

### Preparation Example 1: preparation of 4-(5-(4-methoxyphenyl)isoxazole-3-yl)aniline

### Step 1) Preparation of tert-butyl (E)-(4-((hydroxyimino)methyl)phenyl)carbamate

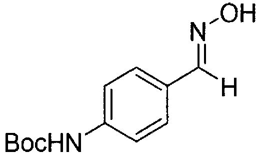

tert-Butyl (4-formylphenyl)carbamate (3.00 g, 13.56 mmol), sodium acetate (3.34 g, 40.67 mmol), and hydroxylamine hydrochloride (2.83 g, 40.67 mmol) were dissolved in ethanol (50 mL) and stirred at room temperature for 3 hours. After completion of the reaction, water was added dropwise. The reaction mixture was extracted three times with ethyl acetate, dried over anhydrous magnesium sulfate, and concentrated under reduced pressure. The resulting residue was purified by flash column chromatography (ethyl acetate:n-hexane = 1:4), and the resulting solution was concentrated to obtain the target compound (2.10 g) in 66% yield.

¹H NMR (DMSO-d6, 800 MHz) δ 10.98 (s, 1H), 9.50 (s, 1H), 8.02 (s, 1H), 7.48-7.45 (m, 4H), 1.47 (s, 9H)

### Step 2) tert-Butyl (4-(5-(4-methoxyphenyl)isoxazol-3-yl)phenyl)carbamate

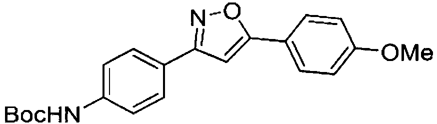

tert-Butyl (E)-(4-((hydroxyimino)methyl)phenyl)carbamate (750 mg, 3.17 mmol) prepared in Step 1) and 4-ethynylanisole (286 mg, 2.12 mmol) were dissolved in 1,4-dioxane:water (5:1, 36 mL), and [bis(trifluoroacetoxy)iodo]benzene (1.82 g, 4.24 mmol) was added dropwise. The reaction mixture was stirred at room temperature for 3 hours. After completion of the reaction, water was added dropwise. The mixture was extracted three times with ethyl acetate, dried over anhydrous magnesium sulfate, and concentrated under reduced pressure. The resulting residue was purified by flash column chromatography (ethyl acetate:n-hexane = 1:3), and the resulting solution was concentrated to obtain the target compound (650 mg) in 82% yield.

¹H NMR (DMSO-d6, 800 MHz) δ 9.62 (s, 1H), 7.84 (d, J = 8.8 Hz, 2H), 7.79 (d, J = 8.7 Hz, 2H), 7.61 (d, J = 8.4 Hz, 2H), 7.37 (s, 1H), 7.12 (d, J = 8.8 Hz, 2H), 3.84 (s, 3H), 1.49 (s, 9H)

### Step 3) Preparation of 4-(5-(4-methoxyphenyl)isoxazol-3-yl)aniline

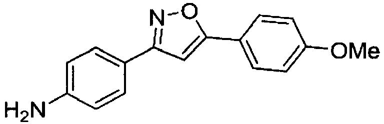

tert-Butyl (4-(5-(4-methoxyphenyl)isoxazol-3-yl)phenyl)carbamate (310 mg, 0.85 mmol), prepared in step 2), was dissolved in dichloromethane (15 mL), and trifluoroacetic acid (0.65 mL, 8.46 mmol) was added dropwise. The reaction mixture was stirred at room temperature for 3 hours. After completion of the reaction, the mixture was concentrated under reduced pressure. The resulting residue was dissolved in dichloromethane, and a saturated aqueous sodium bicarbonate solution was added. The mixture was extracted with dichloromethane three times, dried over anhydrous magnesium sulfate, and concentrated under reduced pressure. The resulting residue was purified by flash column chromatography (ethyl acetate:n-hexane = 1:2), and the resulting solution was concentrated to obtain the target compound (215 mg, 96% yield).

¹H NMR (DMSO-d6, 800 MHz) δ 7.81 (d, J = 8.8 Hz, 2H), 7.55 (d, J = 8.6 Hz, 2H), 7.23 (s, 1H), 7.10 (d, J = 8.9 Hz, 2H), 6.65 (d, J = 8.6 Hz, 2H), 5.56 (s, 2H), 3.83 (s, 3H)

### Preparation Example 2: preparation of 4-(5-(3-methoxyphenyl)isoxazole-3-yl)aniline

### Step 1) Preparation of tert-Butyl (4-(5-(3-methoxyphenyl)isoxazol-3-yl)phenyl)carbamate

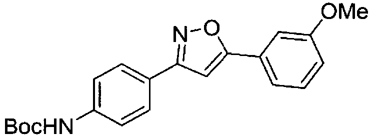

The procedure of Preparation Example 1, Step 2) was repeated to obtain the target compound (160 mg, 62% yield), except that 1-ethynyl-3-methoxybenzene (93 mg, 0.71 mmol) was used instead of 4-ethynylanisole.

¹H NMR (DMSO-d₆, 800 MHz) δ 9.63 (s, 1H), 7.80 (d, J = 8.7 Hz, 2H), 7.62 (d, J = 8.5 Hz, 2H), 7.56 (s, 1H), 7.49-7.47 (m, 2H), 7.44-7.43 (m, 1H), 7.11-7.08 (m, 1H), 3.86 (s, 3H), 1.50 (s, 9H)

### Step 2) Preparation of 4-(5-(3-methoxyphenyl)isoxazole-3-yl)aniline

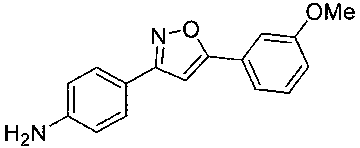

The procedure of Preparation Example 1, Step 3) was repeated to obtain the target compound (64 mg, 88% yield), except that tert-butyl (4-(5-(3-methoxyphenyl)isoxazol-3-yl)phenyl)carbamate (100 mg, 0.27 mmol) was used instead of tert-butyl (4-(5-(4-methoxyphenyl)isoxazol-3-yl)phenyl)carbamate.

¹H NMR (DMSO-d₆, 800 MHz) δ 7.57 (d, J = 8.5 Hz, 2H), 7.48-7.44 (m, 2H), 7.42 (s, 1H), 7.41-7.40 (m, 1H), 7.08 (ddd, J = 7.6, 2.5, 1.9 Hz, 1H), 6.65 (d, J = 8.5 Hz, 2H), 5.57 (s, 2H), 3.85 (s, 3H)

### Preparation Example 3: preparation of 4-(5-(2-methoxyphenyl)isoxazole-3-yl)aniline

### Step 1) Preparation of tert-Butyl (4-(5-(2-methoxyphenyl)isoxazol-3-yl)phenyl)carbamate

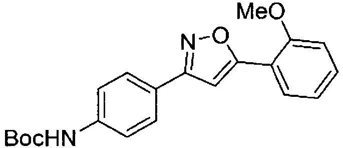

The procedure of Preparation Example 1, Step 2) was repeated to obtain the target compound (185 mg, 72% yield), except that 1-ethynyl-2-methoxybenzene (93 mg, 0.71 mmol) was used instead of 4-ethynylanisole.

¹H NMR (DMSO-d₆, 800 MHz) δ 9.61 (s, 1H), 7.88 (dd, J = 7.7, 1.6 Hz, 1H), 7.84 (d, J = 8.7 Hz, 2H), 7.61 (d, J = 8.4 Hz, 2H), 7.53-7.51 (m, 1H), 7.31 (s, 1H), 7.25 (d, J = 8.3 Hz, 1H), 7.14-7.12 (m, 1H), 3.99 (s, 3H), 1.50 (s, 9H)

### Step 2) Preparation of 4-(5-(2-methoxyphenyl)isoxazole-3-yl)aniline

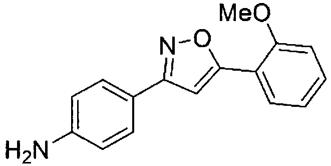

The procedure of Preparation Example 1, Step 3) was repeated to obtain the target compound (19 mg, 87% yield), except that tert-butyl (4-(5-(2-methoxyphenyl)isoxazol-3-yl)phenyl)carbamate (30 mg, 0.08 mmol) was used instead of tert-butyl (4-(5-(4-methoxyphenyl)isoxazol-3-yl)phenyl)carbamate.

¹H NMR (DMSO-d₆, 800 MHz) δ 7.86 (dd, J = 7.7, 1.6 Hz, 1H), 7.59 (d, J = 8.5 Hz, 2H), 7.50 (ddd, J = 8.5, 7.2, 1.5 Hz, 1H), 7.23 (d, J = 8.1 Hz, 1H), 7.18 (s, 1H), 7.12 (td, J = 7.5, 0.8 Hz, 1H), 6.65 (d, J = 8.6 Hz, 2H), 5.55 (s, 2H), 3.98 (s, 3H)

### Preparation Example 4: preparation of 4-(5-(3,4-dimethoxyphenyl)isoxazole-3-yl)aniline

### Step 1) Preparation of tert-butyl (4-(5-(3,4-dimethoxyphenyl)isoxazol-3-yl)phenyl)carbamate

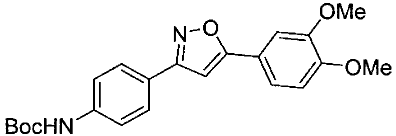

The procedure of Preparation Example 1, Step 2) was repeated to obtain the target compound (120 mg, 43% yield), except that 4-ethynyl-1,2-dimethoxybenzene (115 mg, 0.71 mmol) was used instead of 4-ethynylanisole.

¹H NMR (DMSO-d₆, 800 MHz) δ 9.62 (s, 1H), 7.79 (d, J = 8.6 Hz, 2H), 7.61 (d, J = 8.5 Hz, 2H), 7.47 (dd, J = 8.3, 1.9 Hz, 1H), 7.43 (dd, J = 8.3, 1.9 Hz, 1H), 7.43 (s, 1H), 7.13 (d, J = 8.4 Hz, 1H), 3.87 (s, 3H), 3.83 (s, 3H), 1.50 (s, 9H)

### Step 2) Preparation of 4-(5-(3,4-dimethoxyphenyl)isoxazole-3-yl)aniline

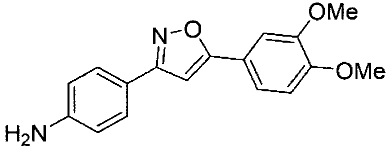

The procedure of Preparation Example 1, Step 3) was repeated to obtain the target compound (68 mg, 91% yield), except that tert-butyl (4-(5-(3,4-dimethoxyphenyl)isoxazol-3-yl)phenyl)carbamate (100 mg, 0.25 mmol) was used instead of tert-butyl (4-(5-(4-methoxyphenyl)isoxazol-3-yl)phenyl)carbamate.

¹H NMR (DMSO-d₆, 800 MHz) δ 7.55 (d, J = 8.5 Hz, 2H), 7.44 (dd, J = 8.3, 2.0 Hz, 1H), 7.41 (d, J = 2.0 Hz, 1H), 7.29 (s, 1H), 7.11 (d, J = 8.4 Hz, 1H), 6.65 (d, J = 8.6 Hz, 2H), 5.55 (s, 2H), 3.86 (s, 3H), 3.83 (s, 3H)

### Preparation Example 5: preparation of 4-(5-(4-(tert-butyl)phenyl)isoxazole-3-yl)aniline

### Step 1) Preparation of tert-butyl (4-(5-(4-(tert-butyl)phenyl)isoxazol-3-yl)phenyl)carbamate

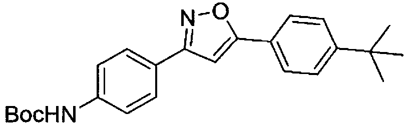

The procedure of Preparation Example 1, Step 2) was repeated to obtain the target compound (194 mg, 70% yield), except that 1-(tert-butyl)-4-ethynylbenzene (112 mg, 0.71 mmol) was used instead of 4-ethynylanisole.

¹H NMR (DMSO-d₆, 800 MHz) δ 9.63 (s, 1H), 7.83 (d, J = 8.4 Hz, 2H), 7.81 (d, J = 8.7 Hz, 2H), 7.61 (d, J = 8.4 Hz, 2H), 7.59 (d, J = 8.5 Hz, 2H), 7.46 (s, 1H), 1.50 (s, 9H), 1.32 (s, 9H)

### Step 2) Preparation of 4-(5-(4-(tert-butyl)phenyl)isoxazole-3-yl)aniline

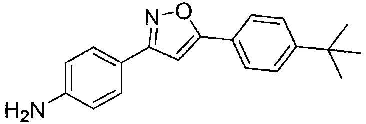

The procedure of Preparation Example 1, Step 3) was repeated to obtain the target compound (61 mg, 91% yield), except that tert-butyl (4-(5-(4-(tert-butyl)phenyl)isoxazol-3-yl)phenyl)carbamate (90 mg, 0.23 mmol) was used instead of tert-butyl (4-(5-(4-methoxyphenyl)isoxazol-3-yl)phenyl)carbamate.

¹H NMR (DMSO-d₆, 800 MHz) δ 7.80 (d, J = 8.4 Hz, 2H), 7.57 (d, J = 8.4 Hz, 4H), 7.32 (s, 1H), 6.65 (d, J = 8.5 Hz, 2H), 5.56 (s, 2H), 1.32 (s, 9H)

### Preparation Example 6: preparation of 4-(5-(4-fluorophenyl)isoxazole-3-yl)aniline

### Step 1) Preparation of tert-butyl (4-(5-(4-fluorophenyl)isoxazol-3-yl)phenyl)carbamate

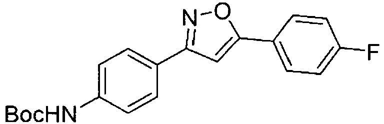

The procedure of Preparation Example 1, Step 2) was repeated to obtain the target compound (210 mg, 84% yield), except that 1-ethynyl-4-fluorobenzene (85 mg, 0.71 mmol) was used instead of 4-ethynylanisole.

¹H NMR (DMSO-d₆, 800 MHz) δ 9.64 (s, 1H), 7.96 (dd, J = 8.7, 5.3 Hz, 2H), 7.80 (d, J = 8.7 Hz, 2H), 7.62 (d, J = 8.4 Hz, 2H), 7.52 (s, 1H), 7.43 (t, J = 8.8 Hz, 2H), 1.50 (s, 9H)

### Step 2) Preparation of 4-(5-(4-fluorophenyl)isoxazole-3-yl)aniline

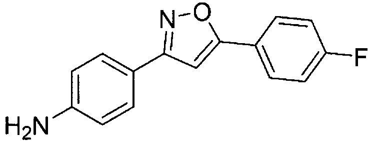

The procedure of Preparation Example 1, Step 3) was repeated to obtain the target compound (20 mg, 93% yield), except that tert-butyl (4-(5-(4-fluorophenyl)isoxazol-3-yl)phenyl)carbamate (30 mg, 0.08 mmol) was used instead of tert-butyl (4-(5-(4-methoxyphenyl)isoxazol-3-yl)phenyl)carbamate.

¹H NMR (DMSO-d₆, 800 MHz) δ 7.93 (dd, J = 8.8, 5.4 Hz, 2H), 7.56 (d, J = 8.5 Hz, 2H), 7.41 (t, J = 88 Hz, 2H), 7.38 (s, 1H), 6.65 (d, J = 8.5 Hz, 2H), 5.58 (s, 2H)

### Preparation Example 7: preparation of 4-(5-(4-(trifluoromethyl)phenyl)isoxazole-3-yl)aniline

### Step 1) Preparation of tert-butyl (4-(5-(4-(trifluoromethyl)phenyl)isoxazol-3-yl)phenyl)carbamate

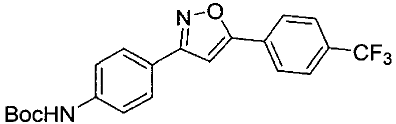

The procedure of Preparation Example 1, Step 2) was repeated to obtain the target compound (195 mg, 85% yield), except that 1-ethynyl-4-(trifluoromethyl)benzene (96 mg, 0.56 mmol) was used instead of 4-ethynylanisole.

¹H NMR (DMSO-d₆, 800 MHz) δ 9.65 (s, 1H), 8.13 (d, J = 8.2 Hz, 2H), 7.95 (d, J = 8.3 Hz, 2H), 7.82 (d, J = 8.6 Hz, 2H), 7.75 (s, 1H), 7.63 (d, J = 8.4 Hz, 2H), 1.50 (s, 9H)

### Step 2) Preparation of 4-(5-(4-(trifluoromethyl)phenyl)isoxazole-3-yl)aniline

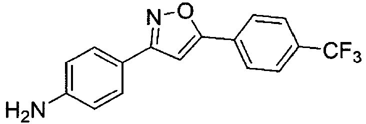

The procedure of Preparation Example 1, Step 3) was repeated to obtain the target compound (52 mg, 86% yield), except that tert-butyl (4-(5-(4-(trifluoromethyl)phenyl)isoxazol-3-yl)phenyl)carbamate (80 mg, 0.20 mmol) was used instead of tert-butyl (4-(5-(4-methoxyphenyl)isoxazol-3-yl)phenyl)carbamate.

¹H NMR (DMSO-d₆, 800 MHz) δ 8.10 (d, J = 8.2 Hz, 2H), 7.93 (d, J = 8.3 Hz, 2H), 7.61 (s, 1H), 7.59 (d, J = 8.5 Hz, 2H), 6.66 (d, J = 8.6 Hz, 2H), 5.61 (s, 2H)

### Preparation Example 8: preparation of 4-(5-(4-propoxyphenyl)isoxazole-3-yl)aniline

### Step 1) preparation of tert-butyl (4-(5-(4-propoxyphenyl)isoxazol-3-yl)phenyl)carbamate

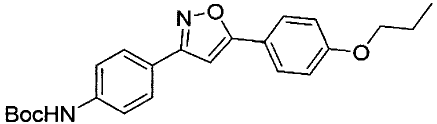

The procedure of Preparation Example 1, Step 2) was repeated to obtain the target compound (220 mg, 79% yield), except that 1-ethynyl-4-propoxybenzene (114 mg, 0.71 mmol) was used instead of 4-ethynylanisole.

¹H NMR (DMSO-d₆, 800 MHz) δ 9.62 (s, 1H), 7.82 (d, J = 8.7 Hz, 2H), 7.79 (d, J = 8.7 Hz, 2H), 7.61 (d, J = 8.4 Hz, 2H), 7.36 (s, 1H), 7.10 (d, J = 8.8 Hz, 2H), 4.01 (t, J = 6.4 Hz, 2H), 1.78-1.74 (m, 2H), 1.00 (t, J = 7.3 Hz, 3H)

### Step 2) Preparation of 4-(5-(4-propoxyphenyl)isoxazole-3-yl)aniline

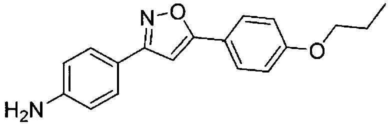

The procedure of Preparation Example 1, Step 3) was repeated to obtain the target compound (19 mg, 85% yield), except that tert-butyl (4-(5-(4-propoxyphenyl)isoxazol-3-yl)phenyl)carbamate (30 mg, 0.08 mmol) was used instead of tert-butyl (4-(5-(4-methoxyphenyl)isoxazol-3-yl)phenyl)carbamate.

¹H NMR (DMSO-d₆, 800 MHz) δ 7.79 (d, J = 8.8 Hz, 2H), 7.55 (d, J = 8.5 Hz, 2H), 7.22 (s, 1H), 7.09 (d, J = 8.8 Hz, 2H), 6.64 (d, J = 8.5 Hz, 2H), 5.55 (s, 2H), 4.01 (t, J = 6.5 Hz, 2H), 1.78-1.73 (m, 2H), 0.99 (t, J = 7.4 Hz, 3H)

### Preparation Example 9: preparation of 3-(5-(4-methoxyphenyl)isoxazole-3-yl)aniline

### Step 1) Preparation of tert-butyl (E)-(3-((hydroxyimino)methyl)phenyl)carbamate

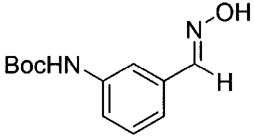

The procedure of Preparation Example 1, Step 1) was repeated to obtain the target compound (355 mg, 42% yield), except that tert-butyl (3-formylphenyl)carbamate (800 mg, 3.62 mmol) was used instead of tert-butyl (4-formylphenyl)carbamate.

¹H NMR (DMSO-d₆, 800 MHz) δ 8.08 (s, 1H), 7.62 (s, 1H), 7.35 (d, J = 7.8 Hz, 1H), 7.28 (t, J = 7.9 Hz, 1H), 7.24 (s, 2H), 7.22 (d, J = 7.7 Hz, 1H), 1.50 (s, 9H)

### Step 2) Preparation of tert-butyl (3-(5-(4-methoxyphenyl)isoxazol-3-yl)phenyl)carbamate

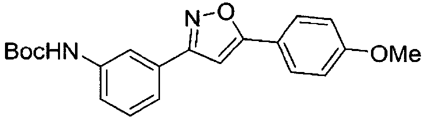

The procedure of Preparation Example 1, Step 2) was repeated to obtain the target compound (250 mg, 84% yield), except that tert-butyl (*E*)-(3-((hydroxyimino)methyl)phenyl)carbamate (290 mg, 1.23 mmol) was used instead of tert-butyl (*E*)-(4-((hydroxyimino)methyl)phenyl)carbamate.

¹H NMR (DMSO-d₆, 800 MHz) δ 9.55 (s, 1H), 8.11 (s, 1H), 7.88 (d, J = 8.8 Hz, 2H), 7.51 (d, J = 7.7 Hz, 1H), 7.47 (dt, J = 6.3, 1.3 Hz, 1H), 7.41 (t, J = 7.8 Hz, 1H), 7.35 (s, 1H), 7.12 (d, J = 8.9 Hz, 2H), 3.84 (s, 3H), 1.50 (s, 9H)

### Step 3) Preparation of 3-(5-(4-methoxyphenyl)isoxazol-3-yl)aniline

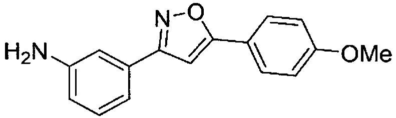

The procedure of Preparation Example 1, Step 3) was repeated to obtain the target compound (135 mg, 88% yield), except that tert-butyl (3-(5-(4-methoxyphenyl)isoxazol-3-yl)phenyl)carbamate (210 mg, 0.57 mmol) was used instead of tert-butyl (4-(5-(4-methoxyphenyl)isoxazol-3-yl)phenyl)carbamate.

¹H NMR (DMSO-d₆, 800 MHz) δ 7.85 (d, J = 8.9 Hz, 2H), 7.28 (s, 1H), 7.16 (t, J = 7.7 Hz, 1H), 7.12-7.10 (m, 3H), 6.99 (dt, J = 6.2, 1.2 Hz, 1H), 6.68 (ddd, J = 7.9, 2.2, 0.8 Hz, 1H), 5.31 (s, 2H), 3.84 (s, 3H)

### Preparation Example 10: preparation of N-(4-(5-(4-methoxyphenyl)isoxazole-3-yl)phenyl)acetamide

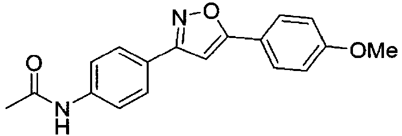

4-(5-(4-methoxyphenyl)isoxazol-3-yl)aniline (20 mg, 0.08 mmol) prepared in Step 3) of Preparation Example 1 and N,N-diisopropylethylamine (0.04 mL, 0.23 mmol) were dissolved in dichloromethane (2 mL), and acetyl chloride (0.02 mL, 0.15 mmol) was added thereto dropwise at 0°C. The mixture was stirred at room temperature for 12 hours, and after the reaction was completed, water was added dropwise. The mixture was extracted three times with dichloromethane, dried over anhydrous magnesium sulfate, and concentrated under reduced pressure. The resulting residue was purified by flash column chromatography (ethyl acetate:n-hexane = 1:1), and the resulting solution was concentrated to obtain the target compound (21 mg, 91 % yield).

¹H NMR (DMSO-d₆, 800 MHz) δ 10.16 (s, 1H), 7.84 (d, J = 8.9 Hz, 2H), 7.83 (d, J = 8.9 Hz, 2H), 7.74 (d, J = 8.6 Hz, 2H), 7.38 (s, 1H), 7.12 (d, J = 8.8 Hz, 2H), 3.84 (s, 3H), 2.08 (s, 3H) 13C NMR (DMSO-d6, 200 MHz) δ 169.5, 168.6, 162.1, 160.8, 141.0, 127.2, 127.1, 123.1, 119.6, 119.1, 114.7, 96.9, 55.4, 24.1

### Preparation Example 11: preparation of N-(4-(5-(4-methoxyphenyl)isoxazole-3-yl)phenyl)methanesulfonamide

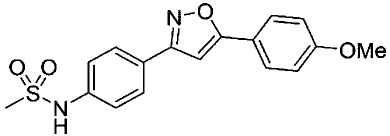

4-(5-(4-methoxyphenyl)isoxazol-3-yl)aniline (50 mg, 0.19 mmol) prepared in Step 3) of Preparation Example 1 was dissolved in pyridine (2.5 mL), and methanesulfonyl chloride (0.02 mL, 0.28 mmol) was added thereto dropwise. The mixture was stirred at room temperature for 1 hour, and after the reaction was completed, water was added dropwise. The mixture was extracted three times with dichloromethane, dried over anhydrous magnesium sulfate, and concentrated under reduced pressure. The resulting residue was purified by flash column chromatography (ethyl acetate:n-hexane = 1:3), and the resulting solution was concentrated to obtain the target compound (60 mg, 93% yield).

¹H NMR (DMSO-d₆, 800 MHz) δ 10.08 (s, 1H), 7.85 (dd, J = 12.7, 8.8 Hz, 4H), 7.40 (s, 1H), 7.34 (d, J = 8.6 Hz, 2H), 7.12 (d, J = 8.9 Hz, 2H), 3.84 (s, 3H), 3.08 (s, 3H)

### Preparation Example 12: preparation of N-(4-(5-(4-methoxyphenyl)isoxazole-3-yl)phenyl)-4-methylbenzenesulfonamide

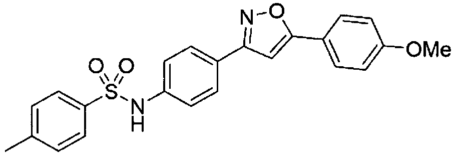

4-(5-(4-methoxyphenyl)isoxazol-3-yl)aniline (30 mg, mmol) prepared in Step 3) of Preparation Example 1 and pyridine (0.02 mL, 0.23 mmol) were dissolved in dichloromethane (3 mL), and 4-toluenesulfonyl chloride (43 mg, 0.23 mmol) was added thereto dropwise. The mixture was stirred at room temperature for 6 hours, and after the reaction was completed, water was added dropwise. The mixture was extracted three times with dichloromethane, dried over anhydrous magnesium sulfate, and concentrated under reduced pressure. The resulting residue was purified by flash column chromatography (ethyl acetate:n-hexane = 1:2), and the resulting solution was concentrated to obtain the target compound (30 mg, 63% yield).

¹H NMR (DMSO-d₆, 800 MHz) δ 10.56 (s, 1H), 7.81 (d, J = 8.8 Hz, 2H), 7.75 (d, J = 8.7 Hz, 2H), 7.70 (d, J = 8.4 Hz, 2H), 7.36 (d, J = 8.2 Hz, 2H), 7.32 (s, 1H), 7.23 (d, J = 8.7 Hz, 2H), 7.11 (d, J = 8.9 Hz, 2H), 3.83 (s, 3H), 2.33 (s, 3H)

### Preparation Example 13: preparation of N-(4-(5-(4-hydroxyphenyl)isoxazole-3-yl)phenyl)-4-methylbenzenesulfonamide

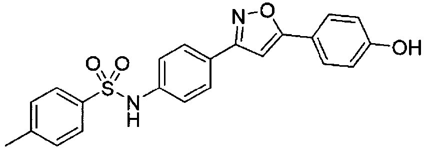

N-(4-(5-(4-methoxyphenyl)isoxazol-3-yl)phenyl)-4-methylbenzenesulfonamide (30 mg, 0.07 mmol) prepared in Preparation Example 12 was dissolved in dichloromethane (3 mL), and boron tribromide (0.11 mL, 0.11 mmol) was added thereto dropwise. The mixture was stirred at room temperature for 12 hours, and after the reaction was completed, water was added dropwise. The mixture was extracted three times with dichloromethane, dried over anhydrous magnesium sulfate, and concentrated under reduced pressure. The resulting residue was purified by flash column chromatography (ethyl acetate:n-hexane = 1:1), and the resulting solution was concentrated to obtain the target compound (18 mg, 62% yield).

¹H NMR (DMSO-d₆, 800 MHz) δ 10.54 (s, 1H), 10.08 (s, 1H), 7.74 (d, J = 8.7 Hz, 2H), 7.70 (d, J = 8.2 Hz, 2H), 7.69 (d, J = 8.7 Hz, 2H), 7.36 (d, J = 8.1 Hz, 2H), 7.23 (d, J = 8.8 Hz, 2H), 7.22 (s, 1H), 6.91 (d, J = 8.7 Hz, 2H), 2.33 (s, 3H)

### Preparation Example 14: preparation of N-(4-(5-(4-methoxyphenyl)isoxazole-3-yl)phenyl)-4-methylbenzamide

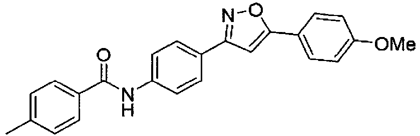

4-(5-(4-methoxyphenyl)isoxazol-3-yl)aniline (20 mg, 0.08 mmol) prepared in Step 3) of Preparation Example 1 and *N*,*N*-diisopropylethylamine (0.04 mL, 0.23 mmol) were dissolved in dichloromethane (3 mL), and p-toluoyl chloride (0.02 mL, 0.15 mmol) was added thereto dropwise. The mixture was stirred at room temperature for 12 hours, and after the reaction was completed, water was added dropwise. The mixture was extracted three times with dichloromethane, dried over anhydrous magnesium sulfate, and concentrated under reduced pressure. The resulting residue was purified by flash column chromatography (ethyl acetate:n-hexane = 1:3), and the resulting solution was concentrated to obtain the target compound (21 mg, 73% yield).

¹H NMR (DMSO-d₆, 800 MHz) δ 10.38 (s, 1H), 7.96 (d, J = 8.7 Hz, 2H), 7.90 (d, J = 8.2 Hz, 2H), 7.89 (d, J = 8.7 Hz, 2H), 7.85 (d, J = 8.8 Hz, 2H), 7.43 (s, 1H), 7.36 (d, J = 7.8 Hz, 2H), 7.13 (d, J = 8.8 Hz, 2H), 3.85 (s, 3H), 2.40 (s, 3H) ¹³C NMR (DMSO-d₆, 200 MHz) δ 169.6, 165.6, 162.1, 160.9, 141.8, 141.0, 131.8, 129.0, 127.8, 127.3, 127.0, 123.6, 120.4, 119.6, 114.7, 96.9, 55.4, 21.0

### Preparation Example 15: preparation of N-(4-(5-(4-methoxyphenyl)isoxazole-3-yl)phenyl)acrylamide

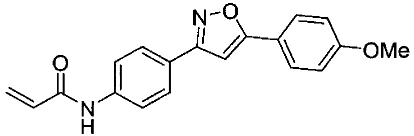

4-(5-(4-methoxyphenyl)isoxazol-3-yl)aniline (20 mg, 0.08 mmol) prepared in Step 3) of Preparation Example 1 was dissolved in a mixture of tetrahydrofuran: and saturated aqueous sodium bicarbonate solution (4:1, 2.5 mL), and acrylyl chloride (0.01 mL, 0.11 mmol) was added thereto dropwise. The mixture was stirred at room temperature for 12 hours, and after the reaction was completed, water was added dropwise. The mixture was extracted three times with dichloromethane, dried over anhydrous magnesium sulfate, and concentrated under reduced pressure. The resulting residue was purified by flash column chromatography (ethyl acetate:n-hexane = 1: 1), and the resulting solution was concentrated to obtain the target compound (17 mg, 71 % yield).

¹H NMR (DMSO-d₆, 800 MHz) δ 10.38 (s, 1H), 7.87 (d, J = 8.7 Hz, 2H), 7.85 (d, J = 8.8 Hz, 2H), 7.83 (d, J = 8.7 Hz, 2H), 7.41 (s, 1H), 7.13 (d, J = 8.8 Hz, 2H), 6.47 (dd, J = 16.9, 10.2 Hz, 1H), 6.30 (dd, J = 17.0, 1.7 Hz, 1H), 5.80 (dd, J = 10.1, 1.7 Hz, 1H), 3.84 (s, 3H) ¹³C NMR (DMSO-d₆, 200 MHz) δ 169.6, 163.4, 162.1, 160.9, 140.7, 131.7, 127.4, 127.2, 127.2, 123.6, 119.6, 119.5, 114.7, 96.9, 55.4

### Preparation Example 16: preparation of N-(4-(5-(3-methoxyphenyl)isoxazole-3-yl)phenyl)acetamide

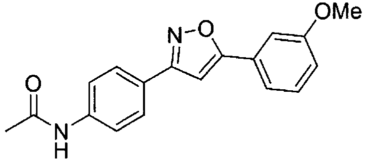

The procedure of Preparation Example 10 was repeated to obtain the target compound (21 mg, 91% yield), except that 4-(5-(3-methoxyphenyl)isoxazol-3-yl)aniline (20 mg, 0.08 mmol) prepared in Step 2) of Preparation Example 2 was used instead of 4-(5-(4-methoxyphenyl)isoxazol-3-yl)aniline.

¹H NMR (DMSO-d₆, 800 MHz) δ 10.17 (s, 1H), 7.84 (d, J = 8.6 Hz, 2H), 7.75 (d, J = 8.6 Hz, 2H), 7.58 (s, 1H), 7.50-7.47 (m, 2H), 7.45-7.44 (m, 1H), 7.11-7.09 (m, 1H), 3.86 (s, 3H), 2.08 (s, 3H) ¹³C NMR (DMSO-d₆, 200 MHz) δ 169.3, 168.6, 162.2, 159.7, 141.1, 130.5, 128.1, 127.2, 122.9, 119.1, 117.8, 116.4, 110.6, 98.7, 55.4, 24.1

### Preparation Example 17: preparation of N-(4-(5-(2-methoxyphenyl)isoxazole-3-yl)phenyl)acetamide

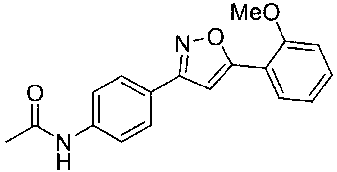

The procedure of Preparation Example 10 was repeated to obtain the target compound (6 mg, 86% yield), except that 4-(5-(2-methoxyphenyl)isoxazol-3-yl)aniline (6 mg, 0.02 mmol) prepared in Step 2) of Preparation Example 3 was used instead of 4-(5-(4-methoxyphenyl)isoxazol-3-yl)aniline.

¹H NMR (DMSO-d₆, 800 MHz) δ 10.17 (s, 1H), 7.90-7.88 (m, 3H), 7.74 (d, J = 8.6 Hz, 2H), 7.52 (ddd, J = 8.8, 7.4, 1.7 Hz, 1H), 7.33 (s, 1H), 7.25 (d, J = 8.2 Hz, 1H), 7.13 (td, J = 7.6, 0.9 Hz, 1H), 3.99 (s, 3H), 2.08 (s, 3H) ¹³C NMR (DMSO-d₆, 200 MHz) δ 168.6, 165.7, 162.0, 156.0, 141.0, 131.8, 127.2, 127.0, 123.1, 120.8, 119.1, 115.4, 112.2, 101.4, 55.8, 24.1

### Preparation Example 18: preparation of N-(4-(5-(3,4-dimethoxyphenyl)isoxazole-3-yl)phenyl)acetamide

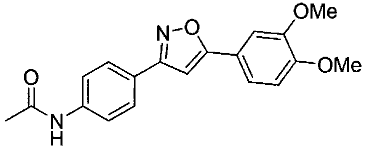

The procedure of Preparation Example 10 was repeated to obtain the target compound (17 mg, 74% yield), except that 4-(5-(3,4-dimethoxyphenyl)isoxazol-3-yl)aniline (20 mg, 0.07 mmol) prepared in Step 2) of Preparation Example 4 was used instead of 4-(5-(4-methoxyphenyl)isoxazol-3-yl)aniline.

¹H NMR (DMSO-d₆, 800 MHz) δ 10.17 (s, 1H), 7.83 (d, J = 8.7 Hz, 2H), 7.74 (d, J = 8.6 Hz, 2H), 7.47 (dd, J = 8.3, 2.0 Hz, 1H), 7.44 (s, 1H), 7.44 (s, 1H), 7.13 (d, J = 8.4 Hz, 1H), 3.87 (s, 3H), 3.84 (s, 3H), 2.08 (s, 3H) ¹³C NMR (DMSO-d₆, 200 MHz) δ 169.7, 168.6, 162.1, 161.6, 149.1, 133.2, 127.1, 123.1, 119.7, 119.1, 118.5, 116.4, 112.0, 108.9, 97.1, 55.7, 55.6, 24.2

### Preparation Example 19: preparation of N-(4-(5-(4-propoxyphenyl)isoxazole-3-yl)phenyl)acetamide

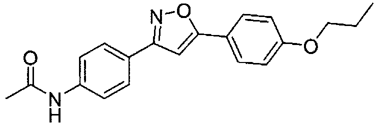

The procedure of Preparation Example 10 was repeated to obtain the target compound (8 mg, 78% yield), except that 4-(5-(4-propoxyphenyl)isoxazol-3-yl)aniline (9 mg, 0.03 mmol) prepared in Step 2) of Preparation Example 8 was used instead of 4-(5-(4-methoxyphenyl)isoxazol-3-yl)aniline.

¹H NMR (DMSO-d₆, 800 MHz) δ 10.17 (s, 1H), 7.82 (dd, J = 8.7, 3.1 Hz, 4H), 7.73 (d, J = 8.6 Hz, 2H), 7.37 (s, 1H), 7.11 (d, J = 8.8 Hz, 2H), 4.02 (t, J = 6.5 Hz, 2H), 2.08 (s, 3H), 1.78-1.74 (m, 2H), 1.00 (t, J = 7.4 Hz, 3H) ¹³C NMR (DMSO-d₆, 200 MHz) δ 169.6, 168.6, 162.1, 160.3, 141.0, 127.2, 127.1, 123.1, 119.4, 119.1, 115.1, 96.8, 69.2, 24.1, 21.9, 10.3

### Preparation Example 20: preparation of N-(4-(5-(4-(trifluoromethyl)phenyl)isoxazole-3-yl)phenyl)acetamide

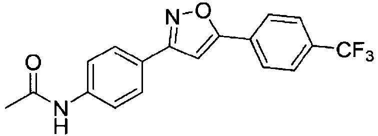

The procedure of Preparation Example 10 was repeated to obtain the target compound (25 mg, 81% yield), except that 4-(5-(4-(trifluoromethyl)phenyl)isoxazol-3-yl)aniline (27 mg, 0.09 mmol) prepared in Step 2) of Preparation Example 7 was used instead of 4-(5-(4-methoxyphenyl)isoxazol-3-yl)aniline.

¹H NMR (DMSO-d₆, 800 MHz) δ 10.15 (s, 1H), 8.09 (d, J = 8.1 Hz, 2H), 7.92 (d, J = 8.3 Hz, 2H), 7.83 (d, J = 8.7 Hz, 2H), 7.74-7.71 (m, 4H), 2.05 (s, 3H)

### Preparation Example 21: preparation of N-(4-(5-(4-fluorophenyl)isoxazole-3-yl)phenyl)acetamide

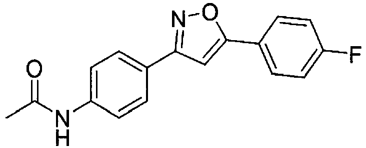

The procedure of Preparation Example 10 was repeated to obtain the target compound (9 mg, 77% yield), except that 4-(5-(4-fluorophenyl)isoxazol-3-yl)aniline (10 mg, 0.04 mmol) prepared in Step 2) of Preparation Example 6 was used instead of 4-(5-(4-methoxyphenyl)isoxazol-3-yl)aniline

¹H NMR (DMSO-d₆, 800 MHz) δ 10.18 (s, 1H), 7.98-7.96 (m, 2H), 7.84 (d, J = 8.7 Hz, 2H), 7.75 (d, J = 9.0 Hz, 2H), 7.53 (s, 1H), 7.44-7.41 (m, 2H), 2.08 (s, 3H) ¹³C NMR (DMSO-d₆, 200 MHz) δ 168.7, 168.6, 163.8, 162.5, 162.3, 141.1, 133.2, 128.1, 128.0, 127.2, 123.6, 122.9, 119.1, 116.5, 116.4, 98.3, 24.1

### Preparation Example 22: preparation of N-(4-(5-(4-(tert-butyl)phenyl)isoxazole-3-yl)phenyl)acetamide

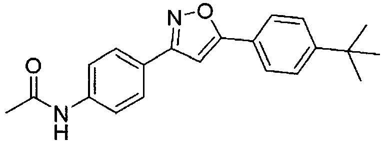

The procedure of Preparation Example 10 was repeated to obtain the target compound (11 mg, 64% yield), except that 4-(5-(4-(tert-butyl)phenyl)isoxazol-3-yl)aniline (15 mg, 0.05 mmol) prepared in Step 2) of Preparation Example 5 was used instead of 4-(5-(4-methoxyphenyl)isoxazol-3-yl)aniline.

¹H NMR (DMSO-d₆, 800 MHz) δ 10.17 (s, 1H), 7.85 (d, J = 8.7 Hz, 2H), 7.83 (d, J = 8.5 Hz, 2H), 7.74 (d, J = 8.6 Hz, 2H), 7.59 (d, J = 8.5 Hz, 2H), 7.48 (s, 1H), 2.08 (s, 3H), 1.33 (s, 9H) ¹³C NMR (DMSO-d₆, 200 MHz) δ 169.6, 168.6, 162.1, 153.2, 141.1, 127.2, 126.1, 125.4, 124.3, 123.0, 119.1, 97.8, 34.7, 30.9, 24.1

### Preparation Example 23: preparation of N-(4-(5-(pyridine-4-yl)isoxazole-3-yl)phenyl)acetamide

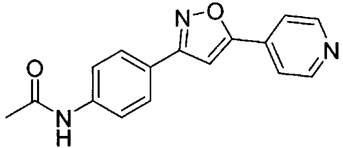

The procedure of Preparation Example 1, Step 2) was repeated to obtain tert-butyl (4-(5-(pyridin-4-yl)isoxazol-3-yl)phenyl)carbamate (130 mg, 45% yield), except that 4-ethynylpyridine (58.2 mg, 0.56 mmol) was used instead of 4-ethynylanisole.

The procedure of Preparation Example 1, Step 3) was repeated to obtain the target compound (62 mg, 77% yield), except that the obtained tert-butyl (4-(5-(pyridin-4-yl)isoxazol-3-yl)phenyl)carbamate was used instead of tert-butyl (4-(5-(4-methoxyphenyl)isoxazol-3-yl)phenyl)carbamate.

The procedure of Example 10 was repeated to obtain the final target compound (18 mg, 77% yield), except that the obtained 4-(5-(pyridin-4-yl)isoxazol-3-yl)aniline (20 mg, 0.08 mmol) was used instead of 4-(5-(4-methoxyphenyl)isoxazol-3-yl)aniline.

¹H NMR (DMSO-d₆, 800 MHz) δ 10.20 (s, 1H), 8.79 (dd, J = 4.4, 1.6 Hz, 2H), 7.87-7.86 (m, 3H), 7.86 (s, 1H), 7.84 (s, 1H), 7.76 (d, J = 8.6 Hz, 2H), 2.08 (s, 3H) ¹³C NMR (DMSO-d₆, 200 MHz) δ 168.7, 167.1, 162.5, 150.8, 141.3, 133.4, 127.3, 122.5, 119.4, 119.1, 101.4, 24.1

### Preparation Example 24: preparation of N-(3-(5-(4-methoxyphenyl)isoxazole-3-yl)phenyl)acetamide

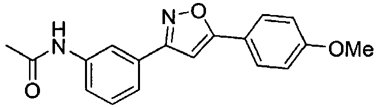

The procedure of Preparation Example 10 was repeated to obtain the target compound (15 mg, 65% yield), except that 3-(5-(4-methoxyphenyl)isoxazol-3-yl)aniline (20 mg, 0.08 mmol) prepared in Step 3) of Preparation Example 9 was used instead of 4-(5-(4-methoxyphenyl)isoxazol-3-yl)aniline.

¹H NMR (DMSO-d₆, 800 MHz) δ 10.14 (s, 1H), 8.18 (t, J = 1.7 Hz, 1H), 7.88 (d, J = 8.9 Hz, 2H), 7.70 (dd, J = 8.1, 1.0 Hz, 1H), 7.54 (dt, J = 6.5, 1.2 Hz, 1H), 7.46 (t, J = 7.9 Hz, 1H), 7.37 (s, 1H), 7.13-7.11 (m, 2H), 3.84 (s, 3H), 2.08 (s, 3H) ¹³C NMR (DMSO-d₆, 200 MHz) δ 169.8, 168.5, 162.5, 160.9, 139.9, 129.5, 129.1, 127.3, 121.5, 120.7, 119.5, 116.6, 114.7, 97.1, 55.4, 24.0

### Preparation Example 25: preparation of N-(3-(5-(4-methoxyphenyl)isoxazole-3-yl)phenyl)-4-methylbenzamide

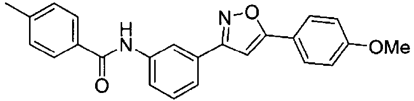

The procedure of Preparation Example 14 was repeated to obtain the target compound (22 mg, 76% yield), except that 3-(5-(4-methoxyphenyl)isoxazol-3-yl)aniline (20 mg, 0.08 mmol) prepared in Step 3) of Preparation Example 9 was used instead of 4-(5-(4-methoxyphenyl)isoxazol-3-yl)aniline.

¹H NMR (DMSO-d₆, 800 MHz) δ 10.36 (s, 1H), 8.39 (t, J = 1.8 Hz, 1H), 7.92 (d, J = 8.1 Hz, 3H), 7.89 (d, J = 8.8 Hz, 2H), 7.61 (dt, J = 6.4, 1.3 Hz, 1H), 7.52 (t, J = 7.9 Hz, 1H), 7.41 (s, 1H), 7.36 (d, J = 7.8 Hz, 2H), 7.13 (d, J = 8.9 Hz, 2H), 3.84 (s, 3H), 2.40 (s, 3H) ¹³C NMR (DMSO-d₆, 200 MHz) δ 169.8, 165.5, 162.5, 160.9, 141.8, 139.9, 131.8, 129.4, 129.0, 129.0, 127.7, 127.3, 122.0, 122.0, 119.5, 118.1, 114.7, 97.1, 55.4, 21.0

### Preparation Example 26: preparation of N-(4-(5-(4-hydroxyphenyl)isoxazole-3-yl)phenyl)acetamide

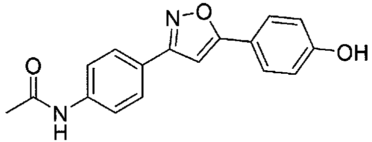

The procedure of Preparation Example 13 was repeated to obtain the target compound (16 mg, 84% yield), except that *N*-(4-(5-(4-methoxyphenyl)isoxazol-3-yl)phenyl)acetamide (20 mg, 0.07 mmol) prepared in Preparation Example 10 was used instead of *N*-(4-(5-(4-methoxyphenyl)isoxazol-3-yl)phenyl)-4-methylbenzenesulfonamide.

¹H NMR (DMSO-d₆, 800 MHz) δ 10.12 (s, 1H), 10.05 (s, 1H), 7.78 (d, J = 8.7 Hz, 2H), 7.69 (d, J = 8.5 Hz, 2H), 7.69 (d, J = 8.6 Hz, 2H), 7.24 (s, 1H), 6.88 (d, J = 8.7 Hz, 2H), 2.04 (s, 3H) ¹³C NMR (DMSO-d₆, 200 MHz) δ 169.9, 168.6, 162.0, 159.4, 141.0, 127.3, 127.1, 123.2, 119.1, 118.1, 116.0, 96.1, 24.1

### Preparation Example 27: preparation of 4-(3-(4-acetamidophenyl)isoxazole-5-yl)phenyl 4-methylbenzenesulfonate

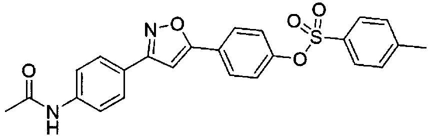

The procedure of Preparation Example 12 was repeated to obtain the target compound (6 mg, 79% yield), except that *N*-(4-(5-(4-hydroxyphenyl)isoxazol-3-yl)phenyl)acetamide (5 mg, 0.02 mmol) prepared in Preparation Example 26 was used instead of 4-(5-(4-methoxyphenyl)isoxazol-3-yl)aniline, and potassium carbonate (3.5 mg, 0.03 mmol) was used instead of pyridine.

¹H NMR (DMSO-d₆, 800 MHz) δ 10.18 (s, 1H), 7.91 (d, J = 8.7 Hz, 2H), 7.82 (d, J = 8.7 Hz, 2H), 7.78 (d, J = 8.3 Hz, 2H), 7.74 (d, J = 8.7 Hz, 2H), 7.55 (s, 1H), 7.49 (d, J = 8.0 Hz, 2H), 7.23 (d, J = 8.8 Hz, 2H), 2.43 (s, 3H), 2.08 (s, 3H) ¹³C NMR (DMSO-d₆, 200 MHz) δ 168.6, 168.2, 162.3, 150.1, 146.1, 141.2, 131.1, 130.3, 128.3, 127.4, 127.2, 126.0, 123.1, 122.7, 119.1, 99.1, 24.1, 21.2

### Preparation Example 28: preparation of N-(4-(3-(4-methoxyphenyl)isoxazole-5-yl)phenyl)acetamide

### Step 1) Preparation of (E)-4-methoxybenzaldehyde oxime

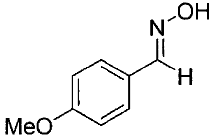

The procedure of Preparation Example 1, Step 1) was repeated to obtain the target compound (1.37 g, 62% yield), except that 4-methoxybenzaldehyde (2.0 g, 14.69 mmol) was used instead of tert-butyl (4-formylphenyl)carbamate.

¹H NMR (CDCl₃, 800 MHz) δ 8.14 (d, J = 9.0 Hz, 2H), 7.90 (s, 1H), 7.05 (d, J = 9.1 Hz, 2H), 3.92 (s, 3H)

### Step 2) Preparation of N-(4-(3-(4-methoxyphenyl)isoxazol-5-yl)phenyl)acetamide

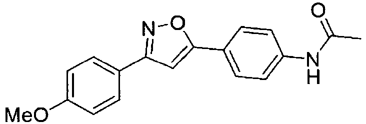

The procedure of Preparation Example 1, Step 2) was repeated to obtain the target compound (560 mg, 68% yield), except that (*E*)-4-methoxybenzaldehyde oxime (610 mg, 4.04 mmol) prepared in Step 1) of Preparation Example 28 was used instead of tert-butyl (*E*)-(4-((hydroxyimino)methyl)phenyl)carbamate, and *N*-(4-ethynylphenyl)acetamide (428 mg, 2.69 mmol) was used instead of 4-ethynylanisole.

¹H NMR (DMSO-d₆, 800 MHz) δ 10.22 (s, 1H), 7.85-7.82 (m, 4H), 7.76 (d, J = 8.6 Hz, 2H), 7.40 (s, 1H), 7.09 (d, J = 8.8 Hz, 2H), 3.83 (s, 3H), 2.09 (s, 3H) ¹³C NMR (DMSO-d₆, 200 MHz) δ 169.3, 168.7, 162.1, 160.7, 141.2, 128.0, 126.3, 121.5, 121.0, 119.1, 114.5, 97.2, 55.3, 24.1

### Preparation Example 29: preparation of N-(4-(3-(4-methoxyphenyl)isoxazole-5-yl)phenyl)-4-methylbenzenesulfonamide

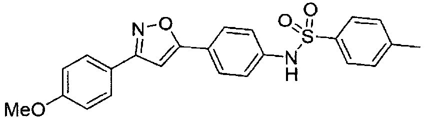

*N*-(4-(3-(4-methoxyphenyl)isoxazol-5-yl)phenyl)acetamide (10 mg, 0.03 mmol) prepared in Step 2) of Preparation Example 28 was dissolved in ethanol (2 mL), and a 37% aqueous hydrochloric acid solution (0.3 mL) was added thereto dropwise. After heating to 70°C, the mixture was stirred for 3 hours. After the reaction was completed, a saturated aqueous sodium bicarbonate solution was added thereto dropwise at room temperature. The mixture was extracted three times with a mixed solution of chloroform and isopropyl alcohol (4:1), dried over anhydrous magnesium sulfate, and concentrated under reduced pressure. The resulting residue was purified by flash column chromatography (ethyl acetate:n-hexane = 1:2), and the resulting solution was concentrated to obtain the target compound (8.0 mg, 93% yield).

The procedure of Preparation Example 12 was repeated to obtain the final target compound (14 mg, 89% yield), except that the obtained 4-(3-(4-methoxyphenyl)isoxazol-5-yl)aniline (10 mg, 0.04 mmol) was used instead of 4-(5-(4-methoxyphenyl)isoxazol-3-yl)aniline.

¹H NMR (DMSO-d₆, 800 MHz) δ 10.64 (s, 1H), 7.81 (d, J = 8.8 Hz, 2H), 7.74 (d, J = 8.2 Hz, 2H), 7.70 (d, J = 8.3 Hz, 2H), 7.36 (d, J = 7.8 Hz, 1H), 7.35 (s, 1H), 7.23 (d, J = 7.9 Hz, 2H), 7.08 (d, J = 8.9 Hz, 2H), 3.82 (s, 3H), 2.33 (s, 3H) ¹³C NMR (DMSO-d₆, 200 MHz) δ 162.1, 160.7, 129.8, 128.0, 126.7, 120.9, 119.4, 114.5, 55.3, 20.9

### Preparation Example 30: preparation of N-(4-(3-(4-hydroxyphenyl)isoxazole-5-yl)phenyl)-4-methylbenzenesulfonamide

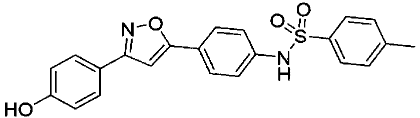

The procedure of Preparation Example 13 was repeated to obtain the target compound (11 mg, 76% yield), except that N-(4-(3-(4-methoxyphenyl)isoxazol-5-yl)phenyl)-4-methylbenzenesulfonamide (15 mg, 0.04 mmol) prepared in Step 2) of Preparation Example 29 was used instead of N-(4-(5-(4-methoxyphenyl)isoxazol-3-yl)phenyl)-4-methylbenzenesulfonamide.

¹H NMR (DMSO-d₆, 800 MHz) δ 10.63 (s, 1H), 9.92 (s, 1H), 7.74 (d, J = 8.7 Hz, 2H), 7.70 (d, J = 7.9 Hz, 2H), 7.69 (d, J = 8.5 Hz, 2H), 7.36 (d, J = 8.1 Hz, 2H), 7.30 (s, 1H), 7.25 (d, J = 8.7 Hz, 2H), 6.88 (d, J = 8.7 Hz, 2H), 2.33 (s, 3H) ¹³C NMR (DMSO-d₆, 200 MHz) δ 168.7, 162.3, 159.2, 143.6, 139.7, 136.4, 129.8, 128.1, 126.7, 126.7, 122.3, 119.4, 119.3, 115.8, 97.5, 20.9

### Preparation Example 31: preparation of N-(4-(3-(4-hydroxyphenyl)isoxazole-5-yl)phenyl)acetamide

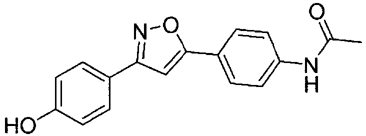

The procedure of Preparation Example 13 was repeated to obtain the target compound (10 mg, 70% yield), except that N-(4-(3-(4-methoxyphenyl)isoxazol-5-yl)phenyl)acetamide (15 mg, 0.05 mmol) prepared in Step 2) of Preparation Example 28 was used instead of N-(4-(5-(4-methoxyphenyl)isoxazol-3-yl)phenyl)-4-methylbenzenesulfonamide.

¹H NMR (DMSO-d₆, 800 MHz) δ 10.21 (s, 1H), 9.95 (s, 1H), 7.82 (d, J = 8.7 Hz, 2H), 7.75 (d, J = 8.7 Hz, 2H), 7.72 (d, J = 8.6 Hz, 2H), 7.33 (s, 1H), 6.89 (d, J = 8.6 Hz, 2H), 2.08 (s, 3H) ¹³C NMR (DMSO-d₆, 200 MHz) δ 169.1, 168.7, 162.3, 141.1, 128.1, 126.3, 121.6, 119.4, 119.1, 115.8, 97.1, 24.1

### Preparation Example 32: preparation of N-(4-(3-(4-hydroxyphenyl)isoxazole-5-yl)phenyl)-4-methylbenzamide

### Step 1) Preparation of N-(4-(3-(4-methoxyphenyl)isoxazol-5-yl)phenyl)-4-methylbenzamide

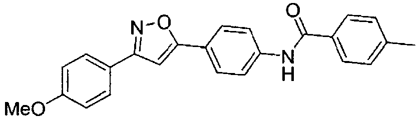

The procedure of Preparation Example 14 was repeated to obtain the target compound (13 mg, 60% yield), except that 4-(3-(4-methoxyphenyl)isoxazol-5-yl)aniline (15 mg, 0.06 mmol) prepared in Step 1) of Preparation Example 29 was used instead of 4-(5-(4-methoxyphenyl)isoxazol-3-yl)aniline.

¹H NMR (DMSO-d₆, 800 MHz) δ 10.42 (s, 1H), 7.99 (d, J = 8.7 Hz, 2H), 7.90 (d, J = 8.1 Hz, 2H), 7.89 (d, J = 8.7 Hz, 2H), 7.85 (d, J = 8.7 Hz, 2H), 7.45 (s, 1H), 7.36 (d, J = 7.9 Hz, 2H), 7.10 (d, J = 8.8 Hz, 2H), 3.84 (s, 3H), 2.40 (s, 3H) ¹³C NMR (DMSO-d₆, 200 MHz) δ 169.3, 165.6, 162.1, 160.7, 141.9, 141.2, 131.8, 129.0, 128.0, 127.8, 126.1, 122.0, 121.0, 120.4, 114.5, 97.4, 55.3, 21.0

### Step 2) Preparation of N-(4-(3-(4-hydroxyphenyl)isoxazol-5-yl)phenyl)-4-methylbenzamide

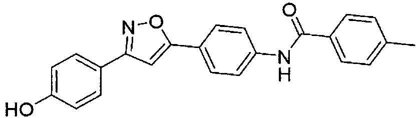

The procedure of Preparation Example 13 was repeated to obtain the target compound (65 mg, 63% yield), except that N-(4-(3-(4-methoxyphenyl)isoxazol-5-yl)phenyl)-4-methylbenzamide (108 mg, 0.28 mmol) prepared in Step 1) was used instead of N-(4-(5-(4-methoxyphenyl)isoxazol-3-yl)phenyl)-4-methylbenzenesulfonamide.

¹H NMR (DMSO-d₆, 800 MHz) δ 10.41 (s, 1H), 9.96 (s, 1H), 7.99 (d, J = 8.7 Hz, 2H), 7.90 (d, J = 8.0 Hz, 2H), 7.88 (d, J = 8.7 Hz, 2H), 7.73 (d, J = 8.6 Hz, 2H), 7.38 (s, 1H), 7.36 (d, J = 7.9 Hz, 2H), 6.90 (d, J = 8.6 Hz, 2H), 2.08 (s, 3H) ¹³C NMR (DMSO-d₆, 200 MHz) δ 169.1, 165.6, 162.3, 159.2, 141.9, 141.1, 131.8, 129.0, 128.1, 127.8, 126.1, 122.0, 120.4, 119.4, 115.8, 97.3, 21.0

### Preparation Example 33: preparation of N-(4-(1-(4-hydroxyphenyl)-1H-1,2,3-triazole-4-yl)phenyl)-4-methylbenzenesulfonamide

### Step 1) Preparation of 4-(4-(4-aminophenyl)-1H-1,2,3-triazol-1-yl)phenol

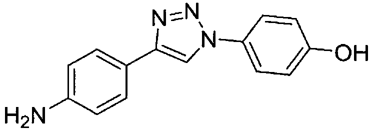

4-Ethynylaniline (100 mg, 0.85 mmol), 4-iodophenol (187.8 mg, 0.85 mmol), sodium azide (82.9 mg, 1.28 mmol), copper(I) iodide (32.4 mg, 0.17 mmol), L-proline (19.6 mg, 0.17 mmol), and sodium carbonate (135.1 mg, 1.28 mmol) were dissolved in dimethyl sulfoxide (3 mL). After heating to 70°C, the mixture was stirred for 12 hours. After the reaction was completed, water was added thereto dropwise. The mixture was extracted three times with a mixed solution of chloroform:isopropyl alcohol (4:1), dried over anhydrous magnesium sulfate, and concentrated under reduced pressure. The resulting residue was purified by flash column chromatography (ethyl acetate:n-hexane = 3:2), and the resulting solution was concentrated to obtain the target compound (114 mg, 80% yield).

¹H NMR (DMSO-d₆, 400 MHz) δ 9.92 (s, 1H), 8.91 (s, 1H), 7.68 (d, J = 8.8 Hz, 2H), 7.57 (d, J = 8.8 Hz, 2H), 6.94 (d, J = 8.8 Hz, 2H), 6.64 (d, J = 6.8 Hz, 2H), 5.27 (s, 2H)

### Step 2) Preparation of N-(4-(1-(4-hydroxyphenyl)-1H-1,2,3-triazol-4-yl)phenyl)-4-methylbenzenesulfonamide

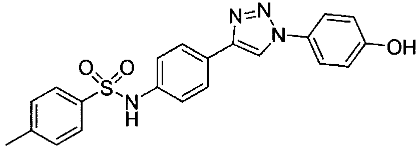

4-(4-(4-Aminophenyl)-1*H-*1,2,3-triazol-1-yl)phenol (50 mg, 0.2 mmol) prepared in Step 1) of Preparation Example 33 and pyridine (0.03 mL, 0.4 mmol) were dissolved in dichloromethane (2 mL), and 4-toluenesulfonyl chloride (45.3 mg, 0.24 mmol) and 4-dimethylaminopyridine (4.8 mg, 0.04 mmol) were added thereto dropwise. After stirring at room temperature for 12 hours, a 1 N aqueous hydrochloric acid solution was added thereto dropwise. The mixture was extracted three times with dichloromethane, dried over anhydrous magnesium sulfate, and concentrated under reduced pressure. The resulting residue was purified by flash column chromatography (ethyl acetate:n-hexane = 2:1), and the resulting solution was concentrated to obtain the target compound (40 mg, 50% yield).

¹H NMR (DMSO-d₆, 800 MHz) δ 10.16 (s, 1H), 8.90 (s, 1H), 7.73 (d, J = 8.5 Hz, 2H), 7.65 (d, J = 8.2 Hz, 2H), 7.64 (d, J = 8.8 Hz, 2H), 7.33 (d, J = 8.2 Hz, 2H), 7.15 (d, J = 8.5 Hz, 2H), 6.93 (d, J = 8.9 Hz, 2H), 2.30 (s, 3H)

### Preparation Example 34: preparation of N-(4-(1-(4-methoxyphenyl)-1H-1,2,3-triazole-4-yl)phenyl)acetamide

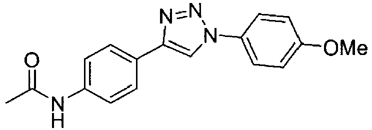

N-(4-ethynylphenyl)acetamide (50 mg, 0.31 mmol), 1-azido-4-methoxybenzene (42 mg, 0.28 mmol), copper(II) sulfate pentahydrate (12 mg, 0.05 mmol), and sodium ascorbate (28 mg, 0.14 mmol) were dissolved in tetrahydrofuran:water (1:1, 3 mL). After stirring at room temperature for 12 hours, a 1 N aqueous hydrochloric acid solution was added thereto dropwise. The mixture was extracted three times with ethyl acetate, dried over anhydrous magnesium sulfate, and concentrated under reduced pressure. The resulting residue was purified by flash column chromatography (ethyl acetate:n-hexane = 1:1), and the resulting solution was concentrated to obtain the target compound (45 mg, 46% yield).

¹H NMR (DMSO-d₆, 800 MHz) δ 10.06 (s, 1H), 9.09 (s, 1H), 7.85-7.83 (m, 4H), 7.69 (d, J = 8.6 Hz, 2H), 7.17 (d, J = 9.0 Hz, 2H), 3.84 (s, 3H), 2.07 (s, 3H) ¹³C NMR (DMSO-d₆, 200 MHz) δ 168.4, 159.2, 147.00, 139.2, 130.1, 125.7, 125.1, 121.6, 119.2, 118.9, 114.9, 55.6, 24.0

### Preparation Example 35: preparation of N-(4-(5-(4-hydroxyphenyl)-1H-pyrazol-3-yl)phenyl)-4-methylbenzenesulfonamide

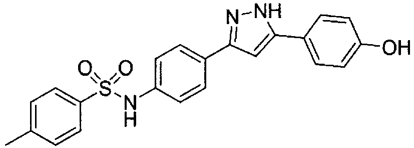

*(E)-N-(4-(3-(4-hydroxyphenyl)acrylyl)phenyl)-4-methylbenzenesulfonamide* (TSAHC) (100 mg, 0.25 mmol) and hydrazine hydrate (0.03 mL, 0.51 mmol) were dissolved in ethanol (2.5 mL). The mixture was heated to 70°C and stirred for 12 hours. After the reaction was completed, water was added thereto dropwise. The mixture was extracted three times with ethyl acetate, dried over anhydrous magnesium sulfate, and concentrated under reduced pressure. The resulting residue was purified by flash column chromatography (ethyl acetate:n-hexane = 2:1), and the resulting solution was concentrated to obtain the target compound (45 mg, 44% yield).

¹H NMR (DMSO-d₆, 800 MHz) δ 13.05 (s, 1H), 10.32 (s, 1H), 9.78 (s, 1H), 7.65-7.63 (m, 4H), 7.57 (d, J = 8.2 Hz, 2H), 7.33 (d, J = 8.2 Hz, 2H), 7.11 (d, J = 8.5 Hz, 2H), 6.87 (s, 1H), 6.80 (d, J = 8.5 Hz, 2H), 2.30 (s, 3H)

### Preparation Example 36: preparation of N-(4-(5-(4-hydroxyphenyl)-4,5-dihydroisoxazole-3-yl)phenyl)-4-methylbenzenesulfonamide

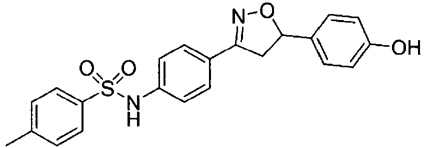

*(E)-N-(4-(3-(4-hydroxyphenyl)acrylyl)phenyl)-4-methylbenzenesulfonamide* (TSAHC) (100 mg, 0.25 mmol) and hydroxylammonium chloride (35.3 mg, 0.51 mmol) were dissolved in ethanol (2.5 mL). The mixture was heated to 70°C and stirred for 12 hours. After the reaction was completed, water was added thereto dropwise. The mixture was extracted three times with ethyl acetate, dried over anhydrous magnesium sulfate, and concentrated under reduced pressure. The resulting residue was purified by flash column chromatography (ethyl acetate:n-hexane = 1:2), and the resulting solution was concentrated to obtain the target compound (32 mg, 30% yield).

¹H NMR (DMSO-d₆, 800 MHz) δ 9.68 (s, 1H), 7.66 (d, J = 8.3 Hz, 2H), 7.52 (d, J = 8.7 Hz, 2H), 7.33 (d, J = 8.1 Hz, 2H), 7.16-7.12 (m, 4H), 6.73 (d, J = 8.6 Hz, 2H), 5.52 (dd, J = 10.6, 9.1 Hz, 1H), 4.67-4.65 (m, 1H), 3.22 (dd, J = 17.1, 8.9 Hz, 1H), 2.30 (s, 3H)

### Preparation Example 37: preparation of N-(4-(5-(4-methoxyphenyl)-4,5-dihydroisoxazole-3-yl)phenyl)acetamide.

### Step 1) Preparation of tert-butyl (4-(5-(4-methoxyphenyl)-4,5-dihydroisoxazol-3-yl)phenyl)carbamate

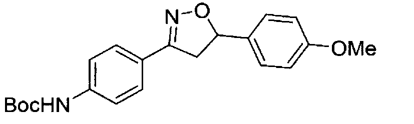

4-vinylstyrene (0.11 mL, 0.83 mmol), (diacetoxyiodo)benzene (266 mg, 0.83 mmol), and trifluoroacetic acid (0.015 mL) were dissolved in methanol (10 mL), and tert-butyl (E)-(4-((hydroxyimino)methyl)phenyl)carbamate (150 mg, 0.64 mmol) prepared in Step 1) of Preparation Example 1 was slowly added thereto dropwise. After stirring at room temperature for 12 hours, a saturated aqueous sodium bicarbonate solution was added thereto dropwise. The mixture was extracted three times with ethyl acetate, dried over anhydrous magnesium sulfate, and concentrated under reduced pressure. The resulting residue was purified by flash column chromatography (ethyl acetate:n-hexane = 1:4), and the resulting solution was concentrated to obtain the target compound (154 mg, 66% yield).

¹H NMR (DMSO-d₆, 800 MHz) δ 9.59 (s, 1H), 7.59 (d, J = 8.8 Hz, 2H), 7.53 (d, J = 8.6 Hz, 2H), 7.31 (d, J = 8.7 Hz, 2H), 6.94 (d, J = 8.7 Hz, 2H), 3.78-3.73 (m, 4H), 3.31 (m, 1H), 1.48 (s, 9H)

### Step 2) Preparation of N-(4-(5-(4-methoxyphenyl)-4,5-dihydroisoxazol-3-yl)phenyl)acetamide

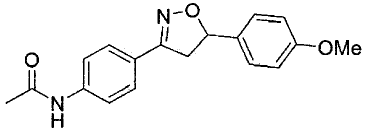

The procedure of Preparation Example 1, Step 3) was repeated to obtain the target compound (51 mg, 70% yield), except that tert-butyl (4-(5-(4-methoxyphenyl)-4,5-dihydroisoxazol-3-yl)phenyl)carbarmate (100 mg, 0.27 mmol) prepared in Step 1) was used instead of tert-butyl (4-(5-(4-methoxyphenyl)isoxazol-3-yl)phenyl)carbamate.

The procedure of Preparation Example 10 was repeated to obtain the final target compound (48 mg, 83% yield), except that the obtained 4-(5-(4-methoxyphenyl)-4,5-dihydroisoxazol-3-yl)aniline (50 mg, 0.186 mmol) was used instead of 4-(5-(4-methoxyphenyl)isoxazol-3-yl)aniline.

¹H NMR (DMSO-d₆, 800 MHz) δ 10.13 (s, 1H), 7.66 (d, J = 8.8 Hz, 2H), 7.63 (d, J = 8.8 Hz, 2H), 7.32 (d, J = 8.7 Hz, 2H), 6.94 (d, J = 8.7 Hz, 2H), 5.63 (dd, J = 10.6, 9.0 Hz, 1H), 3.77 (dd, J = 16.9, 10.7 Hz, 1H), 3.75 (s, 3H), 3.33 (d, J = 9.3 Hz, 1H), 2.06 (s, 3H) ¹³C NMR (DMSO-d₆, 200 MHz) δ 168.6, 159.1, 156.1, 140.9, 132.7, 127.7, 127.3, 123.8, 118.8, 113.9, 81.7, 55.1, 41.9, 24.1

### Experimental Example 1: Experimental Preparation

### 1.1. Cell culture

Human hepatocellular carcinoma cell lines that do not express TM4SF5, such as SNU449, SNU761, or Huh7_{KO} (a cell line in which the TM4SF5 gene was removed using the CRISPR/Cas9 system), were infected with a retrovirus expressing TM4SF5 or transfected with cDNA to create cell lines (SNU449Tp, SNU449T₇, SNU449-TM4SF5, SNU761-TM4SF5, or Huh7_{KO}-TM4SF5), and cell lines infected with a control virus or transfected with a control empty vector (SNU449Cp, SNU449-EV, SNU761-EV, or Huh7_{KO}-EV) were cultured in DMEM or RPMI-1640 medium (WelGene, Daegu, Republic of Korea) containing 10% FBS and antibiotics (Invitrogen, CA, USA) in a cell incubator at 37°C and 5% CO₂. For the human natural killer (NK cell) NK92 cell line, α-MEM (Invitrogen) medium containing 200.0 U/ml of recombinant human IL-2 (Peprotech), 12.5% FBS, and 12.5% fetal horse serum (GenDEPOT) was used. NK92-TM4SF5-HA was created by infecting NK92 cells with a retrovirus expressing TM4SF5-HA. HEK293FT or human Jurkat T cells were cultured in RPMI-1640 medium supplemented with 10% FBS.

### 1.2. Transfection (Infection) and inhibition of expression

In some cases, wildtype (WT) or mutant cDNAs of TM4SF5, SLAMF7, or PD-L1 contained in an empty vector (EV) or mammalian expression plasmids were transfected into cell lines using Lipofectamine 3000 (Thermo Fisher Scientific Inc.) and PEI (Polysciences), or lentiviruses obtained by infecting host cells with lentiviral vectors containing an empty vector or cDNA were infected, to artificially express the genes in the cell lines under study.

### 1.3. Evaluation of inhibition of cell survival/proliferation (sphere growth) in a 3D Aqueous Culture Environment

TM4SF5-non-expressing cells (SNU449Cp) and TM4SF5-expressing cells (SNU449T₇) of the same number (200 cells/well or 1000 cells/well) were added per well into Costar Ultra-Low Attachment Multiple Well Plates (CORNING), and were then treated with vehicle (DMSO) or drugs at various concentrations and cultured in a cell incubator under conditions of 37°C and 5% CO₂. After repeatedly treating with the same concentrations of drugs every few days at regular intervals as indicated from the date of cell culture, the proliferation of the cells (spheres) was observed and photographed using an optical microscope on the last day of observation.

### 1.4. Analysis of cell migration path and migration function

SNU449-TM4SF5 cells, which were made to express TM4SF5 using a lentivirus in human hepatocellular carcinoma cells SNU449, and SNU449-EV cells, into which a control vector (empty vector, EV) was injected so that TM4SF5 is not expressed, were seeded to a density of 40% in 8-chamber plates (Thermo) coated with 10 µg/ml collagen type 1 (Advanced Biomatrix). Three hours after seeding in a cell culture environment containing 10% FBS (GenDEPOT), DMSO and drugs were treated in the culture medium at the indicated concentrations. After treating with the drugs, the positions of the same moving cells were repeatedly photographed using a microscope every 20 minutes for 17 hours. The cells were photographed using an IX81-ZDC microscope (Olympus) equipped with a UPLSAPO 10X2, NA0.4 Super Apochromatic objective lens (Olympus). While photographing the cells, a general cell culture environment was created by constantly injecting 5% CO₂ at a rate of 40-60 mL/min at 37°C using a controller (Live Cell Instrument). The migration distance and path of the cells were calculated using the "Track Object" application of MetaMorph software (Molecular Devices LLC). As a result of measuring the migration paths of several cells at the same time, the original position of each cell was set as the origin of concentric circles, and the migration paths were indicated by lines of different colors, thereby representing the degree of cell migration.

### 1.5. Preparation of mouse in which the mouse TM4SF5 gene is overexpressed only in hepatocytes

For details related to the production of mice described in Experimental Examples 1.5 and 1.6, refer to Korean Patent Application Nos. 10-2022-0007048, 10-2021-0171614, and 10-2021-0150240 filed by the present inventors. However, the description of the mouse production described in the Korean patent applications is summarized as follows.

A plasmid made to express the mouse Tm4sf5 gene (NM_029360) in hepatocytes, which have high albumin expression, by linking it to an albumin promoter sequence was microinjected into C57BL/6 mouse fertilized eggs. Two-week-old founder mice were confirmed for a 689 bp product by PCR using (ALB Forward-CAGCTTGGCTTGAACTCGTTC-3', TM4SF5 Reverse-CAATTCCTGGACACAGCACCA-3'), and then produced by identifying overexpressed mice (pALB-mTm4sf5-(Flag)₃-transgenic mouse: *Alb*-Tg^{Tm4sf5-Flag}).

### 1.6. Preparation of Mouse in which the TM4SF5 gene is knocked out (KO)

Cas9/RGEN KO mice were produced using C57BL/6 mice, and at this time, mice in which various 29 bp were deleted were obtained using RGEN sites (sgRNA sequences for targeting mouse *Tm4sf5* Exon 1: sgRNA1 5'-GAGGTTGCCGTCCGTCCAGGTGG-3', sgRNA2 5'-GCTGAGGTTGCCGTCCGTCCAGG-3') [custom-made by Macrogen Co., Ltd.]. A 556 bp PCR product was confirmed using mouse TM4SF5 primers (Forward, 5'-ACTTCCTCAGGGCCTCTCTC-3'; Reverse, 5'-CCTTTCCCACATTCCTCAGA-3'), and mutant mice were found by observing heteroduplex formation between WT/mutant PCR products through T7E1 assay, and accurately knocked-out mice were used for experiments. At this time, we performed experiments with (*Tm4sf5* Exon1^{-/-}) in which 29 bp including Exon 1, where Threonine at amino acid position 36 at which the extracellular loop 2 of mouse TM4SF5 begins exists, is deleted.

### 1.7. Establishment of non-alcoholic steatohepatitis (NASH) disease mouse model through MCD (methionine-choline-deficient) diet

Wildtype (WT), *Alb*-TG^{Tm4sf5-Flag}, and *Tm4sf5*^{*-*/*-*} (*Tm4sf5* gene-deficient) KO C57BL/6N mice were raised in a pathogen-free room animal facility where temperature and humidity were well controlled. For the experiment to establish the steatohepatitis NASH disease model, 22-week-old mice were used. For the induction of hepatic steatohepatitis through an MCD (TD.90262, Teklad) diet, 6-week-old male mice were randomly divided into normal chow diet (NCD, Purina, Cat # 38057) and MCD groups and allowed free access to the diet for 4 weeks (7 mice per group). When treating with drugs, intraperitoneal injection was performed at a concentration of 5 mg/kg twice a week. After the end of the experiment, liver tissues were obtained and analyzed through methods of H&E, immunohistochemistry tissue staining, Western blot, and qRT-PCR.

### 1.8. Liver cancer pathogenesis model using DEN drug

A liver cancer model was induced in 2-week-old male wild type (WT), *Alb-*TG^{Tm4sf5-Flag} [genetically modified animals in which Tm4sf5 expression is excessively regulated by the promoter of the *Albumin* gene, custom-made by Macrogen Co., Ltd.], or *Tm4sf5*^{*-*/*-*} KO C57BL/6N male animals by a single intraperitoneal injection (IP injection) of DEN (diethylnitrosamine, Sigma-Aldrich) at a concentration of 25 mg/kg, followed by raising for 10 months. After the DEN treatment, the animals were raised in a pathogen-free room animal facility where temperature and humidity were well controlled under a normal diet for 40 weeks. During the last 6 weeks, an isoxazole drug (dissolved in 40% DMSO) at the indicated concentration was treated by intraperitoneal injection twice a week. Forty-six weeks after the DEN injection, the animals were sacrificed, and liver tissues were analyzed through methods of tissue staining, Western blot, and qRT-PCR.

### 1.9. Evaluation of anticancer efficacy of isoxazole compounds in a subcutaneous xenograft model of TM4SF5-expressing liver cancer cell lines

Cells that do not express TM4SF5 (SNU449Cp) or cells that express TM4SF5 (SNU449T₇) were cultured in a cell incubator at 37°C and 5% CO₂. The next day, the cells were detached by treating with a T/E (Trypsin/EDTA) solution and centrifuged at 150x g for 3 minutes to obtain a pellet. Thereafter, the supernatant was removed, and the pellet was prepared by resuspension in phosphate buffered saline (PBS). For the generation of liver cancer, 10⁷ cells/100 µl were injected once subcutaneously into the right thigh area of 8-week-old male BALB/c-Nude mice. At this time, the animals were anesthetized by inhalation with 20% isoflurane and injected. From the 7th day after the cell injection, drugs ST-2-001, ST-3-006, and ST-3-011 were each intraperitoneally injected at 5 mg/kg of body weight for a total of 8 times every 3 days. The body weight of the animals was measured every week from the day of cell injection, and the tumor volume was measured every 3 days from the 7th day after the cell injection. The tumor volume was calculated as (length×width×width)/2 (mm³) by measuring the length and width of the tumor. Finally, on the 31st day after the cell injection, the animals were sacrificed by a standard method, and liver tissues were obtained to analyze the liver cancer tissues.

### 1.10. Evaluation of anticancer efficacy of isoxazole compounds in a liver-orthotopic xenograft model of TM4SF5-expressing liver cancer cell lines

Cells that do not express TM4SF5 (SNU449Cp) or cells that express TM4SF5 (SNU449T₇) were cultured in a cell incubator at 37°C and 5% CO₂. The next day, the cells were detached by treating with a T/E (Trypsin/EDTA) solution and centrifuged at 150x g for 3 minutes to obtain a pellet. Thereafter, the supernatant was removed, leaving only the pellet. After mixing with matrigel matrix (CORNING) such that the final concentration of EGF was 50 ng/µl, the cell pellet was prepared by resuspension in the mixture of EGF and matrigel. Then, 5x10⁵ cells/20 µl were directly injected once into the liver of 6-week-old male BALB/c-Nude mice. At this time, the animals were anesthetized by inhalation with 20% isoflurane and injected. From one week after the cell injection, drug ST-5-002 was intraperitoneally injected at 5 mg/kg twice a week. After injecting the drug for a total of 6 times over 3 weeks, the animals were sacrificed at the 4th week of cell injection, and liver tissues were obtained and analyzed.

### 1.11. Evaluation of anticancer efficacy of isoxazole compounds in a TM4SF5-expressing PDX (patient-derived tissue xenograft) animal model

The experiment was conducted by obtaining patient-derived liver cancer segments (P0; HCC cube, 1 mm³) confirmed to express TM4SF5 from Seoul National University Hospital, which passed the normal IRB approval process (H-2102-059-1195). The hair from the right thigh to the flank of 6-week-old male NOD/SCID mice was removed, and the mice were anesthetized by inhalation with 20% isoflurane. Thereafter, a cancer segment was implanted into the right axillary region of the mice. When the cancer segments were engrafted and grew beyond a certain size at 4 to 5 weeks after implanting the tissue, the animals were sacrificed to obtain cancer segments (P1). The cancer segments were cut into a size of 1 mm³ and placed in cryotubes with Recovery-Cell Culture Freezing Medium (Gibco) and stored in a -80°C liquid nitrogen tank. After obtaining P2 and P3 cancer segments in the same manner as above, one P3 cancer segment (1 mm³) was implanted per 6-week-old male NOD/SCID mouse to establish the HCC-PDX mouse model used in this experiment. From the 8th day after implanting the cancer segments, vehicle, drug ST-5-001, or ST-5-002 was intraperitoneally injected at 5 mg/kg twice a week for a total of 6 times. On the 28th day after implanting the cancer tissue, the animals were sacrificed, and liver cancer tissues were obtained and used for analysis.

### 1.12. Evaluation of anticancer efficacy of isoxazole compounds in subcutaneous xenograft of TM4SF5 expressing liver cancer cell lines using severely combined immunodeficient animals

Cells expressing TM4SF5 (SNU449T₇) were cultured in a cell incubator at 37°C and 5% CO₂. The next day, the cells were detached by treating with a T/E (Trypsin/EDTA) solution and centrifuged at 150 × g for 3 minutes to obtain a cell pellet. Thereafter, the supernatant was removed, and the pellet was prepared by resuspension in phosphate buffered saline (PBS). For the generation of cancer tissue, 5 × 10⁵ cells/100 µl were injected once subcutaneously into the right thigh area of 6-week-old male NOD/SCID mice. At this time, the animals were anesthetized by inhalation with 20% isoflurane and injected. From the 15th day after the cell injection, drug ST-5-002 was intraperitoneally injected at 2.5 mg/kg of body weight at 100 µl per dose for a total of 8 times every 3 days. The body weight and tumor volume of the animals were measured every 3 days from the 12th day after the cell injection. The tumor volume was calculated as (length × width × width)/2 (mm³) by measuring the length and width of the tumor. On the 36th day after the cell (SNU449T₇) injection, the animals were sacrificed, and cancer tissues were obtained and analyzed.

### 1.13. Western blotting of cell and tissue extracts

Cell extracts were obtained from cells grown to a density of 70-80% without any treatment, or from cells treated with vehicle DMSO or drugs at the given concentrations for 24 hours. For the extract of hepatocytes or liver cancer cells, a lysis buffer (150 mM NaCl, 1% NP-40, 50 mM Tris-HCl, 0.25% sodium deoxycholate, pH 7.4) including a protease inhibitor cocktail (GenDEPOT) was used. In addition, after washing animal liver tissue twice with PBS, SDS, Na₃VO₄, and protease inhibitor cocktails (GenDEPOT) were added to a lysis buffer [50 mM Tris-HCl, pH 7.4, 1% NP40, 0.25% sodium deoxycholate, 150 mM NaCl, 1 mM EDTA (based on 500 ml)], followed by treatment at 4°C for 15 minutes. After centrifugation at 13,000 rpm at 4°C for 30 minutes, only the supernatant was transferred to a new microcentrifuge tube. After quantification using BCA reagent (Thermo Scientific), a 4 × sample buffer [100% glycerol 4 ml, Tris-HCl, pH 6.8, 2.4 ml, SDS 0.8 g, Bromophenol blue 4 mg, betamercaptoethanol 0.4 ml, H₂O 3.1 ml (based on 10 ml)] was added, followed by boiling at 100°C for 5 minutes. After performing SDS-PAGE electrophoresis with the above samples and transferring them to Nitrocellulose Membranes Protran^{™} nitrocellulose membranes (Whatman), pre-treatment was performed with 5% skim milk for 1 hour. After pre-treatment, primary antibodies of p-FAK(Y861) (SC-16663), p-FAK(Y577) (SC-16665), c-Src (SC-8056), STAT3 (SC-8019), p-AKT 1/2/3(S473) (SC-7985), STATS (SC-835), Laminin γ2 (SC-393225), COL1A1 (SC-28657), CCL5(RANTES) (SC-514019) CCL20(MIP-3α) (SC-517441), SIRT1 (SC-74465), ChREBP (SC-515922), SREBP1 (SC-366513), DGAT1 (SC-271934), DGAT2 (SC-293211), MICA/B (SC-137242), SLAMF7 (SC-390840), α-tublin (SC-5286), β-actin (SC-47778), FUCA (SC-365496) (the above from Santa Cruz Biotech, USA), p-FAK(Y397) (cat # 611723), FAK (cat # 610088), p27 (cat # 610242) (the above from BD Transduction, USA), p-FAK(Y925) (#3284) p-c-Src(Y416) (#2101), p-STAT3(Y705) (#9145), p70S6K1 (#9202), p-p70S6K1(T389) (#9205), p-STAT5(Y694) (#9314), ACC (#3676), FASN (#3189), CPT1A (#12252), p-ACC(S79) (#11818), HA (#3724), PD-L1 (#13684), PD-1 (#86163), p-ERK1/2(T202/Y204) (#9101s), ERK1/2 (#9102s) (the above from Cell Signaling Tech., USA), pS10p27(S10) (ab62364), human SLAMF6 (ab224201), TIGIT (ab233404), CD34 (ab81289) (the above from Abcam, UK), mouse SLAMF6 (PA5-87823), NKG2D (PA5-102038) (the above from Thermofisher, USA), α-SMA (Sigma Aldrich, A5228), CCL2 (Invitrogen, MA5-17040), and CD155(PVR) (Biolegend, 337601), HA (Biolegend, USA), Strep-HRP(horse-radish peroxidase), StrepMAB-HRP (IBA, USA), AFP (FineTest, FNab00203) were reacted at 4°C for 15 hours. The next day, secondary antibodies were reacted, and development was performed on X-ray film using ECL (Pierce, USA). Anti-TM4SF5_{C-ter} (epitope region of RKKQDTPH¹⁹⁷) and anti-TM4SF5_{EC2} (CLMNGEWGYHFEDTAGA¹³⁰) were custom-made at a professional antibody production company (Pro-Sci, Poway, CA, USA).

### 1.14. Immunofluorescence microscopy for quantitative analysis of localization changes

After putting a cover glass into each well of a 12-well plate, 10 µg/ml fibronectin (35600, BD Biosciences) diluted in PBS was added thereto and left at room temperature for 1 hour for coating. After washing three times with PBS, 20,000 cells were seeded on each cover glass and cultured in a cell incubator at 37°C for 16 hours. After cultivation, the various expression vectors mentioned above were transfected using Lipofectamine 3000 (L3000015, Invitrogen) and cultured for 24 hours, and then isoxazole was treated or not treated for 24 hours. The cells were fixed with cold 99% methanol at room temperature for 15 minutes and then blocked with 1% BSA in PBS for 1 hour. Primary antibodies, anti-FLAG (NB600-344, Novus Biologicals), anti-LAMP1 (#9091, Cell Signaling Tech,), anti-ZO-1 (402200, Invitrogen), anti-HA (901515, BioLegend), and anti-SLAMF7 (sc-390840, Santa Cruz Biotech.), were diluted in 1% BSA in PBS at a ratio of 1:500 and reacted at 4°C for 16 hours. The next day, the cover glasses were washed three times with PBS, and then fluorescence-conjugated secondary antibodies (Alexa Fluor 488 [goat: A11055 and mouse: A21202] and 555 [goat: A21432 and mouse: A31570] from Invitrogen) were diluted in 1% BSA in PBS at a ratio of 1:500 and reacted at room temperature for 1 hour. After washing three times with PBS, mounting was performed using ProLong^{™} Gold Antifade (P36930, Invitrogen). Cells were randomly photographed using a Nikon Eclipse Ti microscope with a C2 confocal system, and then the images were analyzed using NIS-Elements software (Nikon, Melville, NY, USA), and colocalization of SLAMF7 with LAMP1 or ZO-1 was calculated using Pearson's correlation coefficients. All images were photographed under the same software settings and equally corrected with Adobe Photoshop. Each dot in the graphs represents a result measured from a single cell.

### 1.15. Immunoprecipitation assay

Cells were cultured to ~60% density in a cell incubator at 37°C and 5% CO₂, and then transfected using PEI and cultured in a cell incubator at 37°C for two days. After cultivation, cell extracts were obtained by the method described above. After precipitating a desired protein from the cell extracts using an immunoprecipitation method, the binding proteins were identified by the Western blot technique described above. Specifically, cells cultured in a 100 mm cell culture dish for two days were washed twice with PBS, and protease inhibitor cocktails (GenDepot, USA) were added to 500 µl of immunoprecipitation lysis buffer (0.5% Triton X-100 or Brij 58, 40 mM HEPES, 150 mM NaCl, 1 mM EDTA, pH 7.4), followed by treatment at 4°C for 15 minutes. After collecting the cells, centrifugation was performed at 12,000x g at 4°C for 15 minutes, and the supernatant was transferred to a new microcentrifuge tube to obtain a cell extract. Proteins therein were quantified using a BCA reagent (Thermo Scientifics, USA). An electrophoresis sample was prepared by adding 4x laemmli sample buffer to a portion of the quantified sample and boiling at 100°C for 5 minutes. For immunoprecipitation, Streptavidin Agarose Resin (Thermo Fisher Scientific) or anti-HA antibody-coated Sepharose beads were added in proportion to the quantified protein amount, followed by rotation at 4°C for 4 hours, and then centrifugation was performed at 7,000x g at 4°C for 5 minutes and the supernatant was discarded. After adding a sufficient amount of clean lysis buffer and mixing lightly, centrifugation was performed again at 7,000x g at 4°C for 5 minutes and the supernatant was discarded. This process was repeated two more times with lysis buffer and two more times with cold PBS, and then 2x sample buffer was added followed by boiling at 100°C for 5 minutes. Western blot was performed with the samples thus prepared using the various primary antibodies described above. Primary antibodies of EGFR (SC-03), CD44 (SC-7287), IL-6Rα (SC-661), MICA/B (SC-137242), SLAMF7 (SC-390840) (the above from Santa Cruz Biotech, USA), CD133 (#5860), mTOR (#2983), GLUT1 (#12939), GLUT4 (#2213), FLAG (#2368), PD-L1 (#13684) (Cell Signaling Technology, USA), SLAMF6 (ab224201), TIGIT (ab233404), PD-L1 (#205921) (Abcam, UK), or Integrin α5 (Merck Millipore, MAB1956Z) were used. Thereafter, Western blot was performed by reacting with an appropriate secondary antibody solution.

### 1.16. Real-time reverse transcription PCR (qRT-PCR)

After disrupting cells or tissues using Qiazol (Qiagen, USA), chloroform was added thereto, and centrifugation was performed at 12,000x g and 4°C for 15 minutes for phase separation, followed by separating the upper organic layer. After adding isopropanol to the separated organic layer to precipitate RNA, it was washed with 70% ethanol. An RNA pellet was separated by centrifugation at 7,500x g for 5 minutes, and ethanol was evaporated for 10 minutes, followed by solubilization in 30 µl of DEPC-water. The solubilized RNA was reverse-transcribed using a reverse transcription kit (Toyobo, Japan) after removing gDNA to obtain cDNA. For the obtained cDNA, expression was measured by real-time PCR (Bio-Rad, USA) using 2x Eva green master mix (Labopass, Republic of Korea) and 0.4 µM forward/reverse primers. The expression level was calculated using Pfaffl's modified delta-delta Ct method. The sequences of primers for qRT-PCR are listed in Table 1.

### 1.17. H&E staining

Paraffin blocks were micro-sectioned and placed on slides, left at 60°C for about 20 minutes for fixation, and then immersed in xylene three times for 5 minutes each to separate the tissue from the paraffin. After being placed in the order of 100%→90%→80%→70% ethanol→distilled water for 3 minutes each, they were reacted by immersing in a hematoxylin solution for 5 minutes. After washing sufficiently with tap water, they were reacted with an eosin solution for about 5 minutes and washed again with tap water. Then, after undergoing a dehydration process by immersing in the order of 70%→80%→90%→100% ethanol→xylene for 3 minutes each, the slides were mounted.

### 1.18. Masson's Trichrome staining

Liver tissues were stained using a Trichrome Stain Kit (Connective Tissue Stain) (ab150686, Abcam, UK). After first separating the tissues from paraffin, they were reacted with a pre-heated bouin's solution for 1 hour. Then, after rinsing with tap water until the solution was completely removed, they were reacted with a hematoxylin solution for 10 minutes and rinsed again with tap water. Thereafter, they were reacted with a biebrich scarlet-acid fuchsin solution for 5 minutes, immersed in distilled water, and then reacted with a phosphotungstic/phosphomolybdic acid solution for 15 minutes. They were immersed in an aniline blue solution for 10 minutes and 1% acetic acid for 1 minute, and the tissues were dehydrated and immersed in xylene, followed by mounting using slides.

### 1.19. Immunohistochemistry

After performing experiments according to IRB approval, immunohistochemistry was performed on animal liver tissues using various primary antibodies. Paraffin blocks and liver tissue sections were obtained through the contract service of Abion Inc. (Seoul, Korea) or directly stained by the standard method described above. For antibodies, those against COL1A1 (SC-28657), Laminin γ2 (SC-393225), p-STAT3(Y705) (9145), Ki67 (SC-23900), CCL20(MIP-3α) (SC-517441), CCL5(RANTES) (SC-514019), human/mouse SLAMF7(CS1) (sc-390840) (the above from Santa Cruz Biotech, USA); ACC (#3676), FASN (#3189), F4/80 (#70076), PD-L1 (#13684), PD-1 (#86163), and human TIGIT (#99567) (the above from Cell Signaling Technology, USA); human/mouse NKG2D (PA5-102038, Thermo Fisher, USA); mouse Tigit (ab233404, Abcam, UK); AFP (FineTest, FNab00203); CCL2 (Invitrogen, MA5-17040); and human TM4SF5-EC2 (Commercial product, Pro-Sci, USA) were used.

### 1.20. Flow cytometry analysis of NK92 cell lines

NK92-TM4SF5 stably expressing TM4SF5-HA was established by infecting the human NK92 cell line with a control expression vector (empty vector, EV) or a lentivirus expressing TM4SF5-HA. NK cells were obtained by centrifuging the NK92-EV and NK92-TM4SF5 cell lines under culture at 150 × g for 5 min. For drug treatment, vehicle- or drug-mixed culture media were prepared by well-mixing DMSO or drugs at the given concentrations into a cell culture medium (α-Minimum Essential Medium (MEM) (Invitrogen, Grand Island, NY, USA) containing 200 U/mL recombinant human interleukin (IL)-2 (PeproTech, Rocky Hill, NJ, USA), 12.5% FBS, and 12.5% fetal horse serum (GenDEPOT Inc.)). After centrifugation, the cell culture medium was removed using a suction device, and the isolated NK cells were resuspended in the vehicle- or drug-mixed culture medium, and the same number of cells were respectively placed in culture vessels to start normal cultivation.

24 hours after treating with the drugs, NK cells were isolated through centrifugation at 150 × g for 5 min. The upper culture medium was removed, and the cells were washed twice with PBS. Among the NK cells corresponding to each experimental group, 1 × 10⁶ cells were obtained and stained with a SLAMF7 antibody conjugated with fluorescent dye PE-Cy7 (PE/Cyanine7 anti-human CD319, Cat: 331816) for 20 minutes at room temperature in the dark. A MACSQuant Analyzer 10 (Miltenyi Biotec) was used to confirm changes in the expression of SLAMF7 when NK92-EV or NK92-TM4SF5 cells were treated with DMSO or drugs.

### 1.21. Evaluation of natural killer efficacy of NK cells

When human liver cancer cells Huh7 cells filled the culture vessel to 60-70%, the Cas9 gene was injected into the cells using pSpCas9(BB)-2A-Puro (PX459) V2.0 (Plasmid #62988, Addgene) with polyethylenimine (408727, Sigma-Aldrich). Thereafter, 5'-GGGCCACTAGGGACAGGAT-3' was used as a control gRNA sequence for AAVS1 (adeno-associated virus integration site 1), and 5'-TCCGGGGATTGCAGCCGTT-3' (#1), 5'-ATTGCAGCCGTTCGGGCAG-3' (#2), and 5'-GATTGCAGCCGTTCGGGCA-3' (#3) were used as gRNA sequences for TM4SF5 (exon 2) to knock out (KO) the TM4SF5 gene. After 24 hours, the cell culture medium was treated with puromycin at a concentration of 2 µg/ml for 3 days to select KO cells. Thereafter, stable cell lines were established by infecting the Huh7KO cells in which the TM4SF5 gene was knocked out with a retrovirus inducing expression of pBabe-HA-EV or pBabe-HA-TM4SF5. Then, Huh7KO-EV cells or Huh7KO-TM4SF5 cells (Target cells, T) were suspended in 50 µl of 1% FBS DMEM, and NK92-EV or NK92-TM4SF5 cells (Effector cells, E) were suspended in 1% FBS in MEM-α to adjust the cell number (0.015625 × 10⁵, 0.03125 × 10⁵, 0.0625 × 10⁵, and 0.125 × 10⁵ cells/mL), and E:T ratios of 1.25:1, 2.5:1, 5:1, and 10:1 were mixed in a 96-well plate in triplicate and cultured for 16 hours under conditions of 37°C and 5% CO₂. Thereafter, to evaluate the natural killer efficacy of the NK cells, a lactate dehydrogenase (LDH) cytotoxicity assay kit (Cytotoxicity Detection Kit Plus, Roche) was used. LDH activity values were measured at wavelengths of 492 and 690 nm using a microplate reader (SpectraMax i3x, Molecular Devices). The killing efficacy value was calculated according to the manufacturer's instructions as follows: [LDH (effector-target cell mix)LDH (effector cell)LDH (target cell low control)]/[LDH (target cell high control)LDH (target cell low control)] ×100.

### 1.22. Adoptive transfer model

Severely combined immunodeficient NOD-SCID mice (NOD.CB17-Prkdcscid/J, Orient Bio Inc., Seongnam-si, Korea) were used for adoptive transfer with NK92 cells. At 6 weeks of age, 5 X 10⁵ SNU449T7 cells were injected subcutaneously. Starting 7 days after the SNU449T7 cell injection, NK92-EV cells and NK92-TM4SF5 cells were injected into the tail vein at a dose of 5 X 10⁶ cells. NK cells were injected once a week for a total of five times, and tumor size was measured every 3 days. One day after the fifth injection of NK92 cells, the mice were sacrificed, and liver tissues were analyzed through immunohistochemistry.

### 1.23. Binding analysis of isoxazole ST-5-002 with radioisotope-labelled cholesterol for TM4SF5 or PD-L1

SNU761 cells and HEK293FT cells, which do not express TM4SF5, were transfected with Strep-EV, Strep-TM4SF1, TM4SF5, or mutations of TM4SF5, or Huh7 cells were transfected with Strep-PD-L1 or mutations of PD-L1. For Huh7_{KO} cells, stable cell lines were established by infecting with a retrovirus inducing the expression of pBabe-HA-EV or pBabe-HA-TM4SF5. Cell extracts were obtained, and extracts containing 0.2 or 0.25 mg of protein were reacted with an anti-HA antibody and HA-tagged TM4SF5, or with streptavidin agarose resin for Strep-PD-L1 or Strep-TM4SF5 at 4°C for 2 hours for pulldown. Subsequently, in the presence or absence of cold ST-5-002 (400 µM), the samples were treated with ¹⁴C-labelled ST-5-002 (10 or 25 µM) and reacted at 4°C for 1 hour, after which radioactive isotope values were repeatedly measured using a TriCarb scintillation counter (PerkinElmer, Waltham, MA).

After culturing HEK293FT cells transfected with Strep-EV or Strep-TM4SF5 expression vectors, or Huh7_{KO} cells stably expressing pBabe-HA-EV or pBabe-HA-TM4SF5, cell extracts were obtained. Extracts containing 1 mg of protein were pulled down by reacting with streptavidin agarose resin for Strep-tagged TM4SF5 or with an anti-HA antibody for HA-tagged TM4SF5 at 4°C for 4 hours. The pulled-down proteins were treated with ¹⁴C-labelled ST-5-002 at various concentrations (10 nM, 100 nM, 1 µM, 5 µM, 10 µM, 50 µM) and reacted at 4°C for 1 hour, after which radioactive isotope values were repeatedly measured using a TriCarb scintillation counter (PerkinElmer, Waltham, MA). The EC₅₀ was calculated by normalizing the average radioactive isotope value of TM4SF5 obtained through the measurements by dividing it by the average value of EV.

### 1.24. Immunofluorescence microscopy

SNU449-TM4SF5 cells overexpressing TM4SF5 were seeded on cover glasses pre-coated with collagen Type I (10 µg/ml), and 15 hour later, PD-L1-HA cDNA was transfected using Lipofectamine 3000 (Thermo Fisher). SNU761-HA-EV or SNU761-HA-TM4SF5 cells were transfected with cDNA of FLAG-SLAMF7 wildtype, N-glycosylation site mutants (1NQ; N204Q, or 3NQ; N172/176/204Q), or deletion mutants (ΔECD₁₋₂₂₅; a mutant in which the extracellular domain of amino acids 1-225 is removed, ΔTM₂₂₆₋₂₄₇: a mutant in which the transmembrane region of amino acids 226-247 is removed). After one to two days, DMSO, ST-5-001, or ST-5-002 was treated at 2.5 µM for 24 hours. The cells were fixed with cold methanol for 10 minutes and then subjected to a permeabilization process with 0.5% Triton X-100 at room temperature for 10 minutes. After washing twice with cold PBS, the cells were treated with DAPI and reacted with primary antibodies (TM4SF5_{EC2}, PD-L1, LAMP1, or SLAMF7 antibody) at 4°C for 15 hours, followed by washing three times with PBS. After reacting again with secondary antibodies at room temperature for 30 minutes (TRITC fluorescence for LAMP1, TRITC or Alexa Fluor^{®} 488 fluorescence for TM4SF5, and FITC fluorescence for PD-L1 or SLAMF7), the expression levels, localizations, and colocalizations (indicated in yellow) of the fluorescently-stained factors were observed using a confocal fluorescence microscope.

### Experimental Example 2. Experimental results

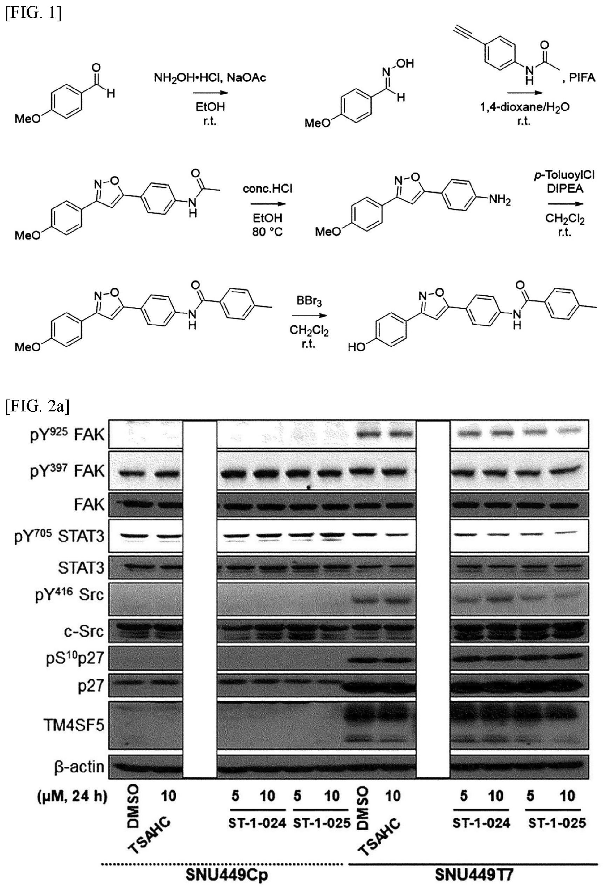

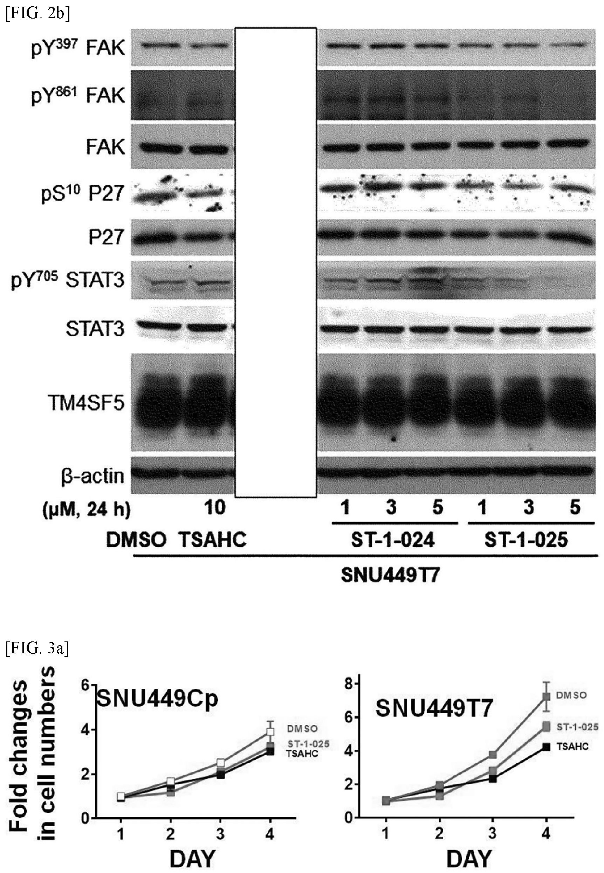

### 2.1. Confirmation of inhibition of TM4SF5-expression dependent signaling factor phosphorylation by isoxazole compounds (FIG. 2)

Cell extracts were obtained before and after treating TM4SF5-non-expressing cells (SNU449Cp) and TM4SF5-expressing cells (SNU449T₇) with ST-1-024 or ST-1-025 drugs at the given concentrations for 24 hours, and Western blotting was performed for signaling factors whose phosphorylation and activity increase in a TM4SF5-expression dependent manner.

As a result, (a-b) compared to SNU449Cp, the phosphorylation of FAK, c-Src, STAT3, and p27 increased in SNU449T₇ cells, but it was confirmed that these levels decreased when treated with the drug ST-1-025, similar to the positive control [4-(p-toluenesulfonamido)-4-hydroxychalcone, 4'-(p-toluenesulfonylamino)-4-hydroxychalcone, a chalcone-based positive control drug as a TM4SF5-specific inhibitor] TSAHC [refer to Korean Registered Patent Document No. 10-0934706].

Accordingly, by showing the efficacy of inhibiting the phosphorylation of TM4SF5-expression dependent signaling factors to some extent, the potential of isoxazole compounds as TM4SF5-specific inhibitors was confirmed.

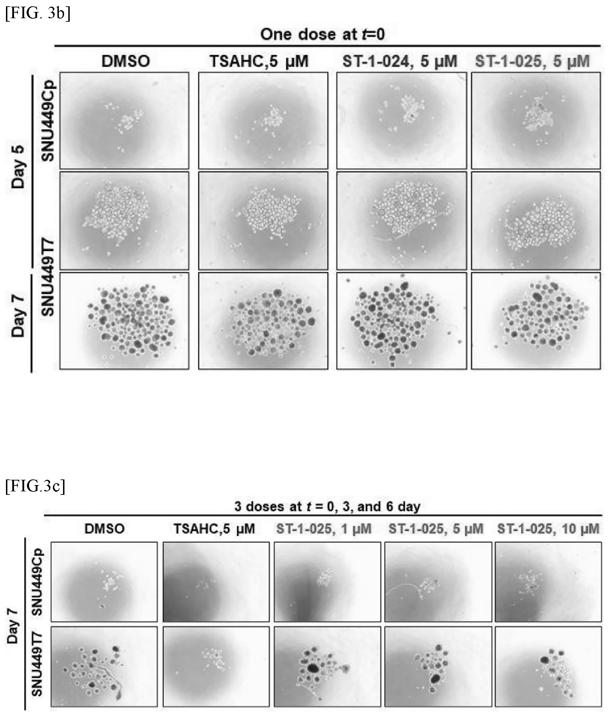

### 2.2. Confirmation of inhibition of TM4SF5-expression dependent 2D and 3D cell growth/proliferation by isoxazole compounds (FIG. 3)

The same number of TM4SF5-non-expressing cells (SNU449Cp) and TM4SF5-expressing cells (SNU449T₇) were cultured two-dimensionally in a normal cell culture vessel or cultured in a three-dimensional culture solution environment, while the change in cell number or the degree of sphere proliferation was observed with an optical microscope. The drugs treated were the positive control drug TSAHC or the ST-1-025 isoxazole compound at the given concentrations. From the time of starting culture (0 day), (FIG. 3a) in the case of 2D culture, the number of cells was measured after treating with the drug every 24 hours, or (FIG. 3b) in a 3D environment, the proliferation of cells (spheres) was observed and photographed with an optical microscope on the 5th and 7th days after a single drug treatment at the start of culture. Meanwhile, (FIG. 3c) in a 3D environment, the degree of sphere proliferation was observed with an optical microscope on the 7th day after repeated drug treatments on the 0, 3, and 6th days.

As a result, (a) it was confirmed that when SNU449T₇ cells growing in a 2D environment were treated with the drug ST-1-025, growth or proliferation decreased like the positive control TSAHC. It was confirmed that there was no significant difference when SNU449Cp was treated with the drug. (b) When the degree of sphere proliferation was observed on the 5th and 7th days after a single treatment of the drugs at 5 µM at the start of culture for cells under a 3D culture environment, it was confirmed that the proliferation degree of SNU449T₇ was inhibited, albeit slightly, in the case of TSAHC and ST-1-025. In the case of SNU449Cp, it was confirmed that it did not form a sphere shape. (c) When the degree of sphere proliferation was observed on the 7th day after repeatedly treating the cells under a 3D culture environment with the drugs at the given concentrations on the 0, 5, and 6th days, although the effect was more minimal than that of TSAHC, it was confirmed that the proliferation degree of SNU449T7 was inhibited by ST-1-025 in a concentration-dependent manner, albeit slightly. In the case of SNU449Cp, it was confirmed that it did not form a sphere shape.

Accordingly, by showing the efficacy of inhibiting the survival/proliferation of TM4SF5-expression dependent 2D and 3D cells and spheres, albeit slightly, the potential of isoxazole compounds as TM4SF5-specific inhibitors was confirmed. However, it suggested that a structure-activity relationship (SAR) study for compounds with higher TM4SF5-specific efficacy is necessary.

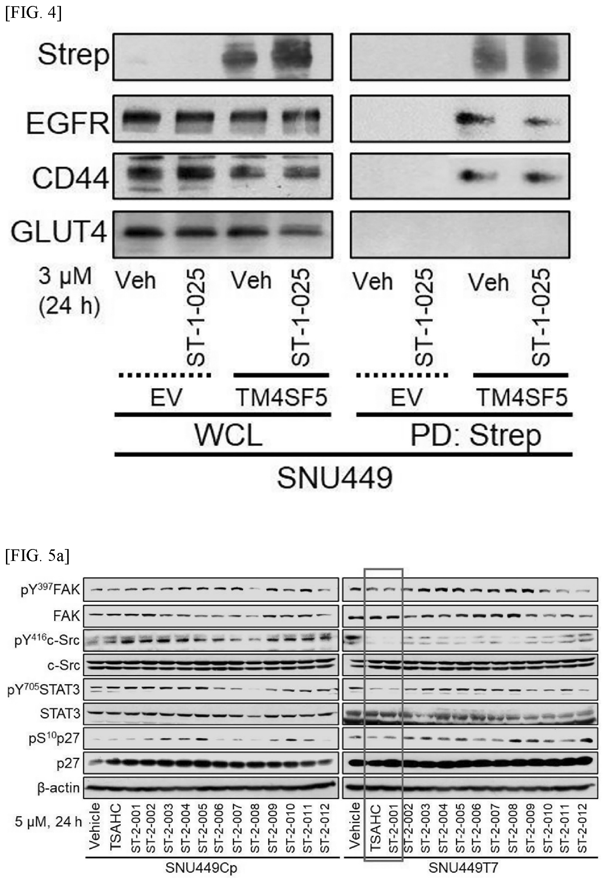

### 2.3. Confirmation of inhibition of binding between TM4SF5 and various cell surface receptors by treatment with isoxazole compounds (FIG. 4)

Vehicle (DMSO) or 3 µM of the drug ST-1-025 was treated for 24 hours under a normal culture environment containing serum to SNU449 hepatocytes stably transfected with a control expression vector (empty vector, EV) or a TM4SF5 expression vector (TM). Thereafter, cell extracts were obtained, pulled down with Streptavidin agarose resin (Thermo, XA335631), and Western blotting was performed using the given antibodies.

As a result, compared to the SNU449 control cell group that does not express TM4SF5, cells expressing TM4SF5 bound to EGFR, CD44, and GLUT4. Compared to the vehicle treatment, the binding of TM4SF5 to EGFR, CD44, and GLUT4 was slightly inhibited by the treatment with the drug ST-1-025.

Accordingly, by showing the efficacy of inhibiting the binding ability of TM4SF5 with membrane receptors important for cell survival/proliferation and metastasis potential, albeit slightly, it suggested that a structure-activity relationship (SAR) study for compounds with higher TM4SF5-specific efficacy is necessary to increase the potential of isoxazole compounds as TM4SF5-specific inhibitors.

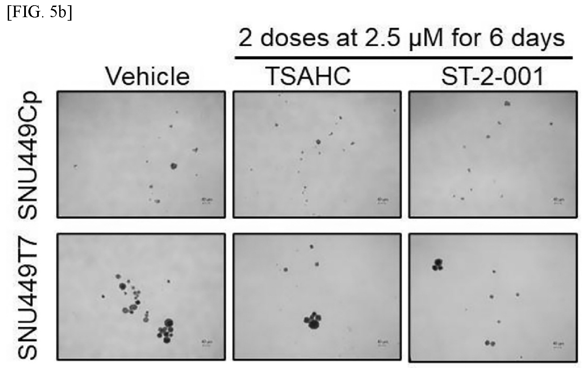

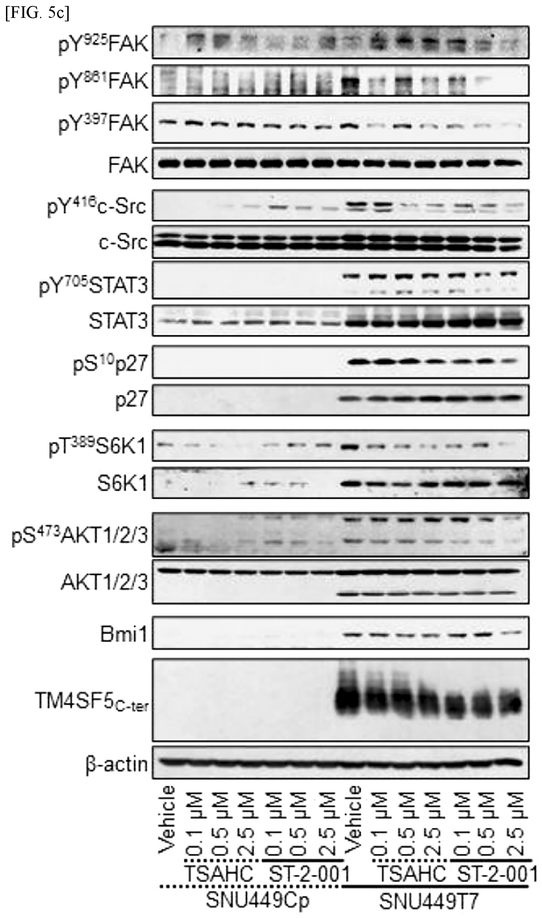

### 2.4. Confirmation of inhibition of TM4SF5-expression dependent signaling factor phosphorylation and 3D sphere proliferation by isoxazole compounds (FIG. 5)

(a) Cell extracts were obtained after treating TM4SF5-non-expressing cells (SNU449Cp) and TM4SF5-expressing cells (SNU449T₇) with vehicle (DMSO), TSAHC, or isoxazole compounds ST-2-001, ST-2-002, ST-2-003, ST-2-004, ST-2-005, ST-2-006, ST-2-007, ST-2-008, ST-2-009, ST-2-010, or ST-2-011 at a dose of 5 µM (for 24 hours). Using these, Western blotting was performed for signaling factors whose phosphorylation and activity increase in a TM4SF5-expression dependent manner. (b) The same number of TM4SF5-non-expressing cells (SNU449Cp) or TM4SF5-expressing cells (SNU449T₇) were cultured three-dimensionally in a normal cell culture vessel, while the degree of sphere proliferation was observed with an optical microscope. After treating with DMSO (vehicle), TSAHC, or the drug ST-2-001 at a concentration of 2.5 µM on the 0 and 3rd days of culture, the proliferation of cells (spheres) was observed with an optical microscope on the 6th day. (c) Cell extracts were obtained after treating SNU449Cp cells and SNU449T₇ cells with DMSO (vehicle), the positive control TSAHC, or the drug ST-2-001 at the given concentrations (for 24 hours), and then Western blotting was performed.

As a result, (a) compared to SNU449Cp, the phosphorylation of FAK, c-Src, STAT3, and p27 increased in SNU449T₇ cells, but it was confirmed that the phosphorylation of signaling factors decreased when treated with the drug ST-2-001 to a degree similar to that when treated with the positive control TSAHC. (b) In the case of SNU449Cp, it was confirmed that it did not form a sphere shape. However, in the case of SNU449T₇, it was confirmed that when treated with the drug ST-2-001, the degree of cell proliferation was inhibited similarly to the treatment with TSAHC compared to the control group. (c) Compared to SNU449Cp, the phosphorylation of FAK, c-Src, STAT3, p27, S6K1, and AKT increased in SNU449T₇ cells, but their phosphorylation decreased in a drug concentration-dependent manner when treated with TSAHC or the ST-2-001 drug. Through this, it was confirmed that the ST-2-001 drug, similar to TSAHC, has the efficacy of inhibiting the phosphorylation of various signaling factors that increase depending on TM4SF5 expression.

Accordingly, when additional isoxazole compounds that underwent SAR were analyzed in terms of their efficacy in inhibiting the phosphorylation of TM4SF5-expression dependent signaling factors and 3D cell survival/proliferation, the drug ST-2-001 showed an inhibitory efficacy to a degree similar to the chalcone-based TM4SF5-specific inhibitor TSAHC, thereby confirming the potential of isoxazole compounds as TM4SF5-specific inhibitors.

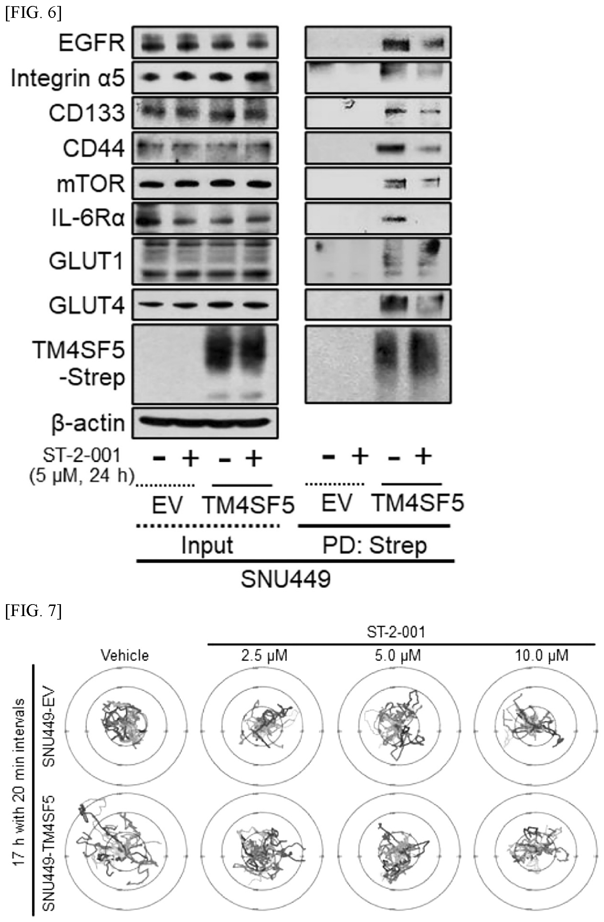

### 2.5. Confirmation of inhibition of binding between TM4SF5 and various cell surface receptors and signaling factors by treatment with isoxazole compounds (FIG. 6)

Cell extracts were obtained after treating SNU449 hepatocytes, into which a control expression vector (empty vector, EV) or a TM4SF5 expression vector (TM) was stably transfected, with vehicle (DMSO) or 5 µM of the drug ST-2-001 for 24 hours under a normal culture environment containing serum. After pulling down with Streptavidin agarose resin (Thermo, XA335631), Western blotting for various membrane receptors was performed using the given antibodies.

As a result, compared to the SNU449 control cell group that does not express TM4SF5, cells expressing TM4SF5 bound to EGFR, integrin α5, CD133, CD44, mTOR, IL-6R α, GLUT1, and GLUT4. However, compared to the vehicle treatment, the binding between TM4SF5 and the above factors was inhibited by the treatment with the drug ST-2-001.

Accordingly, when additional isoxazole compounds that underwent SAR were analyzed in terms of their efficacy in inhibiting the binding ability of TM4SF5 with membrane receptors and signaling factors important for cell survival/proliferation/migration, the drug ST-2-001 showed an inhibitory efficacy against protein-protein interactions to a certain extent similarly to the chalcone-based TM4SF5-specific inhibitor TSAHC, thereby confirming the potential of isoxazole compounds as TM4SF5-specific inhibitors.

### 2.6. Confirmation of inhibition of TM4SF5-expression dependent cell migration by isoxazole compounds (FIG. 7)

SNU449-EV cells, into which a control vector (EV) was injected so that TM4SF5 is not expressed, and SNU449-TM4SF5 cells, into which a TM4SF5 expression vector was stably injected, were seeded to a density of 40% in 8-chamber plates pre-coated with collagen type 1 (10 µg/ml). Three hours after seeding under a normal culture environment containing serum, DMSO and the drug ST-2-001 were treated once at concentrations of 2.5, 5.0, or 10.0 µM. By repeatedly photographing the positions of the same cells every 20 minutes for 17 hours after the drug treatment, an analysis for tracking the migration distance and path of the cells (cellular trafficking) was performed. The original position of each cell was moved to the origin of concentric circles, and the migration track of each cell was indicated by a line of a different color.

As a result, cell migration increased much more in the case of SNU449-TM4SF5 cells than in SNU449-EV treated with the DMSO vehicle. Meanwhile, the cell migration of SNU449-EV cells did not change by the treatment with the drug ST-2-001, but in the case of SNU449-TM4SF5 cells, it was confirmed that the migration of the cells decreased according to the treatment with ST-2-001 in a concentration-dependent manner.

Accordingly, when additional isoxazole compounds that underwent SAR were analyzed in terms of their efficacy in inhibiting TM4SF5-expression dependent cell migration, the drug ST-2-001 showed an inhibitory efficacy in a concentration-dependent manner, thereby confirming the potential of isoxazole compounds as TM4SF5-specific inhibitors.

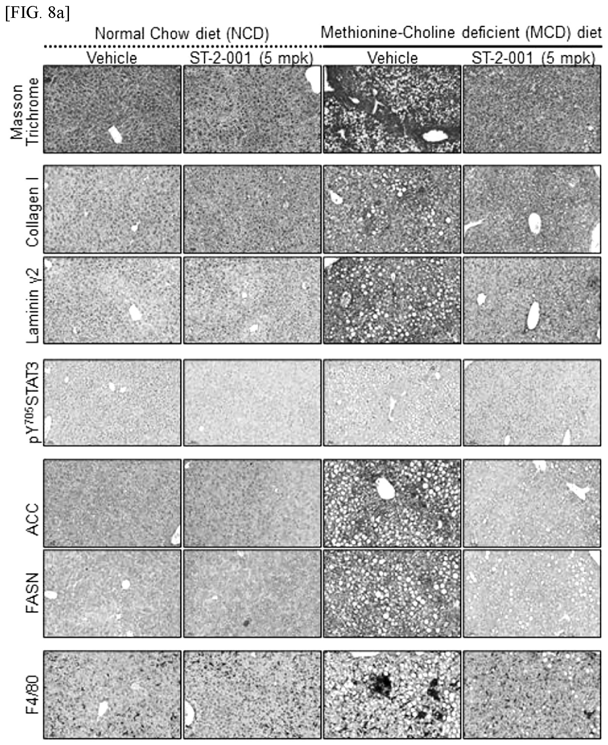

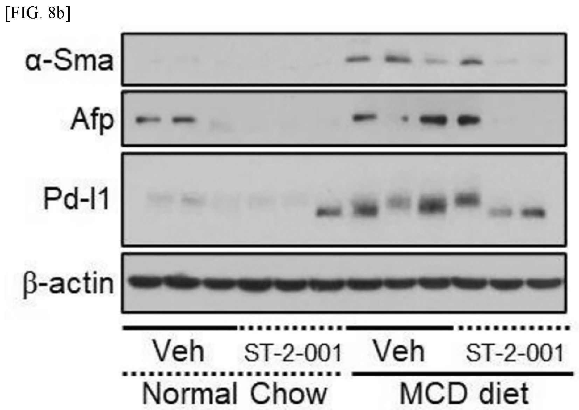

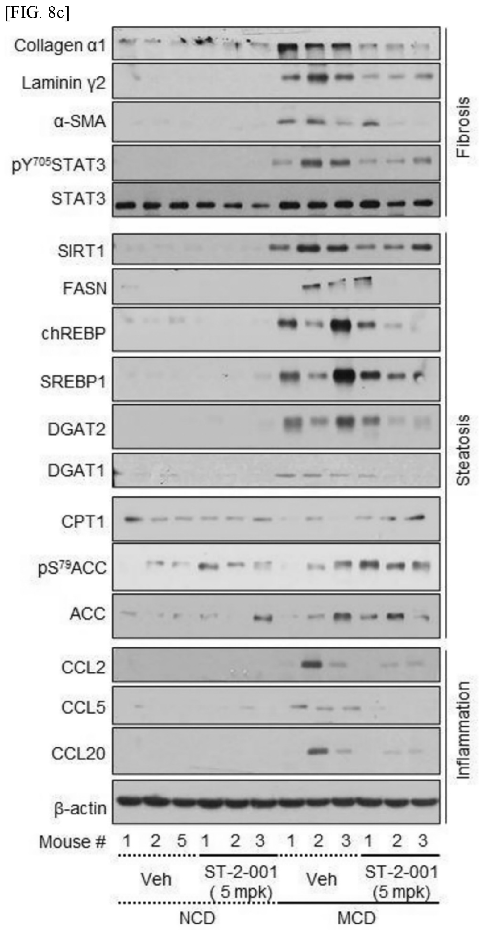

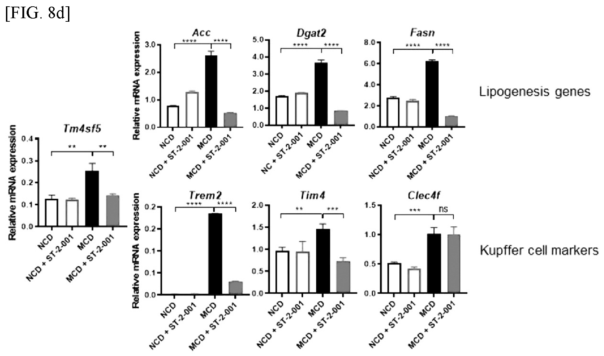

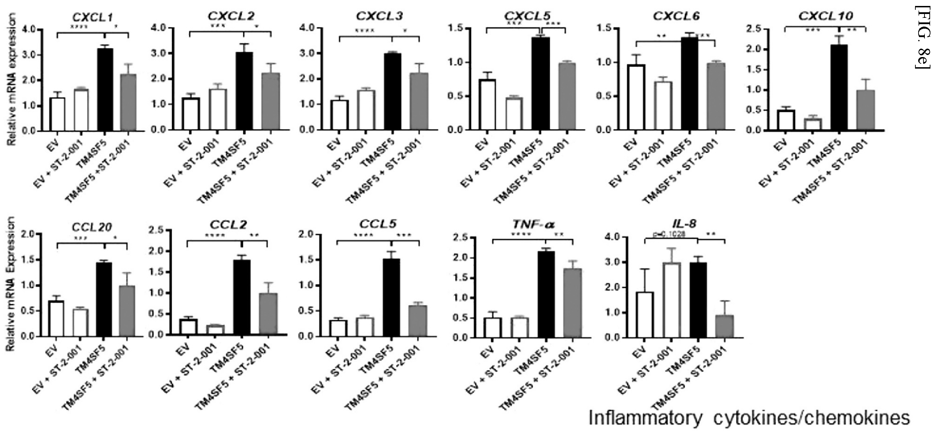

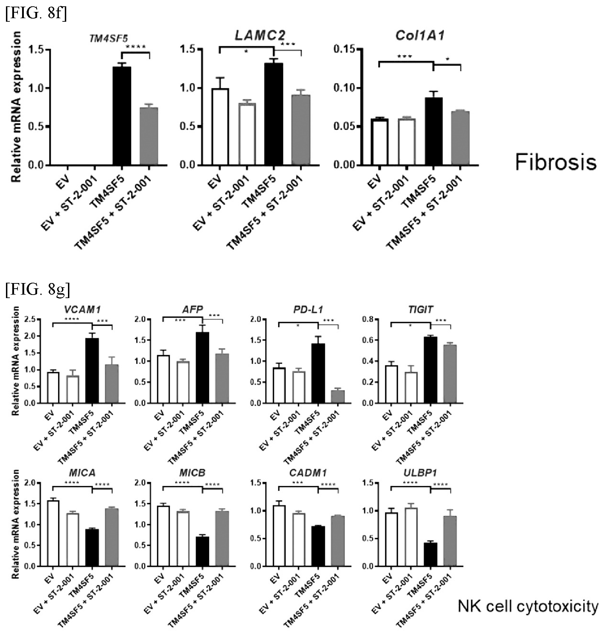

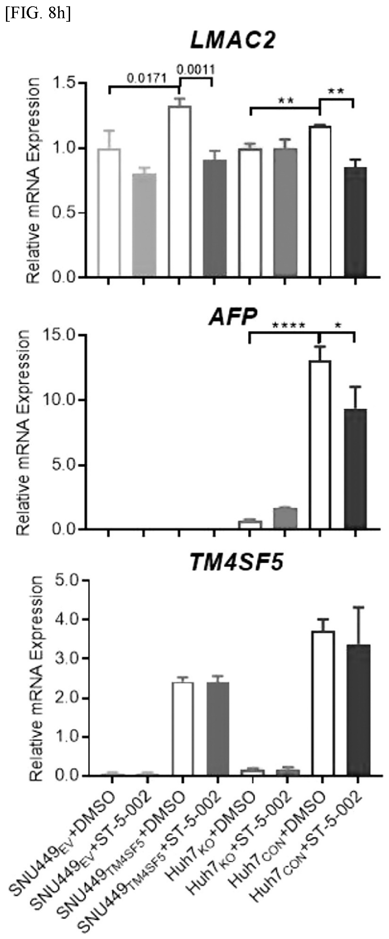

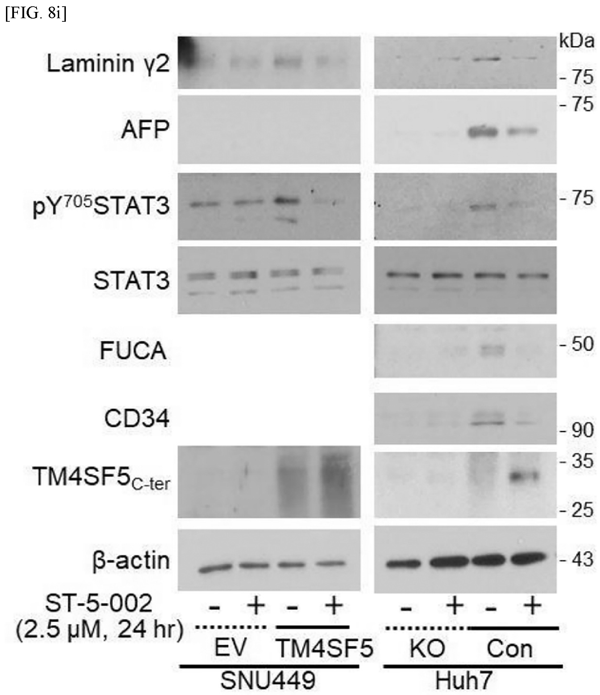

### 2.7. Confirmation of inhibition of TM4SF5-expression dependent NASH induction by isoxazole compounds (FIG. 8)

Normal 22-week-old C57BL/6 mice were randomly divided into a normal chow diet (NCD) group and a Methionine-Choline Deficient (MCD) diet group, which is a non-alcoholic steatohepatitis (NASH) induction diet, with 3 to 6 mice per group, and were allowed free access to the diets for 4 weeks. Simultaneously, vehicle (DMSO) or ST-2-001 at a concentration of 5 mg/kg was treated twice a week per animal via intraperitoneal injection (IP). After 4 weeks, liver tissues were obtained, and Masson's Trichrome staining showing the accumulation of collagen I (degree of fibrosis) and immunohistochemistry using antibodies against the given factors were performed. Representative images were obtained by randomly scanning the stained liver tissue sections using a digital slide scanner (MoticEasyScan, Motic, British Columbia, Canada). Additionally, tissue extracts were obtained from a portion of the liver tissues to perform Western blotting or qRT-PCR for the given factors, thereby confirming the mRNA and protein expression levels and phosphorylation degrees of signaling factors related to the induction of NASH-fibrosis symptoms and the regulation of T or NK cell activity. Meanwhile, in addition to the animal models, it was intended to confirm whether the expressions of TM4SF5, laminin γ2, and AFP correlate with each other at the hepatocyte level and are regulated by the TM4SF5-specific inhibitor isoxazole compound. Among hepatocytes, SNU449-EV and Huh7-KO (cells in which the expression of TM4SF5 was knocked out), which do not express TM4SF5, and SNU449-TM4SF5 and Huh7-Con, which express TM4SF5, were treated with DMSO or 2.5 µM of ST-5-002 for 24 hours under a normal cell culture environment, followed by performing qRT-PCR or obtaining cell extracts and performing Western blotting for the given antibodies.

As a result, (a) no abnormalities in liver tissue were found in animals that consumed NCD, and NASH did not develop, and the treatment with DMSO and the drug ST-2-001 had no effect on the liver tissue or the expression of the stained factors. Masson's Trichrome staining showing the morphology of liver tissue and collagen accumulation, the expression of collagen I α1 (COL1A1) or laminin γ2 (LAMC2), Acetyl-CoA Carboxylase (ACC), and Fatty Acid Synthase (FASN), the phosphorylation of STAT3, and the degree of staining of F4/80 representing the activity and distribution of macrophages did not change. However, compared to the NCD control group, the group treated with the MCD diet showed fibrosis symptoms with intensified accumulation of Collagen I, and the expression of collagen I or laminin γ2, Acetyl-CoA Carboxylase (ACC), and Fatty Acid Synthase (FASN), the phosphorylation of STAT3, and the degree of F4/80 staining were confirmed much more severely, indicating that steatohepatitis (NASH) and liver fibrosis symptoms were evident due to the MCD diet. It could be confirmed that these NASH-fibrosis symptoms were clearly inhibited by the treatment with the drug ST-2-001. (b) As a result of obtaining liver tissue extracts and performing Western blotting, the expressions of alpha-smooth muscle actin (α-Sma), which is an important marker for fibrosis, alpha-fetoprotein (Afp), well known as a clinical biomarker for liver cancer, and Programmed death-ligand 1 (Pd-l1), known to inhibit the activity of immune cells such as T cells and NK cells, were increased in animals treated with the MCD diet compared to NCD. In the case of NCD, the expression of these factors slightly decreased by the treatment with the drug ST-2-001 compared to the DMSO treatment. Meanwhile, in cases where several factors were significantly enhanced due to the induction of NASH-fibrosis by the MCD diet treatment, α-Sma, Afp, and Pd-ll were decreased by the drug ST-2-001 treatment. (c) When checking the expressions and levels of fibrosis-related factors (expression of collagen I α1, laminin γ2, and α-SMA, and phosphorylation of STAT3), factors related to lipogenesis and steatosis [SIRT1 (Sirtuin 1), FASN, chREBP (Carbohydrate response element binding protein), SREBP1 (Sterol regulatory element-binding protein 1), DGAT2 (Diacylglycerol O-Acyltransferase 2), DGAT1 (Diacylglycerol O-Acyltransferase 1), ACC (Acetyl-CoA carboxylase), and ACC phosphorylation], and cytokines/chemokines related to inflammation such as CCL2 (C-C Motif Chemokine Ligand 2), CCL5, and CCL20 using liver tissue extracts, they increased in the case of MCD compared to NCD, and this increase was suppressed while decreasing by the treatment with the drug ST-2-001. However, CPT1 (carnitine palmitoyltransferase 1) is a factor related to lipolysis and rather increased by the drug treatment in the case of the MCD diet, showing that it can inhibit the accumulation of fat. (d) qRT-PCR was performed using liver tissues. The mRNA levels for lipogenesis *(Acc, Dgat2, Fasn)* and activation of Kupffer cells, which are macrophages of liver tissue *(Trem2, Tim4, Clec4j),* increased in the case of MCD compared to NCD, and these increases were inhibited by the treatment with the drug ST-2-001. (e-g) qRT-PCR was performed after treating SNU449-EV or SNU449-TM4SF5 cells with vehicle or 2.5 µM of ST-2-001 for 24 hours. The mRNA levels of inflammation-related cytokines/chemokines such as CXC motif chemokine ligands (*CXCL1, CXCL2, CXCL3, CXCL5, CXCL6, CXCL10*) and CC motif chemokine ligands *(CCL20, CCL2, CCLS),* Tumor necrosis factor alpha (*TNF-α*)*,* Interleukin-8 (*IL-8*)*,* liver fibrosis factors *(Lamc2, Col1A1*)*,* and factors related to T cell or NK cell natural killer activation (*VCAM1, AFP, PD-L1, TIGIT, MICA, MICB, CADM1, ULBP1*) were enhanced in SNU449-TM4SF5 cells compared to SNU449-EV cells, and these were inhibited by the treatment with the drug ST-2-001. (h-i) Meanwhile, in an in vitro cell line model, the mRNA and protein expressions of laminin γ2 and AFP (α-fetoprotein) were higher in cells expressing TM4SF5 compared to hepatocytes that do not express it. At the protein level, in addition to laminin γ2 and AFP, which are known as biomarkers for liver cancer or to be highly expressed in liver cancer, the phosphorylation of STAT3 and the expression of FUCA (Alpha-L-Fucosidase 1) and CD34 were confirmed to be high. However, it was confirmed that the elevated expressions of these factors were inhibited according to the treatment with ST-5-002.

Accordingly, when additional isoxazole compounds that underwent SAR were analyzed in terms of their efficacy in inhibiting the induction of NASH disease according to the TM4SF5-expression dependent MCD diet, the drug ST-2-001 showed that activities or phenotypes related to NASH were inhibited, thereby confirming the potential of the isoxazole compound as a TM4SF5-specific inhibitor, a NASH inhibitor, or an anti-liver disease agent. Furthermore, the expression of laminin γ2 and AFP increases due to TM4SF5 even in the pre-cancerous stage of liver cancer, i.e., the NASH-fibrosis disease stage, and this can be inhibited by the treatment with the TM4SF5-specific inhibitor isoxazole; thus, it was presumed that the efficacy of such an isoxazole compound could inhibit and regulate the transition and intensification stages from NASH-fibrosis liver disease to liver cancer.

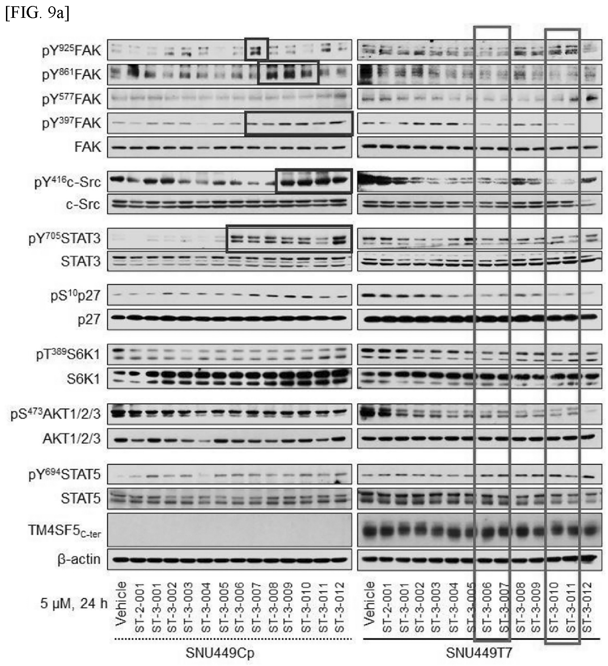

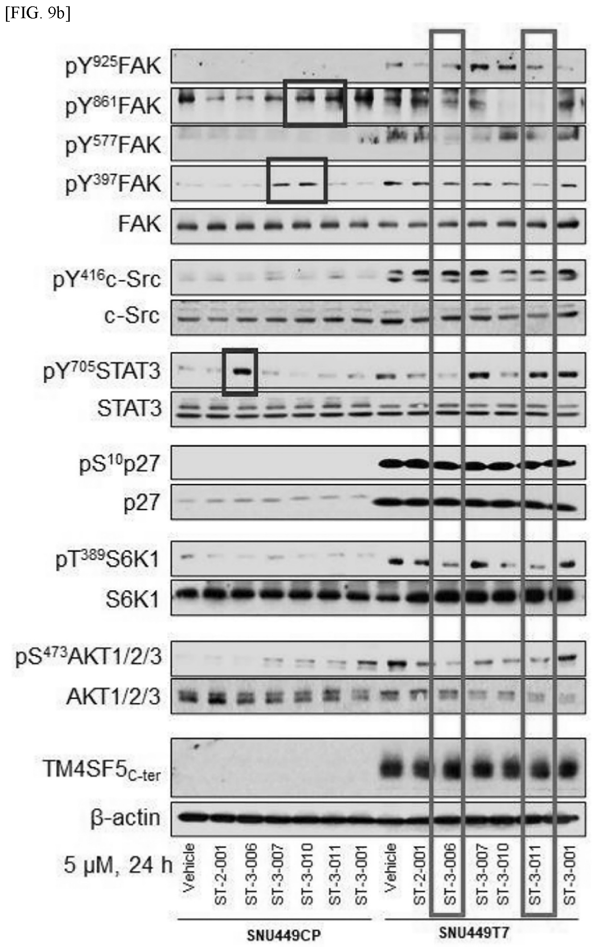

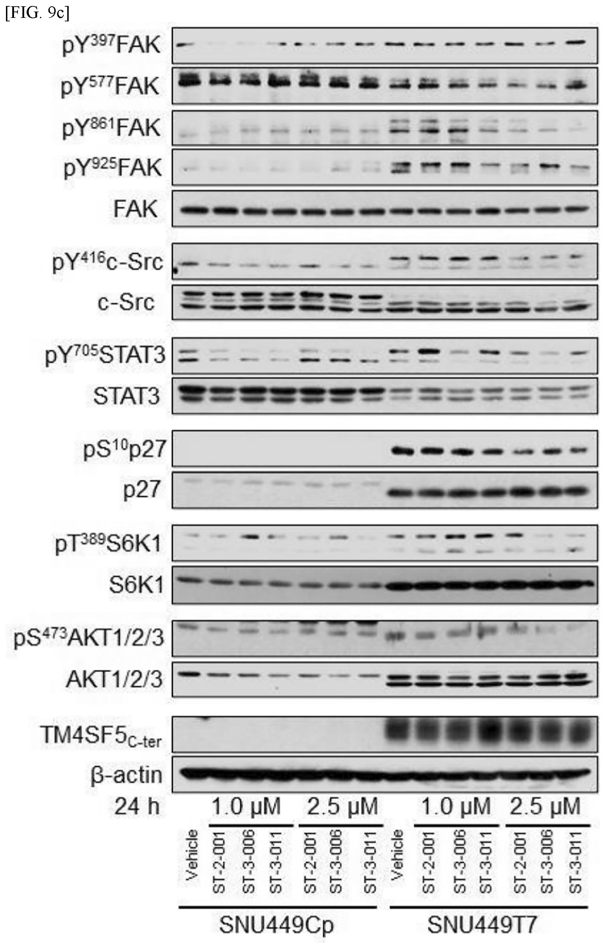

### 2.8. Confirmation of inhibition of TM4SF5-expression dependent signaling factor phosphorylation by isoxazole compounds (FIG. 9)

(a) Cell extracts were obtained after treating TM4SF5-non-expressing cells (SNU449Cp) and TM4SF5-expressing cells (SNU449T₇) with vehicle (DMSO), ST-2-001, or an ST-2-001-based derivative drug at a concentration of 5 µM for 24 hours. (b) Cell extracts were obtained after treating SNU449Cp and SNU449T₇ with DMSO, ST-2-001, ST-3-001, ST-3-006, ST-3-007, ST-3-010, or ST-3-011 at a concentration of 5 µM for 24 hours. (c) Cell extracts were obtained after treating SNU449Cp and SNU449T₇ with DMSO, ST-2-001, ST-3-006, or ST-3-011 at the given concentrations for 24 hours. (a-c) Using the cell extracts obtained after the drug treatment, Western blotting was performed for signaling factors whose phosphorylation and activity increase in a TM4SF5-expression dependent manner.

As a result, (a-b) compared to SNU449Cp, the phosphorylation of FAK, c-Src, STAT3, p27, S6K1, AKT, and STAT5 increased in SNU449T₇ cells, but these levels decreased upon treatment with the drugs ST-2-001 and ST-3-01 to 012. In particular, when SNU449T₇ cells expressing TM4SF5 were treated with the drug ST-3-006, ST-3-007, ST-3-010, or ST-3-011, it was confirmed that the phosphorylation of various signaling factors decreased as indicated by the red-lined boxes, similar to ST-2-001; however, it was confirmed that the phosphorylation of some signaling factors indicated by the blue-lined boxes increased in SNU449Cp cells which do not express TM4SF5. (c) Compared to SNU449Cp, the phosphorylation of FAK, c-Src, STAT3, p27, S6K1, and AKT increased in SNU449T₇ cells, but these levels decreased in a concentration-dependent manner upon treatment with the drugs [ST-2-001, ST-3-006, and ST-3-011]. However, similarly, it was observed that the phosphorylation of some signaling factors increased in SNU449Cp cells that do not express TM4SF5.

Accordingly, when isoxazole compounds (ST-3-00X), which underwent additional SAR to improve the properties of isoxazole, were analyzed in terms of their efficacy in inhibiting the phosphorylation of TM4SF5-expression dependent signaling factors, the drugs ST-3-006, ST-3-007, ST-3-010, and ST-3-011 showed inhibitory efficacy to a degree similar to ST-2-001, thereby confirming the potential of isoxazole compounds as TM4SF5-specific inhibitors. However, they showed a tendency to rather enhance the phosphorylation of some factors (indicated by blue-lined boxes) in cells that do not express TM4SF5.

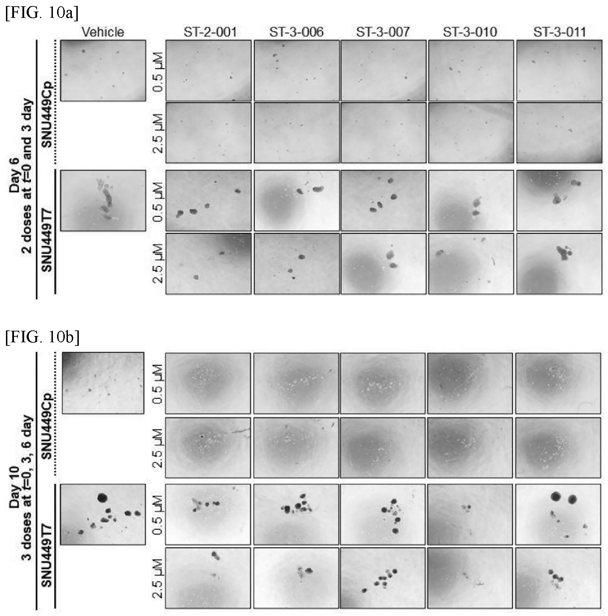

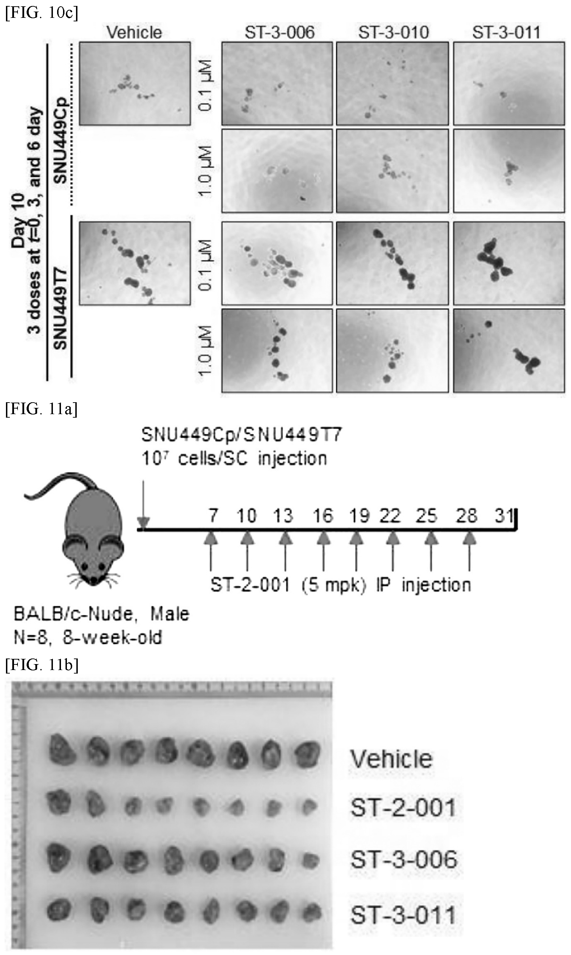

### 2.9. Confirmation of inhibition of TM4SF5-expression dependent 3D cell growth/proliferation by isoxazole compounds (FIG. 10)

The same number of TM4SF5-non-expressing cells (SNU449Cp) or TM4SF5-expressing cells (SNU449T₇) were cultured three-dimensionally in a normal cell culture vessel, while the degree of sphere proliferation was observed with an optical microscope. (a) After treating with DMSO (vehicle) or the drug ST-2-001, ST-3-006, ST-3-007, ST-3-010, or ST-3-011 at a concentration of 0.5 or 2.5 µM on the 0 and 3rd days of culture, the proliferation of cells (spheres) was observed with an optical microscope on the 6th day. (b) Along with the start of culture (0 day), after treating with DMSO (vehicle) or the drugs at a concentration of 0.5 or 2.5 µM on the 3rd and 6th days of culture, the proliferation of cells (spheres) was observed with an optical microscope on the 10th day. (c) Along with the start of culture (0 day), after treating with DMSO (vehicle) or the drug ST-3-006, ST-3-010, or ST-3-011 at a concentration of 0.1 or 1.0 µM on the 3rd and 6th days of culture, the proliferation of cells (spheres) was observed with an optical microscope on the 10th day.

As a result, (a, b) in the case of SNU449Cp, it did not form a sphere shape, but in the case of SNU449T₇, the proliferation of spheres was evident; however, when treated with the drug ST-2-001, ST-3-006, ST-3-007, ST-3-010, or ST-3-011, the degree of three-dimensional cell proliferation was inhibited compared to the vehicle-treated group. Among them, it was confirmed that the cell proliferation inhibitory effect of the drugs ST-2-001, ST-3-006, and ST-3-010 was strong. (c) The sphere formation ability of SNU449T₇ was superior to that of SNU449Cp, but the degree of three-dimensional cell proliferation decreased by the treatment with the drugs ST-3-006, ST-3-010, and ST-3-011.

Accordingly, when isoxazole compounds (ST-3-00X), which underwent additional SAR to improve the pharmacological properties of ST-2-001 isoxazole, were analyzed in terms of their efficacy in inhibiting TM4SF5-expression dependent 3D cell survival/proliferation, the drugs ST-3-006, ST-3-007, ST-3-010, and ST-3-011 showed inhibitory effects to a degree similar to ST-2-001, thereby confirming the potential of isoxazole compounds to inhibit cancer metastasis as TM4SF5-specific inhibitors.

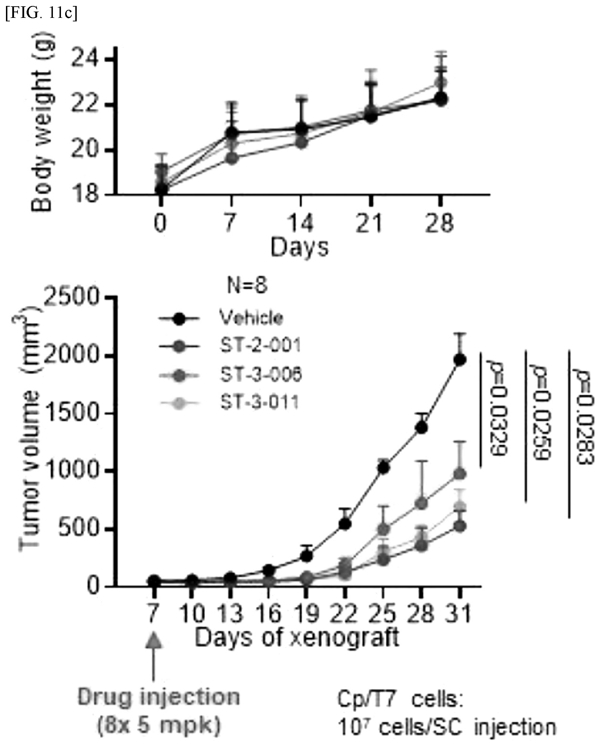

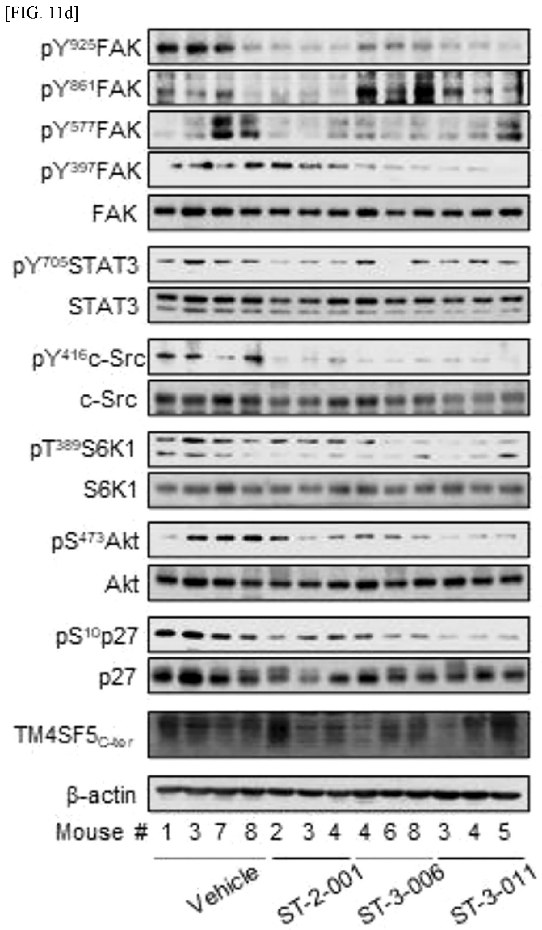

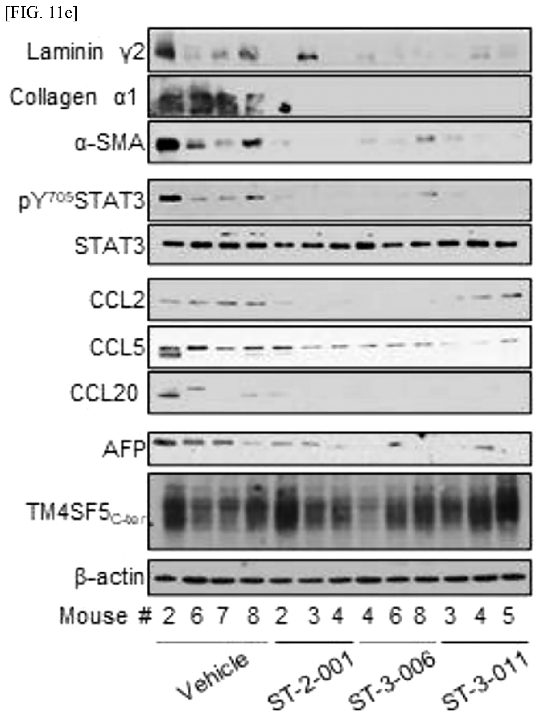

### 2.10. Confirmation of inhibition of TM4SF5-expression dependent xenograft liver cancer formation by isoxazole compounds (FIG. 11)

Eight-week-old male BALB/c-Nude mice (n=8, Orient, Seongnam, Republic of Korea) were allowed free access to a normal diet, and TM4SF5-non-expressing cells (SNU449Cp) and TM4SF5-expressing cells (SNU449T₇) were injected once subcutaneously at a dose of 10⁷ cells per mouse. Starting from the 7th day after the cell injection, drug ST-2-001, ST-3-006, or ST-3-011 was intraperitoneally injected at 5 mg/kg of body weight every 3 days for a total of 8 times. Body weight and tumor volume were measured every week, and on the 31st day after the cell injection, the mice were anesthetized by a standard method, liver tissues were obtained, and the size of the liver cancer tissues was photographed. Thereafter, the tissues were randomly divided into several sets and analyzed by immunohistochemistry, Western blot, qRT-PCR, etc.

As a result, (a) is a schematic diagram of the subcutaneous xenograft experimental method for TM4SF5-non-expressing and TM4SF5-expressing cell lines. (b) When checking the images of the recovered cancer tissues after the end of the experiment, it was confirmed that the liver cancer formation inhibitory effect of ST-2-001 was the greatest compared to the tumor tissues of the control group injected with the vehicle, and the inhibitory effects of ST-3-006 and ST-3-011 were relatively less than those of ST-2-001. (c) The body weight and tumor volume measured every week while intraperitoneally injecting the drugs every 3 days after the subcutaneous cell injection are shown. It was confirmed that the tumor volume was statistically significantly inhibited when each drug was injected compared to the case of the vehicle. (d) Three days after the last drug injection, when Western blotting was performed to measure the phosphorylation of signaling activators related to cancer formation (FAK, STAT3, c-Src, Akt) and a TM4SF5-related factor (p27) using some tissue extracts obtained from the liver cancer tissues, it was confirmed that the phosphorylation of each factor was significantly inhibited in the cases where the drugs were injected compared to the vehicle-treated control group. (e) It was confirmed that the expressions of factors related to fibrosis (collagen I, laminin γ2, α-SMA, STAT3 phosphorylation), inflammation-related cytokines/chemokines (CCL2, CCL5, or CCL20), and AFP as a liver cancer biomarker decreased when the drugs were treated compared to the vehicle.

Accordingly, when additional isoxazole compounds ST-3-006 and ST-3-011, which underwent SAR to improve the properties of isoxazole, were compared with ST-2-001 and their efficacy in inhibiting liver cancer formation by subcutaneous xenograft of TM4SF5-expression dependent liver cancer cell lines was analyzed, the potential of isoxazole compounds as anti-liver cancer inhibitors as TM4SF5-specific inhibitors was confirmed as the drugs ST-3-006 and ST-3-011 showed liver cancer formation inhibitory effects like ST-2-001.

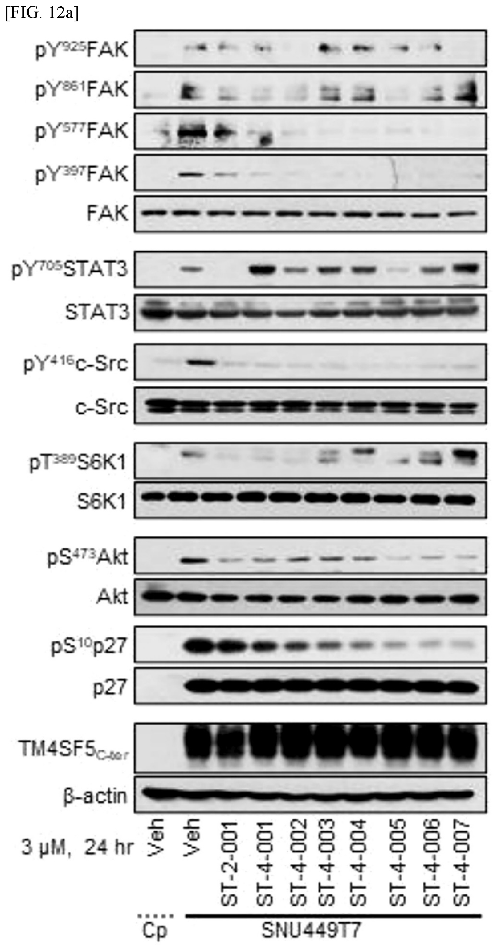

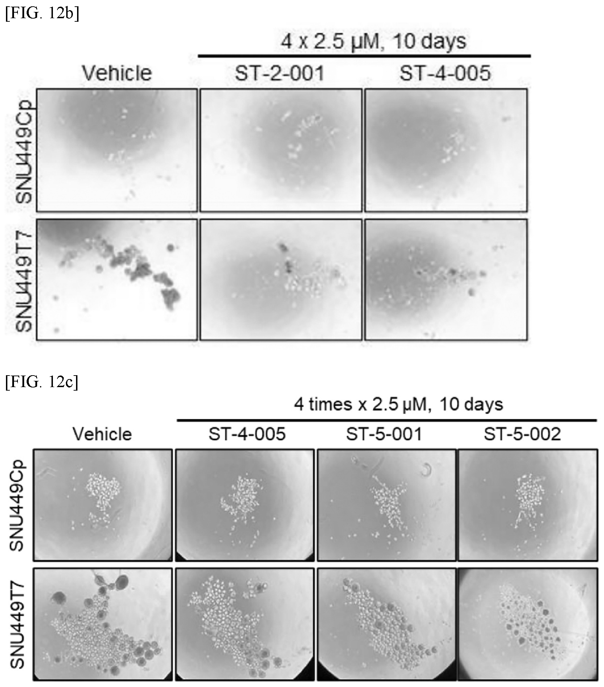

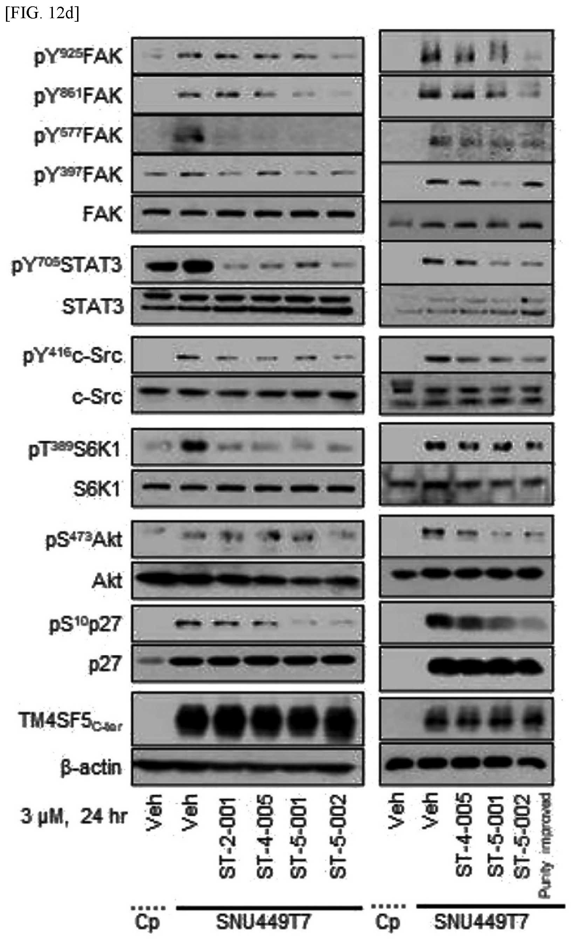

### 2.11. Confirmation of inhibition of TM4SF5-expression dependent signaling factor phosphorylation and 3D cell growth/proliferation by isoxazole compounds (FIG. 12)

Cell extracts were obtained before and after treating TM4SF5-non-expressing cells (SNU449Cp) and TM4SF5-expressing cells (SNU449T₇) with ST-2-001, ST-4-005, ST-5-001, or ST-5-002 drugs at the given concentrations for 24 hours, and Western blotting was performed for signaling factors whose phosphorylation and activity increase in a TM4SF5-expression dependent manner. Meanwhile, while the same number of SNU449Cp and SNU449T₇ cells were cultured in a three-dimensional culture solution environment, vehicle or the above drugs were treated at the given concentrations on the 0, 3, 6, and 9th days, and then the survival and proliferation degree of spheres was observed with an optical microscope on the 10th day.

As a result, (a) compared to the vehicle-treated SNU449Cp cells, the phosphorylation of FAK, STAT3, c-Src, S6K1, Akt, and p27 increased in the case of vehicle-treated SNU449T₇. However, among various drugs, it was confirmed that these increases were inhibited in many cases including STAT3, S6K1, and FAK by ST-2-001 and ST-4-005. (b-c) When the sphere survival/proliferation in a three-dimensional culture solution environment was checked, sphere formation did not occur in the case of SNU449Cp, but it was confirmed that the survival/proliferation of the spheres formed in the case of SNU449T₇ was inhibited by the treatment with drug ST-2-001, ST-4-005, ST-5-001, or ST-5-002. (d) Compared to the vehicle-treated SNU449Cp cells, the phosphorylation of FAK, STAT3, c-Src, S6K1, Akt, and p27 increased in the case of vehicle-treated SNU449T₇. However, it was confirmed that these increases were inhibited by the treatment with ST-2-001, ST-4-005, ST-5-001, or ST-5-002 among several drugs. In particular, in the case of ST-5-002, when the drug with improved purity was treated in the same manner, it could be confirmed that the phosphorylation inhibitory efficacy was maintained and further improved.

Accordingly, when additional isoxazole compounds ST-4-001 to ST-4-007, which underwent SAR to improve the physical properties of isoxazole ST-3-006 and ST-3-011 toward solid characteristics, were compared with ST-2-001 and their efficacy in inhibiting the signaling factor phosphorylation and 3D cell survival/proliferation of TM4SF5-expression dependent liver cancer cell lines was analyzed, the potential of isoxazole compounds as TM4SF5-specific inhibitors was confirmed as the drug ST-4-005 showed inhibitory effects similar to ST-2-001.

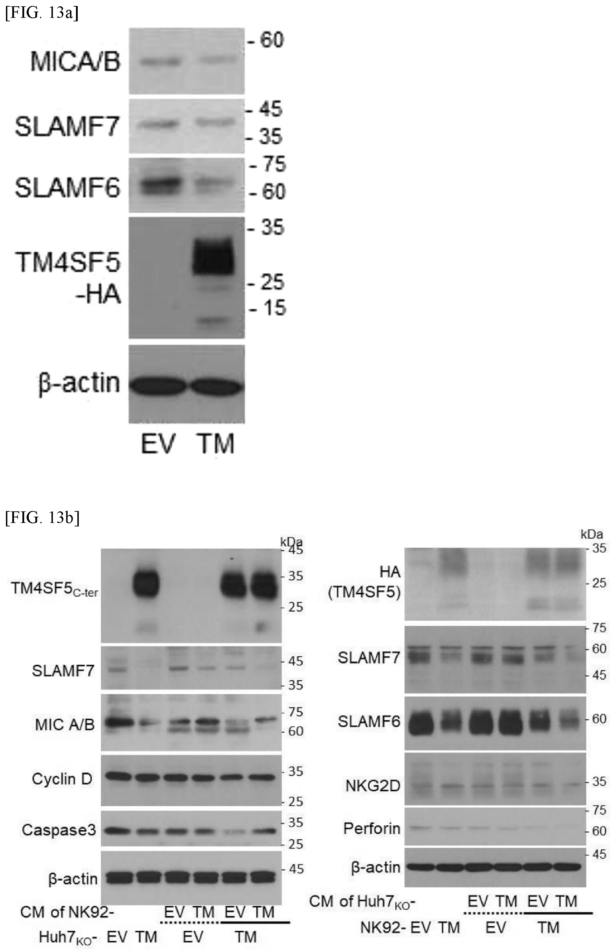

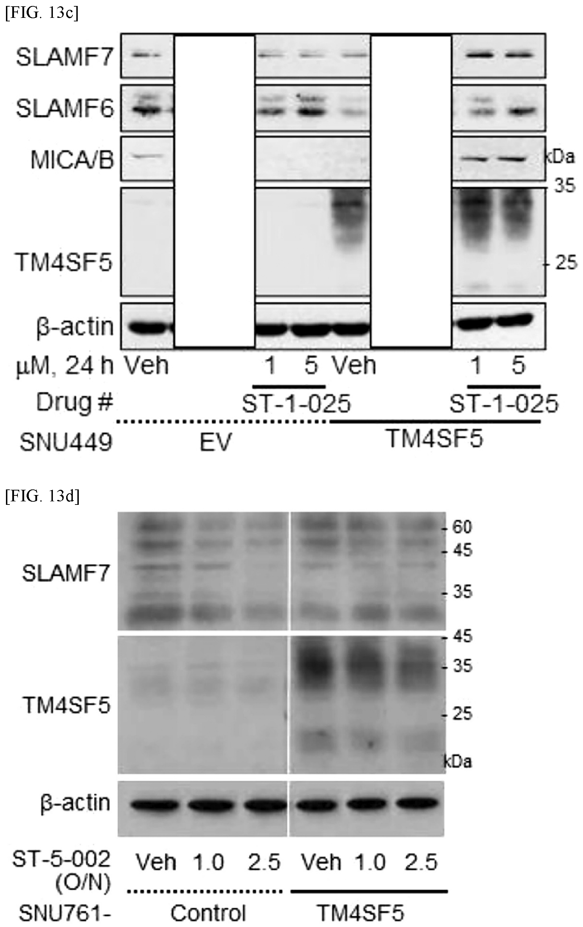

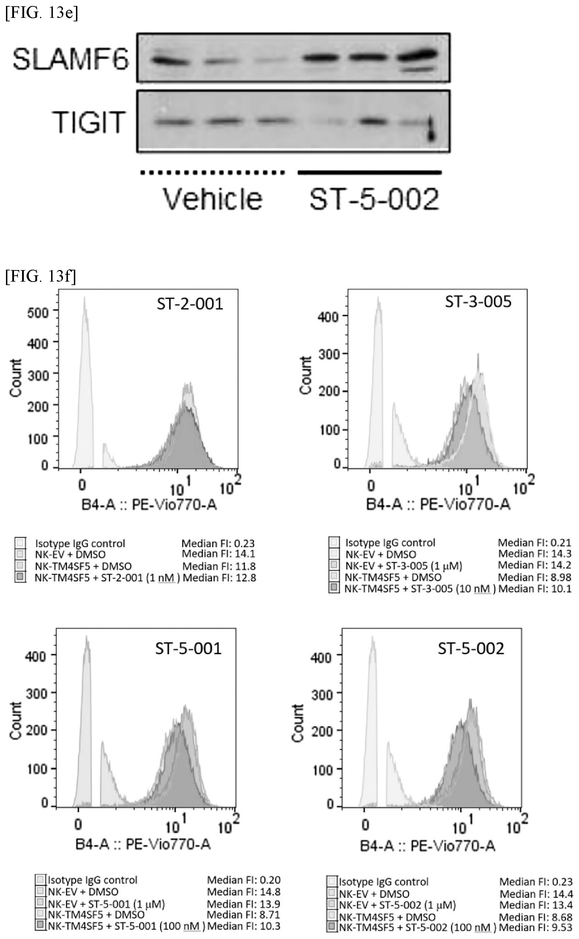

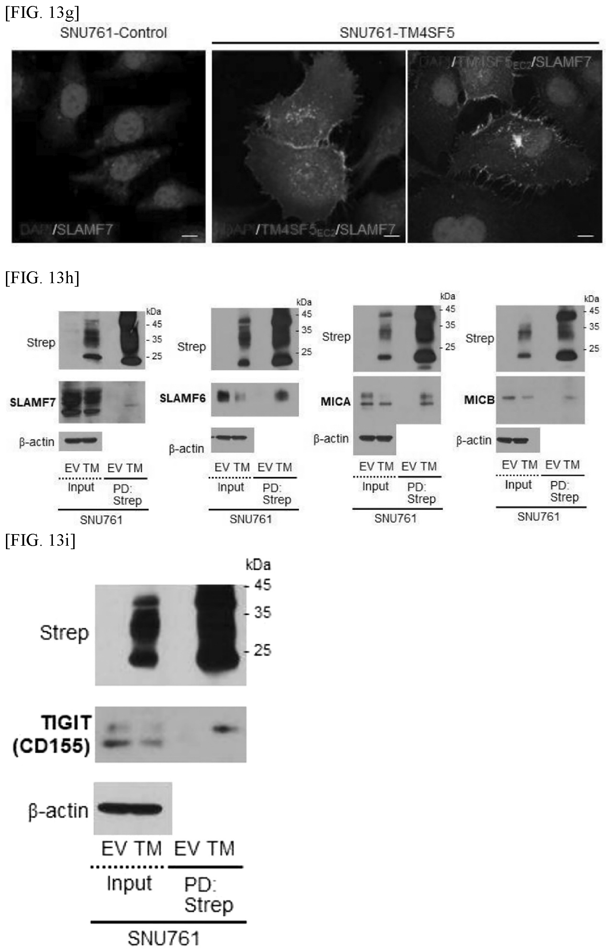

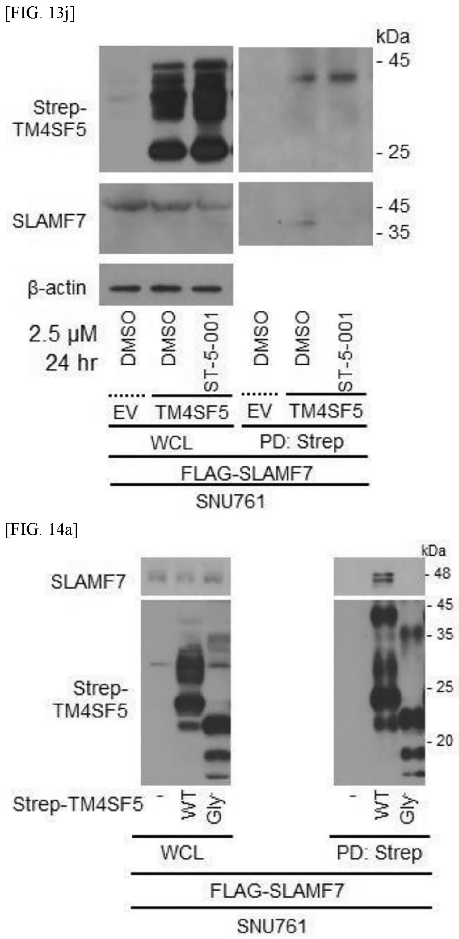

### 2.12. Confirmation of reduction in expression of ligands related to TM4SF5-expression dependent natural killer function of NK cells and inhibition of binding with TM4SF5 by isoxazole compounds (FIG. 13)

(a-d) After transfecting SNU449 or SNU761 cells, which do not originally (endogenously) express TM4SF5, to transiently or stably express a control expression vector (empty vector, EV) or a TM4SF5 vector, the same number of cells were prepared in culture vessels. After 24 hours, vehicle DMSO, drug ST-1-025, or ST-5-002 was treated at the given concentrations. After treating the drugs for 16 hours (overnight, O/N) or 24 hours, cell extracts were obtained and Western blotting was performed. (e) In the case of the mouse xenograft experiment, after treating with DMSO or ST-5-002, liver tissues were obtained, and tissue extracts were secured therefrom to perform Western blotting. (f) After treating NK92-EV or NK92-TM4SF5 cells with DMSO or drugs ST-2-001 (1 nM), ST-3-005 (10 nM or 1 µM), ST-5-001, or ST-5-002 (100 nM or 1 µM) for 24 hours, the expression of SLAMF7 expressed on the cell surface was analyzed through immunostaining using flow cytometry. The expression level of SLAMF7 was confirmed by adding a SLAMF7 antibody to bind with SLAMF7 and then measuring the fluorescence linked to the SLAMF7 antibody using flow cytometry. (g-j) Through immunostaining and fluorescence microscopy analysis of SNU761-EV or SNU761-TM4SF5 cells, the colocalization (yellow stain) of TM4SF5 (red fluorescence) and SLAMF7 (green fluorescence) on the cell membrane was confirmed (g). For another set of cells, either no treatment was performed (h-i), or after treatment with DMSO or ST-5-001 (2.5 µM) for 24 hours (j), cell extracts were obtained. Strep-TM4SF5 present in the extracts was pulled down with Streptavidin agarose resin (Thermo, XA335631), and then Western blotting for co-existing SLAMF7, SLAMF6, MICA, MICB, or CD155 (a protein that interacts with TIGIT) was performed using their antibodies, thereby confirming that the binding between TM4SF5 and them was inhibited by the isoxazole treatment.

As a result, (a) compared to SNU449-EV cells that do not express TM4SF5, in the case of SNU449-TM4SF5 cells that express TM4SF5, the expression of stimulatory ligands that activate the natural killer ability **of NK** cells, such as MICA/B, SLAMF6, and SLAMF7, was decreased. (c) Unlike in SNU449-EV cells, in SNU449-TM4SF5 cells, the expression of the stimulatory ligands increased again when treated with the drug ST-1-025 at the given concentration for 24 hours, compared to the case where the expression was lowered without drug treatment. (d) In the case of SNU761-TM4SF5 cells compared to SNU761-EV cells, the expression of SLAMF7 was also lowered when vehicle was treated. However, when the drug ST-5-002 was treated, the expression of SLAMF7 was lowered in SNU761-EV cells, but in the case of SNU761-TM4SF5, the SLAMF7 expression level, which had been lowered before the drug treatment, was less lowered or remained high after the treatment. (e) In animal liver tissues, the treatment with the drug ST-5-002 increased SLAMF6, an activator **of NK** cell natural killer function, but in the case of TIGIT, an inhibitor of NK cell natural killer function, it was confirmed that its expression level was lowered. (f) Through measurement of the median fluorescence intensity (Median FI) of SLAMF7, the degree of expression on the cell surface was lowered in the case of NK92-TM4SF5 cells compared to NK92-EV cells not treated with the drug, but it was confirmed that the level increased again when each drug was treated. However, in the case of NK92-EV cells, the change according to the drug treatment was very minimal. (g) As a result of confirming the colocalization of TM4SF5 and SLAMF7 with a fluorescence microscope, it was confirmed that they can bind by confirming that they are co-located at the cell membrane site. (h-j) When TM4SF5 was pulled down using the cell extracts, it was confirmed that SLAMF7, SLAMF6, MICA, MICB, or CD155 (TIGIT) were pulled down together when TM4SF5 was present, confirming that TM4SF5 and they can bind. Meanwhile, it was confirmed that the binding between TM4SF5 and SLAMF7 was inhibited after ST-5-001 was treated (j). It was confirmed that this binding was inhibited according to the treatment with the TM4SF5-specific inhibitor isoxazole. That is, it ultimately led to the presumption that it is related to the decrease in the expression of those factors.

Accordingly, when analyzing the effect of treatment with isoxazole compounds on the expression pattern of membrane proteins or factors that regulate NK cell activity in TM4SF5-expression dependent liver cancer cells and liver tissues, the potential of the isoxazole compound as a TM4SF5-specific inhibitor and an NK cell immune checkpoint inhibitor was suggested, as ST-5-002, an additional SAR result of drugs ST-1-025 and ST-3-005, showed the effect of increasing (from the level lowered by TM4SF5 expression) the expression of NK cell activation ligands/receptors such as SLAMF6, SLAMF7, MICA, MICB, and NKG2D.

### 2.13. Confirmation of reduction in expression of ligands related to TM4SF5-expression dependent natural killer function of NK cells and inhibition of binding with TM4SF5 by isoxazole compounds in an environment of indirect interaction between NK92 cells and hepatocytes (FIG. 13b)

Stable NK92 cell lines were obtained by infecting human NK cells, NK92, with a retrovirus inducing the expression of pBabe-HA-EV or pBabe-HA-TM4SF5. To create an environment of indirect interaction between NK92 cells and hepatocytes, experiments were performed by treating hepatocytes with conditioned media (CM) from NK cells or vice versa, and cell extracts were obtained and analyzed in a situation where indirect interaction between the two cells occurred. (b, left) Stable NK92 cell lines were cultured under normal conditions for two days to obtain conditioned media (CM). Meanwhile, when cell lines (TM) in which a control expression vector (empty vector, EV) or a TM4SF5 expression vector was re-expressed in a cell line (Huh7_{KO}) where TM4SF5 was knocked out by a CRISPR/Cas9 strategy in Huh7 cells reached 70-80% confluency, they were treated with the CM diluted to a concentration of 20% with the culture medium of Huh7_{KO}. After 24 hours, cell extracts were obtained, and Western blotting was performed using the given antibodies. (b, right) CM obtained after culturing Huh7_{KO}-EV or Huh7_{KO}-TM4SF5 (TM) cell lines for two days was treated to NK92-EV or NK92-TM4SF5 cells by diluting the CM to a concentration of 20% with the culture medium of NK92. After 24 hours, NK92 cell extracts were obtained, and Western blotting was performed.

As a result, (b) in the case of SLAMF7 and MICA/B, the expression tended to be lower in the Huh7_{KO}-TM4SF5 cell line than in the Huh7_{KO}-EV cell line treated with the CM of NK92-EV or NK92-TM4SF5 (TM) cells, and particularly, showed a tendency to be even lower upon treatment with the CM of NK92-TM4SF5 (TM) (left). Meanwhile, the expressions of SLAMF7, SLAMF6, NKG2D, and perforin were lower in NK92-TM4SF5 cells treated with the CM than in NK92-EV cells treated with the CM of Huh7_{KO}-EV or Huh7_{KO}-TM4SF5 cell lines (right).

Accordingly, (b) it was confirmed that when NK92 cells and hepatic (cancer) epithelial cells meet each other indirectly through CM treatment, the killing efficacy of NK92 cells can be lower when TM4SF5 is expressed in each cell than when it is not, and particularly, when both types of cells express TM4SF5, the killing efficacy of NK92 cells is further lowered.

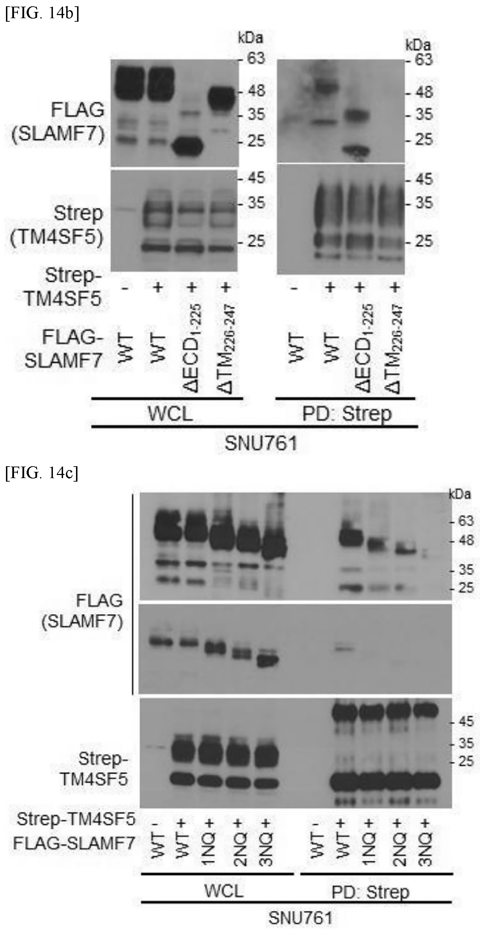

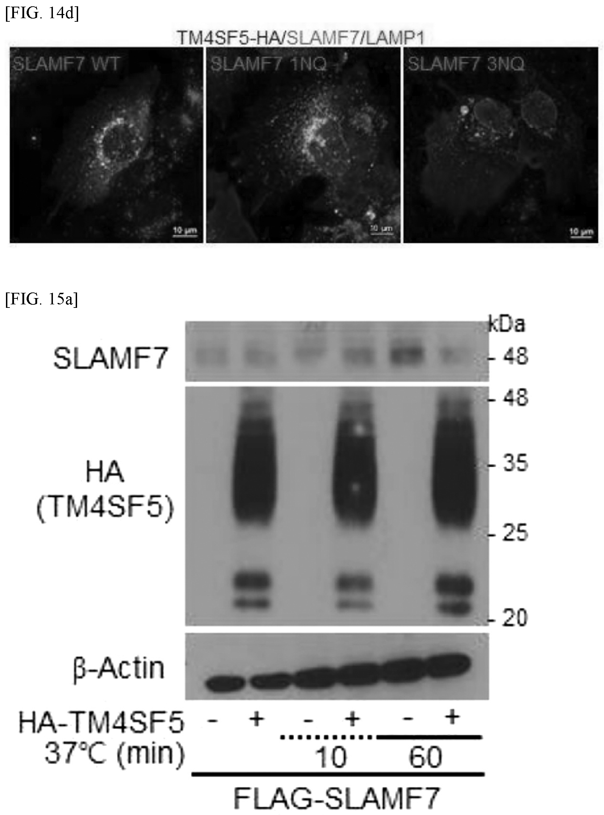

### 2.14. Importance of N-glycosylation of both proteins in the reduction of Slamf7 expression related to TM4SF5-expression dependent natural killer function of NK cells and their binding with TM4SF5 (FIG. 14)

SNU761 hepatocytes were transfected with a control vector (Strep-EV), Strep-TM4SF5 WT, or a Gly- (N-glycosylation-deficient N138A/N155Q mutant) expression vector, or additionally co-transfected with FLAG-SLAMF7 WT, ΔECD1-225 (a mutant in which the extracellular domain of amino acids 1-225 is removed), ΔTM226-247 (a mutant in which the transmembrane domain of amino acids 226-247 is removed), 1NQ (N204Q), 2NQ (N172/N176Q), or 3NQ (N172/176/204Q) expression vectors, and cultured for 48 hours for expression. (a-c) After obtaining whole cell lysates, pulldown was performed using streptavidin-agarose (20353, Thermo Fisher Scientific). Western blotting was performed on the pulled-down samples along with the whole cell lysates. (d) SNU761 cell lines were attached onto cover glasses coated with fibronectin (10 µg/ml, 35600, BD Biosciences) for 16 hours. Thereafter, an HA-TM4SF5 expression vector and a FLAG-SLAMF7 WT, FLAG-SLAMF7-1NQ, or 3NQ (1NQ; N204Q, 3NQ; N172/176/204Q) N-glycosylation mutant expression vector were injected and cultured for 48 hours to allow expression. After fixing the cells with ice-cold 99% methanol for 15 minutes, the cells were blocked with a 1% BSA solution, subjected to immunofluorescence staining using anti-HA (blue), anti-SLAMF7 (green), and anti-LAMP1 (red) antibodies, and observed with a confocal fluorescence microscope (Nikon Eclipse Ti microscope with a C2 confocal system). Representative images for each experimental condition were presented.

As a result, (a) compared to the control EV, SLAMF7 was pulled down together in the sample pulled down with Strep-TM4SF5, confirming that TM4SF5 WT binds to SLAMF7. However, it was confirmed that the TM4SF5 N-glycosylation-deficient mutant (Gly-) does not bind. (b) Meanwhile, it was confirmed that ΔECD₁₋₂₂₅ SLAMF7, which lacks the extracellular domain, binds, but ΔTM₂₂₆₋₂₄₇ SLAMF7, which lacks the transmembrane domain (amino acids 226-247), does not bind to TM4SF5. (c) When the N-glycosylation amino acid residues of SLAMF7 were mutated, the expression level of SLAMF7 did not decrease with the expression of 1NQ, 2NQ, or 3NQ, unlike the case of WT where expression was reduced, and furthermore, it was confirmed that the binding with TM4SF5 WT decreased. (d) SLAMF7 WT appeared to colocalize with TM4SF5 WT at the endosomal membrane site around the cell nucleus, but in the case of the SLAMF7 N-glycosylation mutants (1NQ and 3NQ), the colocalization was not clearly visible. Meanwhile, in the case of 3NQ, unlike WT or 1NQ, it was shown to be uniquely located at sites of liquid-liquid phase separation within the cell, forming isolated circular shapes.

Accordingly, (a-c) it is suggested that the N138/N155 N-glycosylation and surrounding structural aspects of TM4SF5, as well as the N-glycosylation and transmembrane domain region of SLAMF7, play important roles in the binding between TM4SF5 and SLAMF7. (d) It is presumed that in liver cancer cells, TM4SF5 colocalizes with SLAMF7 and LAMP1 in the endosomal membrane or lysosomes, thereby enabling the degradation of SLAMF7 mediated by TM4SF5.

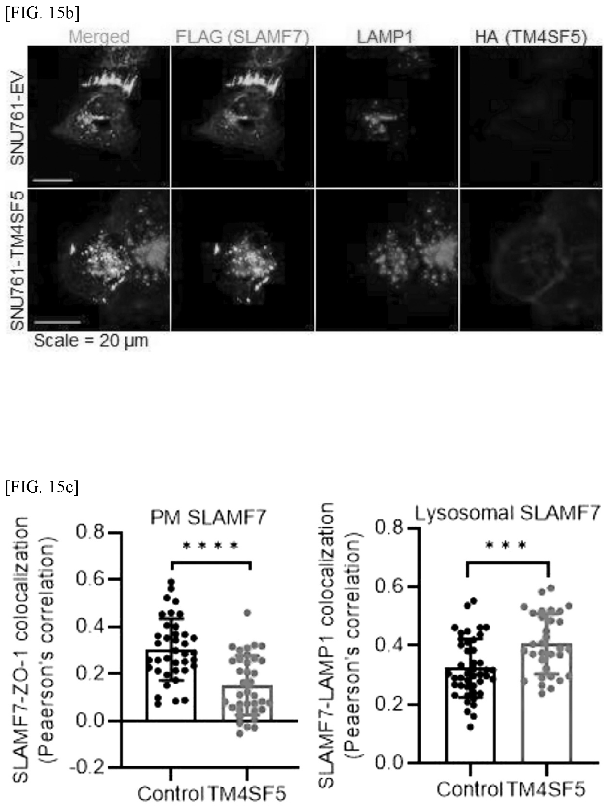

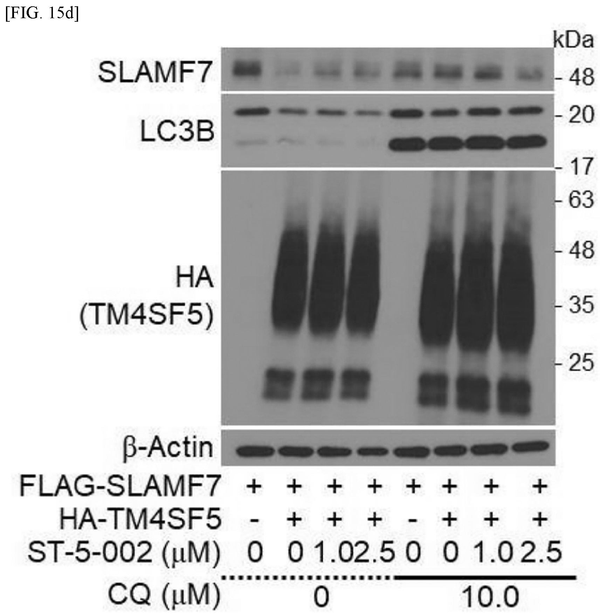

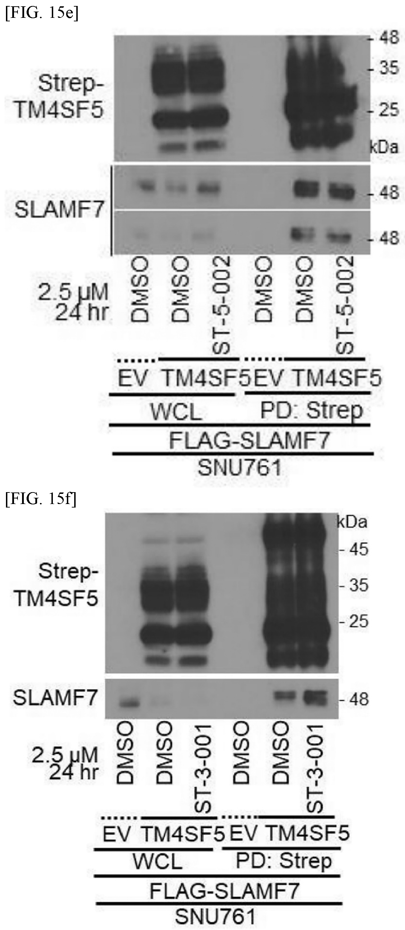

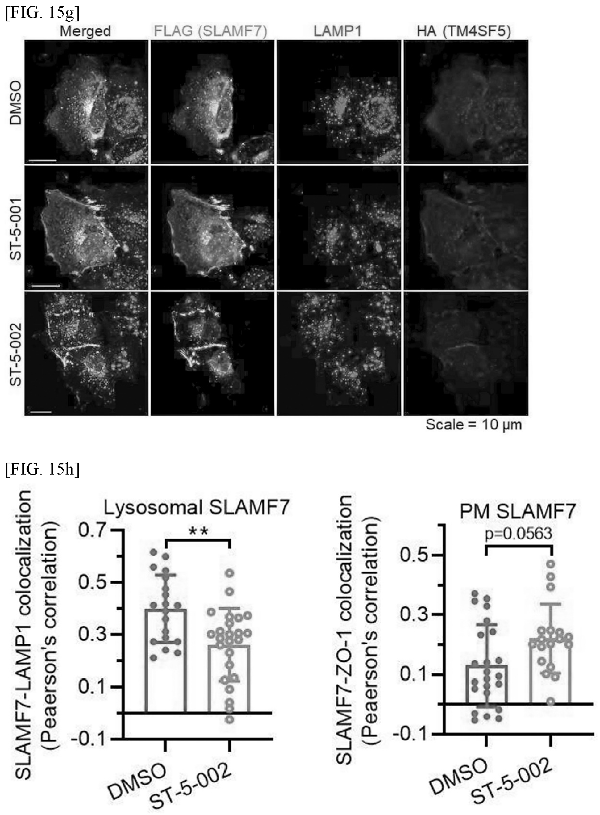

### 2.15. Reduction in expression, degradation, and regulation of intracellular localization of ligands related to TM4SF5-expression dependent natural killer function of NK cells by isoxazole compounds (FIG. 15)

(a) SNU761 cells expressing HA-EV or HA-TM4SF5 and FLAG-SLAMF7 were stored at 4°C and then cultured at 37°C for the given periods. Thereafter, cell extracts were obtained, and Western blotting was performed with the indicated antibodies. (b-c) SNU761 cells expressing HA-TM4SF5 and FLAG-SLAMF7 were immunostained with HA, FLAG, and LAMP1 or ZO-1 antibodies. The colocalization coefficient was calculated by Pearson's correlation (b). Each dot represents the values of cells obtained from three independent experiments, and the figures show representative images (c). (d) SNU761 cells expressing HA-EV/TM4SF5 and FLAG-SLAMF7 were treated or not treated with ST-5-002 and chloroquine (CQ, 10 µM) for 24 hours. Thereafter, cell extracts were obtained, and Western blotting was performed with the indicated antibodies. (e-f) After treating SNU761 cells expressing TM4SF5-Strep and FLAG-SLAMF7 with ST-5-002 (e) or ST-3-001 (f) for 24 hours, cell extracts were obtained using brij58 lysis buffer. Thereafter, the whole-cell lysates were used for streptavidin pulldown and immunoprecipitation. (g-h) SNU761 cells expressing HA-TM4SF5 and FLAG-SLAMF7 were treated with ST-5-001 or ST-5-002 (2.5 µM, 24 h). Thereafter, the cells were fixed with cold methanol and immunostained with the indicated antibodies. The figures show representative images (g). The colocalization coefficient was calculated by Pearson's correlation (h). Each dot represents the values of cells obtained from three independent experiments.

As a result, (a) as time passed after moving SNU761 cells that do not express or express TM4SF5 from 4°C to 37°C, the expression pattern of SLAMF7 increased in the case where TM4SF5 was not expressed, but in the case where TM4SF5 was expressed, it did not increase and rather showed a decrease. (b-c) In the control group where TM4SF5 was not expressed, SLAMF7 was highly expressed/localized at the plasma membrane (PM), but as TM4SF5 was expressed, the amount of expression/localization at the PM decreased, while the amount localized at the lysosomal membrane stained with LAMP1 increased. (d) FLAG-SLAMF7 and HA-TM4SF5 or a control HA expression vector were injected into SNU761 cells, and ST-5-002 was not treated or treated for 24 hours at given concentrations simultaneously with chloroquine (CQ). Cell extracts were obtained to confirm the expression patterns of SLAMF7 and LC3B, a marker for autophagy. At this time, SLAMF7, which had decreased due to the expression of TM4SF5, showed a pattern of recovery by the treatment with ST-5-002, and particularly in cases where CQ was treated, the reduction in SLAMF7 expression was not clearly seen. (e-f) The binding between TM4SF5 and SLAMF7 decreased upon treatment with ST-5-002, and at this time, the expression level of SLAMF7, which had decreased according to the expression of TM4SF5 (in the case of WCL), showed a pattern of increasing again according to the drug treatment (e). However, when the control drug ST-3-001 was treated, the binding between TM4SF5 and SLAMF7 did not decrease but rather showed an increasing trend, and in the case of WCL, the expression of SLAMF7 only decreased and did not show a recovery according to the ST-3-001 treatment (f). (g-h) After injecting FLAG-SLAMF7 and HA-TM4SF5 or a control HA expression vector into SNU761 cells and treating with DMSO, ST-5-001, or ST-5-002 as a control, SLAMF7-FLAG (green), LAMP1 (red), and HA-TM4SF5 (blue) were fluorescently stained, and the degree of localization change or colocalization of FLAG-SLAMF7 and HA-TM4SF5 was quantified. Compared to the DMSO treatment condition, it was confirmed that SLAMF7, which had been strongly colocalized at the lysosomal membrane, decreased upon drug treatment, while the expression/localization at the plasma membrane (PM) rather increased.

Accordingly, (a-c) it was confirmed that SLAMF7 is degraded in lysosomes, considering that the binding between TM4SF5 and SLAMF7 is accompanied by a translocation to the lysosomal membrane and a decrease in the expression level of SLAMF7, and that the phenomenon can be blocked with CQ. At this time, the TM4SF5-specific inhibitor ST-5-002, unlike the control drug ST-3-001, was able to inhibit the degradation of SLAMF7 by inhibiting this TM4SF5-mediated phenomenon. Meanwhile, it was identified that when ST-5-001 or ST-5-002 is treated, the binding of SLAMF7 with TM4SF5 is disrupted, causing it to move from the lysosomal membrane to the plasma membrane and thus not undergo the degradation process.

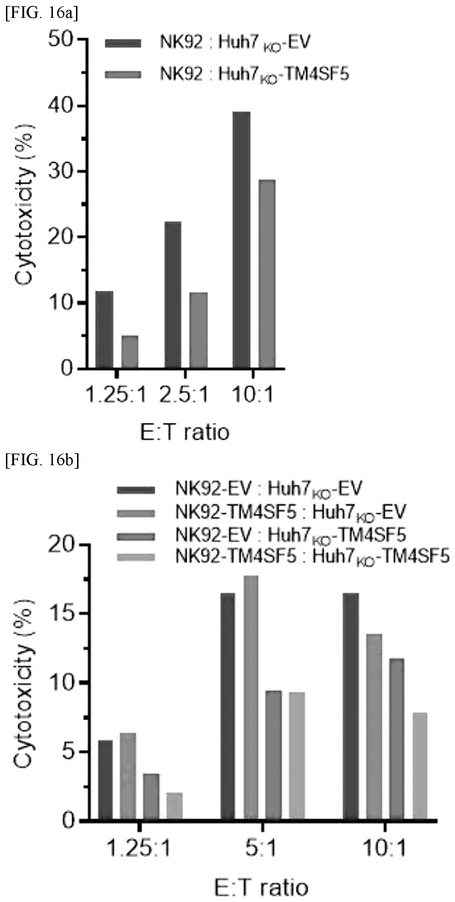

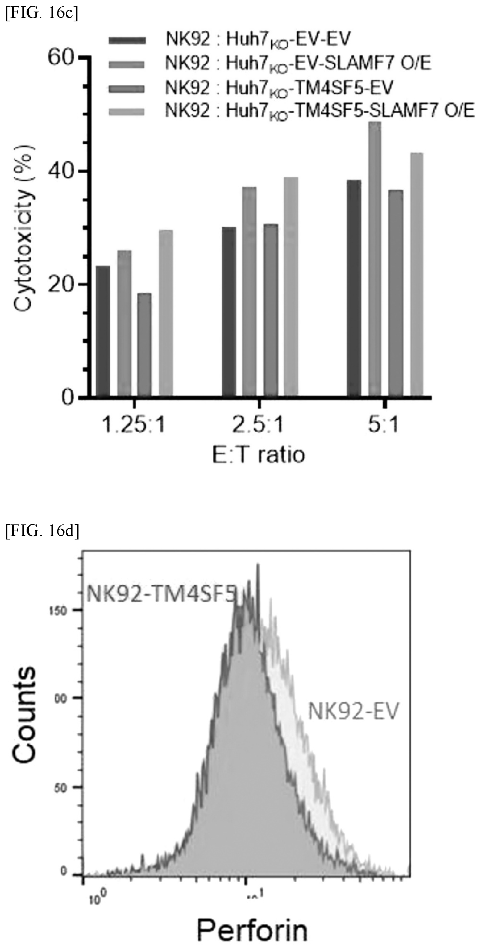

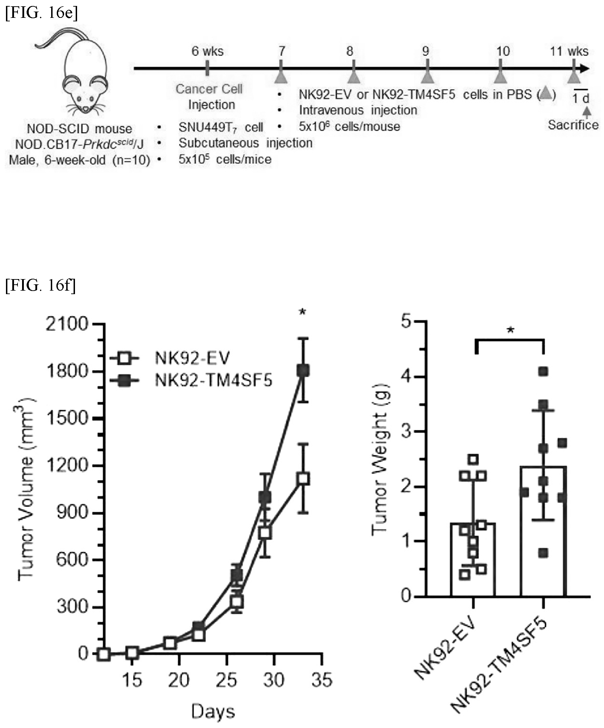

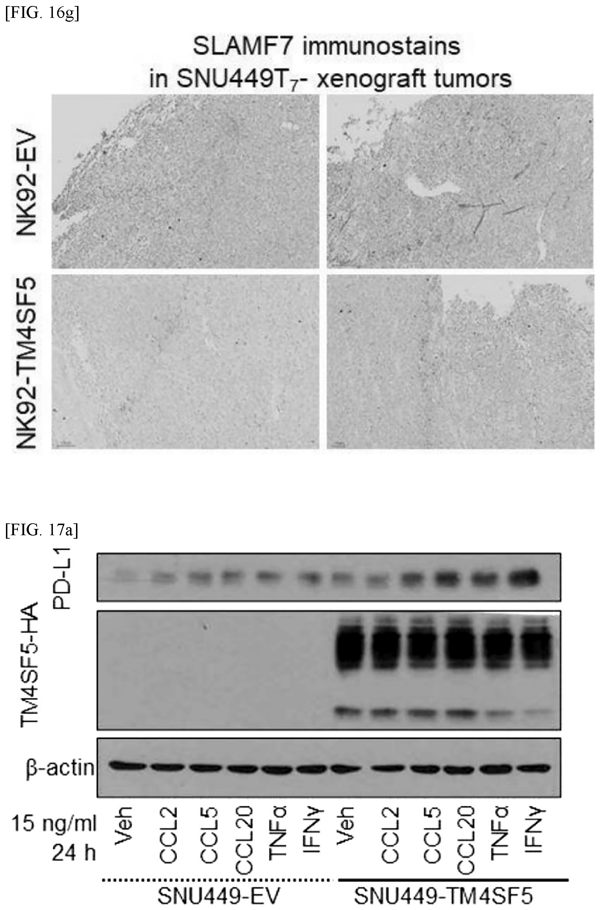

### 2.16. Reduction of natural killer function of NK cells by TM4SF5 expression (FIG. 16)

Stable NK92 cell lines were obtained by infecting human NK cells, NK92, with a retrovirus inducing the expression of pBabe-HA-EV or pBabe-HA-TM4SF5. Additionally, Huh7_{KO}-EV or Huh7_{KO}-TM4SF5 cell lines were transfected with a SLAMF7 expression vector for overexpression (O/E). (a-b) NK92-EV or NK92-TM4SF5 cells (Effector cells, E) and Huh7_{KO}-EV or Huh7_{KO}-TM4SF5 cell lines (Target cells, T) were mixed in a 96-well plate at a given E:T ratio in triplicate and cultured for 4 hours. Thereafter, the cytotoxicity of the NK92 cells was measured using a lactate dehydrogenase (LDH) cytotoxicity assay kit (Cytotoxicity Detection Kit Plus, Roche) according to the protocol. (c) The natural killer function/efficacy was measured using NK92 (e) cells and Huh7_{KO}-EV-SLAMF7 (O/E) or Huh7_{KO}-TM4SF5-SLAMF7 (O/E) cells. (d) The production of perforin in NK92-EV or NK92-TM4SF5 cells was analyzed by flow cytometry after staining with an anti-Perforin antibody. (e-g) Six-week-old male NOD-SCID mice were subcutaneously injected with TM4SF5-expressing SNU449T₇ cells (5 X 10⁵ cells/mice), and starting from one week later, NK92-EV or NK92-TM4SF5 cells (5 X 10⁶ cells/mice) were intravenously injected 5 times, once a week until the 11th week. 24 hours after the fifth and final injection of NK92 cells, the mice were sacrificed to analyze liver tumors and furthermore, immunohistochemistry was performed.

As a result, (a-b) it was confirmed that the natural killer efficacy of NK92 cells was lower in the Huh7_{KO}-TM4SF5 cell line than in Huh7_{KO}-EV. The NK92-TM4SF5 cells also showed the same phenomenon. The natural killer efficacy was lowest when NK92-TM4SF5 and Huh7_{KO}-TM4SF5 were allowed to meet. (c) However, the natural killer efficacy of NK92 cells against liver cancer cells was increased in the case of liver cancer cells overexpressing SLAMF7. (d) It was confirmed that the expression/production of Perforin was lower in NK92-TM4SF5 cells than in NK92-EV cells. (e-g) In the cancer development model created by subcutaneous injection of TM4SF5-expressing liver cancer cells, the tumor volume and weight were higher when the NK92-TM4SF5 cell line was injected than when NK92-EV cells were injected. Furthermore, because the NK92-EV or NK92-TM4SF5 cell lines were injected, the immunohistochemical staining of SLAMF7 appeared similarly in the liver tissues of the NK cell-deficient NOD-SCID mice.

Accordingly, (a-d) these results suggest that the efficacy of NK92 cells in killing liver cancer cells is lower when TM4SF5 is expressed in either the NK92 cells or the hepatic (cancer) epithelial cells than when it is not, which leads to the presumption that TM4SF5 expression can inhibit the SLAMF7-mediated natural killer function of NK cells, whether in liver cancer cells or NK cells. Moreover, the confirmation that the injection of NK92-TM4SF5 cells promoted cancer development more actively than the intravenous injection of NK92-EV cells in an animal cancer model created by subcutaneous injection of TM4SF5-expressing hepatocytes indicates that NK cells expressing TM4SF5 have a reduced anti-cancer natural killer efficacy.

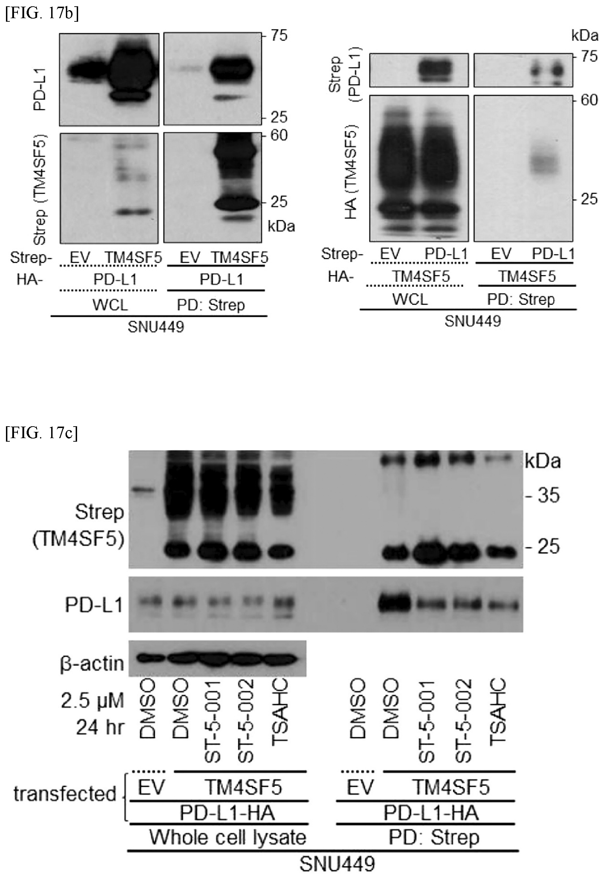

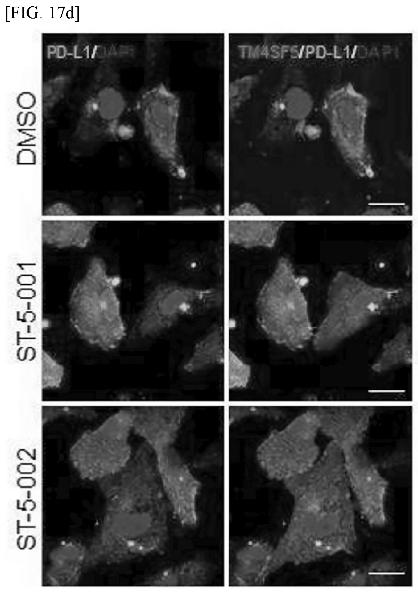

### 2.17. Confirmation of inhibition of TM4SF5-expression dependent PD-L1 expression and binding by isoxazole compounds (FIG. 17)

(a-b) The same number of SNU449-EV or SNU449-TM4SF5 cells were transferred to culture vessels and cultured overnight. After replacing the cell culture medium with a new normal culture medium, vehicle, CCL2, CCL5, CCL20, TNFα, or IFNγ was treated at a concentration of 15 ng/ml each for 24 hours. Thereafter, cell extracts were obtained and Western blotting was performed with the given antibodies, or TM4SF5-strep was pulled down with streptavidin agarose resin, and then Western blotting for co-existing PD-L1 was performed using its antibody, thereby confirming the binding between TM4SF5 and PD-L1. (c-d) PD-L1-HA cDNA was additionally injected into SNU449 cells stably expressing EV or TM4SF5. After one day, DMSO, TSAHC (chalcone-based positive control drug), ST-5-001, or ST-5-002 was treated at 2.5 µM for 24 hours, and then cell extracts were obtained. From the cell extracts, Strep-TM4SF5 was pulled down with strep-agarose beads, and then Western blotting was performed using antibodies against the indicated proteins, or the intracellular localization and expression level of TM4SF5 (red fluorescence) and PD-L1 (green fluorescence) were confirmed with a fluorescence microscope through immunostaining along with DNA staining using DAPI.

As a result, (a) compared to the SNU449-EV cells treated only with the DMSO vehicle as a control without drug treatment, it was confirmed that the expression of PD-L1 increased in the case of SNU449-TM4SF5 cells. However, when various cytokines/chemokines were additionally treated, the increase in PD-L1 expression due to their treatment was minimal in the case of SNU449-EV, but in the case of SNU449-TM4SF5 cells, it was confirmed that the degree of increase was much greater when CCL5, CCL20, TNFα, or IFNγ was treated. (b) SNU449 cells were transfected with Strep-EV, HA-EV, Strep-TM4SF5 (TM₅), HA-PD-L1, Strep-PD-L1, or HA-TM4SF5 (TM₅) individually or together. After 48 hours, cell extracts were obtained, and Strep-TM4SF5 or Strep-PD-L1 was pulled down from the cell extracts. Strep-TM4SF5 and Strep-PD-L1 were each pulled down, and at this time, it was confirmed that PD-L1 and TM4SF5 were pulled down together in each experiment. By this, it was confirmed that TM4SF5 binds to PD-L1. (c) Similar to the inhibition by TSAHC treatment compared to the DMSO-treated control group, this binding between TM4SF5 and PD-L1 was decreased and inhibited by the treatment with the drug ST-5-001 or ST-5-002. (d) When observed with a fluorescence microscope through immunostaining, the cells of the control group treated with DMSO showed high expression of PD-L1 in cells expressing TM4SF5, and colocalization at the cell membrane site was confirmed; however, when the isoxazole drug was treated, it was confirmed that the expression of PD-L1 rather decreased in cells expressing TM4SF5, and the degree of colocalization was minimally inhibited.

SLAMF7 is known as a membrane protein that induces activation, and PD-L1 is known as a membrane protein that can inhibit the immune activity of NK cells and T cells by inhibiting activation. While TM4SF5 can bind to it and the expression of SLAMF7 (along with SLAMF6, MICA, MICB, etc.) decreases in liver cancer cells/tissues expressing TM4SF5, PD-L1 is an immune checkpoint that inhibits the activity of T and NK immune cells, and its expression will increase in liver cancer cells/tissues expressing TM4SF5. Therefore, it was confirmed whether the expression pattern of PD-L1, which is known to be expressible not only in hepatocytes (representative parenchymal cells) but also in non-parenchymal cells [endothelial cells, fibroblasts, and hepatic stellate cells] and immune cells (immune cells such as T cells, B cells, NK cells, and macrophages) in liver tissue, correlates with the expression of TM4SF5 or binds with TM4SF5. Also, the inhibition of the binding between PD-L1 and TM4SF5 according to the treatment with the TM4SF5-specific inhibitor isoxazole may be importantly related to the regulation of PD-L1 expression or intracellular localization/distribution in hepatocytes. The fact that the isoxazole compound can control the signaling function and protein-protein binding ability of TM4SF5, which shows a correlation in the regulation of PD-L1 expression, suggests the potential of the isoxazole compound as a TM4SF5-specific inhibitor and an NK cell and T cell immune checkpoint inhibitor.

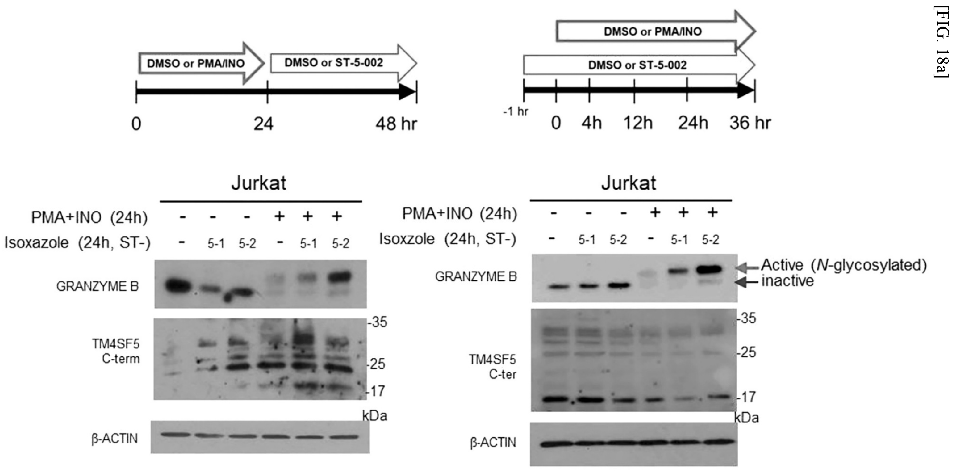

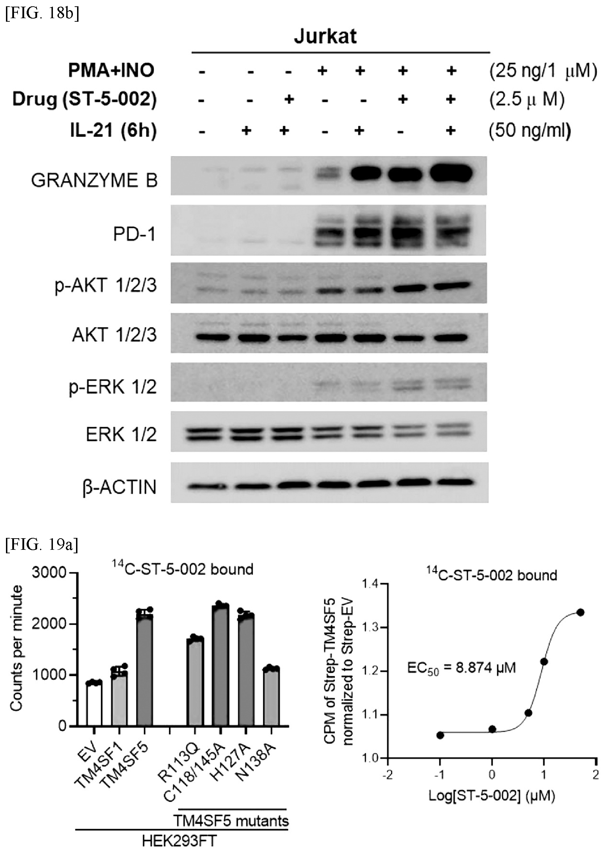

### 2.18. Confirmation of inhibition of TM4SF5 expression dependent PD-1 expression and T cell activity by isoxazole compounds (FIG. 18)

(a) Human Jurkat T cells were activated by treating with PMA (10 ng/ml) and INO (1 µg/ml) for 24 hours. A non-treated state was also included as a control. Meanwhile, it was confirmed that the production of Granzyme B (especially the intensity of the band with a larger molecular weight in the form where N-glycosylation occurred upon activation) gradually increased through PMA/INO treatment. Even when the activation time through PMA/INO treatment preceded or coincided with the time of treating with DMSO or ST-5-002, it was confirmed that the activation by PMA/INO was further increased by the treatment with ST-5-001 or ST-5-002. (b) Human Jurkat T cells were either not treated or treated with PMA (25 ng/ml) and INO (1 µg/ml) for 24 hours, and IL-21 was treated at a level of 50 ng/ml for the last 6 hours. Simultaneously with the PMA/INO treatment, ST-5-002 was treated or not treated at 2.5 µM. At this time, the expression levels of Granzyme B and PD-1, or the phosphorylation of Ser473 of AKTs and Thr202/Tyr204 of ERKs, were enhanced by PMA/INO. When PMA/INO was treated, additional treatment with IL-21 or ST-5-002 alone further increased the expression of Granzyme B and PD-1, and it was confirmed that the level of Granzyme B became even higher when ST-5-002 and IL-21 were additionally treated simultaneously.

Accordingly, it is suggested that even in the case of human Jurkat T cells, when activated by treatment with PMA/INO, the activation of T cells in terms of Granzyme B expression is further achieved by additionally treating with ST-5-002, a TM4SF5-specific inhibitor. That is, the fact that the isoxazole compound can improve the T cell proliferation/survival signaling function and cell-killing ability for the expression of PD-1 and Granzyme B, which increase during the activation process in T cells, suggests the potential of the isoxazole compound as a TM4SF5-specific inhibitor and a T cell immune checkpoint inhibitor.

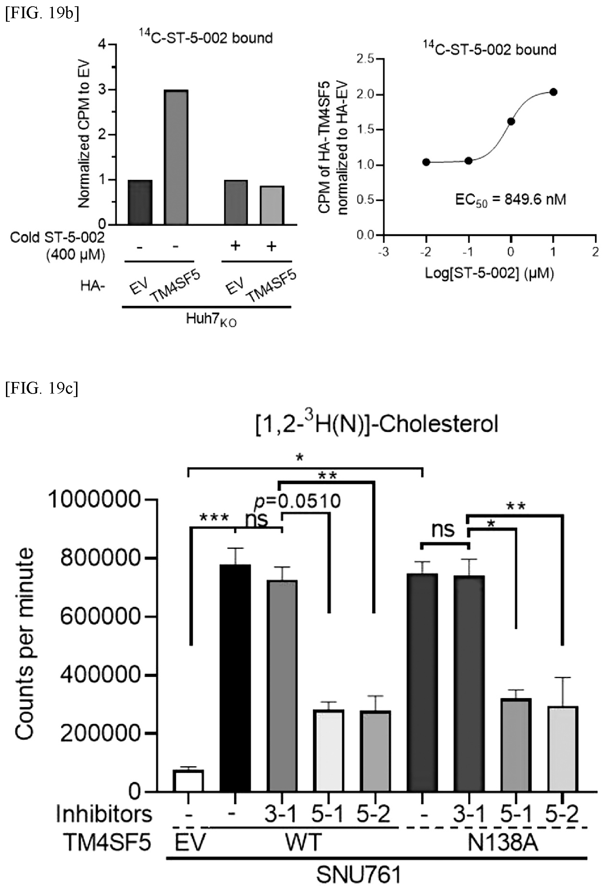

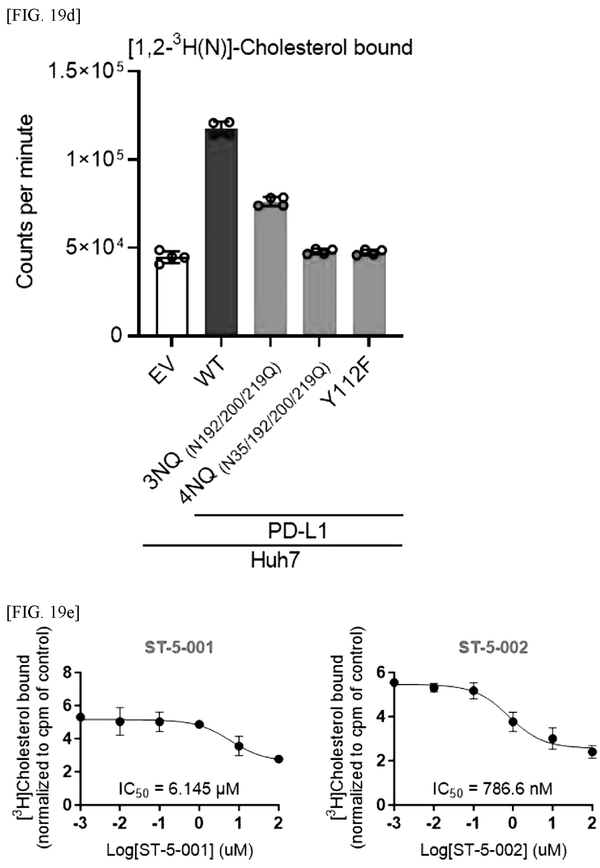

### 2.19. Inhibition of cholesterol binding capacity of TM4SF5 and PD-L1 by isoxazole binding to TM4SF5 and isoxazole treatment (FIG. 19)

(a) Expression vectors for a control vector, TM4SF1, TM4SF5 WT, and R113Q, C118/145A, H127A, and N138A mutants of TM4SF5 were injected into the HEK293FT cell line, which was confirmed not to express TM4SF5, and allowed to express for 48 hours. (b) Huh7_{KO} cell lines, in which TM4SF5 was knocked out in Huh7, were stably infected with a retrovirus encoding pBabe-EV or pBabe-TM4SF5-HA. (c) Strep-EV, Strep-TM4SF5 WT, or N138A mutant expression vectors were injected into the SNU761 cell line, which does not express TM4SF5, to be expressed. (d) Strep-EV, Strep-PD-L1 WT, and 3NQ (N192/200/219Q) and 4NQ (N35/192/200/219Q) *N*-glycosylation mutants were injected into Huh7 cells to be expressed. (e) Strep-EV and Strep-TM4SF5 expression vectors were injected into Huh7_{KO} cells and allowed to express for 48 hours. (a-e) After obtaining cell extracts, TM4SF5-strep, TM4SF5-HA, or PD-L1-strep was pulled down with streptavidin agarose resin or anti-HA antibody from extracts containing 0.2, 0.25, or 1.0 mg of protein for each experimental condition. Each of the pulled-down beads was treated with ¹⁴C-labelled ST-5-002 (10, 25, or various indicated concentrations, µM) for 1 hour in the presence or absence of 400 µM of cold ST-5-002 (a-b). Alternatively, they were treated with 1,2-³H-Cholesterol (50 µM) alone or together with ST-3-001 (negative control compound), ST-5-001, or ST-5-002 (2.5 µM each) for 1 hour (c-e). For the experimental samples of each condition, radioactive isotope values were repeatedly measured using a TriCarb scintillation counter (PerkinElmer, Waltham, MA).

As a result, (a) compared to the control and TM4SF1, Strep-TM4SF5 obtained from HEK293FT bound to ¹⁴C-labelled ST-5-002. In particular, it was confirmed that the amino acids R113 and N138 of TM4SF5 are important for the binding. Meanwhile, when reacted with various concentrations of ¹⁴C-labelled ST-5-002, the EC₅₀ of binding to TM4SF5 (the concentration of ¹⁴C-labelled ST-5-002 that binds to 50% of TM4SF5) showed a value of 8.874 µM. (b) It was confirmed that the binding of ¹⁴C-labelled ST-5-002 to HA-TM4SF5 obtained from Huh7_{KO}-HA-TM4SF5 was decreased by simultaneous treatment with cold ST-5-002. Also, when reacted with various ¹⁴C-labelled ST-5-002, the EC₅₀ of binding to TM4SF5 showed a value of 849.6 nM. (c) The wild-type and N138A mutant forms of TM4SF5 could also effectively bind to cholesterol. Meanwhile, the binding of TM4SF5 to cholesterol was not affected by the control drug ST-3-001, but was inhibited by the treatment with ST-5-001 and ST-5-002. (d) Like TM4SF5, PD-L1 WT also binds to cholesterol, and in particular, the binding with cholesterol was inhibited in the N-glycosylation mutant and the phosphorylation mutant Y112F. (e) It was confirmed that the degree of TM4SF5 binding to cholesterol decreased as the concentration of simultaneously treated ST-5-001 or ST-5-002 increased. It was confirmed that the degree of binding between TM4SF5 and cholesterol was reduced by 50% at 6.145 µM for ST-5-001 and at 786.6 nM for ST-5-002.

Accordingly, the TM4SF5-specific inhibitors isoxazole ST-5-001 and ST-5-002 bind to TM4SF5, and the EC₅₀ of the binding showed values of 8.874 µM for Strep-TM4SF5 and 874.6 nM for HA-TM4SF5, showing EC₅₀ values of 0.85 to 8.8 µM depending on the cell source and the type of tag. Meanwhile, cholesterol can also bind to TM4SF5 and PD-L1, which are known to be involved in the pathogenesis of cancer, and it was confirmed that ST-5-001 and ST-5-002 inhibit that binding. Although it has not been confirmed what significance the binding of cholesterol to TM4SF5 and PD-L1 has when the cytotoxic activity of T cells and NK cells is regulated dependently on TM4SF5, the fact that the TM4SF5-specific inhibitor isoxazole compound can inhibit that binding allows for the presumption of or enables an additional mechanism of action for an anti-liver cancer strategy.

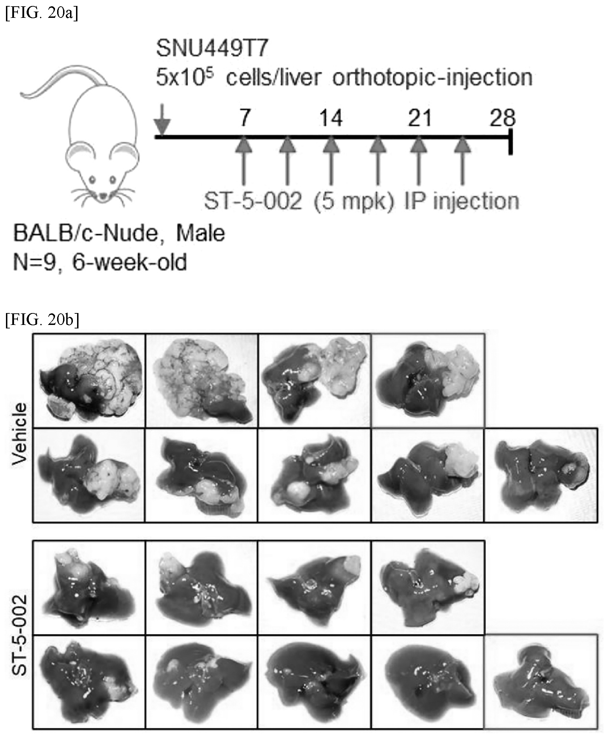

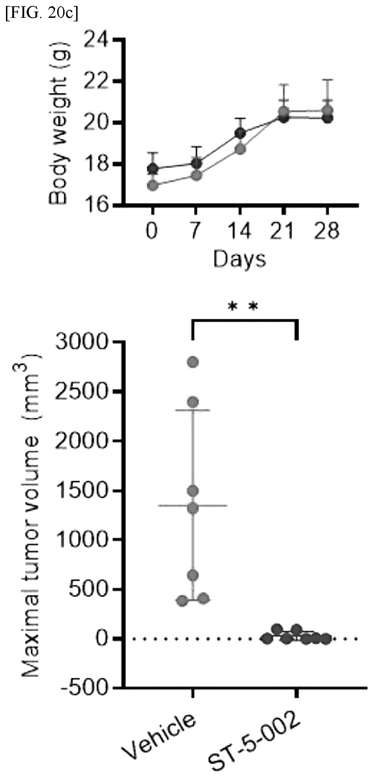

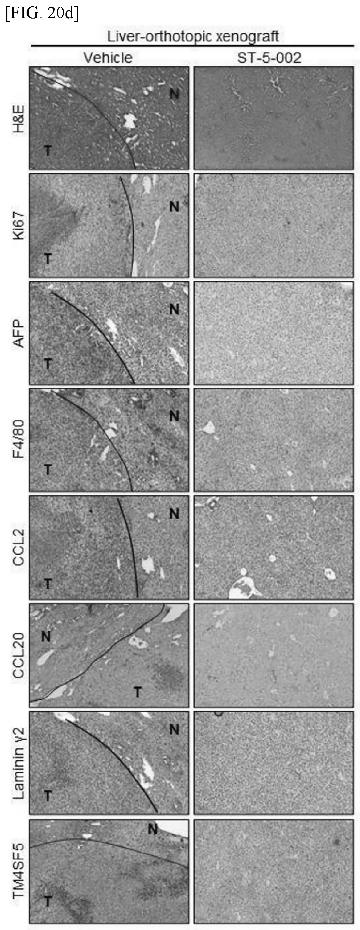

### 2.20. Confirmation of inhibition of liver cancer formation and expression of liver cancer markers by isoxazole compounds in a liver-orthotopic xenograft model of a TM4SF5-expressing cell line (FIG. 20)

(a-d) A liver-orthotopic xenograft model was established by directly injecting 5x10⁵ SNU449_{T7} cells into the livers of 6-week-old male BALB/c-Nude mice (n=9), which are deficient in T and B cells but still capable of activating NK cell functions. Starting one week after the cell injection, the drug ST-5-002 was intraperitoneally injected at 5 mg/kg twice a week (a). After a total of 6 injections over 3 weeks, the animals were sacrificed at the 4th week of cell injection, and liver tissues were obtained and images of the livers were taken (b). Body weight was measured every week during the drug injections. Meanwhile, although cancer formation occurred in several places in the liver tissue, the cancer masses were quite large, and since it was practically difficult to measure the size or volume of small cancer masses due to time constraints for tissue specimen processing, the volume of the largest cancer mass was measured for each liver tissue. The tumor volumes calculated from n=7 liver tissues, excluding the maximum and minimum values, are shown (c). (d) For a portion of the liver tissues, the expression level, distribution, and localization of each factor, as well as the degree of damage to the liver cancer tissue, were confirmed through immunohistochemistry or H&E staining for the given protein factors.

As a result, (a) is a schematic diagram of the liver-orthotopic xenograft experimental method for the TM4SF5-expressing SNU449T₇ cell line. (b) When checking the images of the recovered cancer tissues after the end of the experiment, it was confirmed that the inhibitory effect on liver cancer formation was distinct when treated with ST-5-002 compared to the tumor tissues of the control group injected with the vehicle. (c) Body weight was measured every week after the cell injection, and on the 28th day after the cell injection, liver cancer tissues were obtained, and the size of the largest cancer mass in each liver tissue was measured to calculate the tumor volume. It was confirmed that the tumor volume was significantly reduced in the case of drug treatment compared to the case of vehicle treatment. (d) As a result of performing immunohistochemistry or H&E staining for the given protein factors using a portion of the liver tissue, cancer formation in the liver tissue was inhibited when treated with the drug ST-5-002 compared to the case of vehicle treatment, and it was confirmed that the expressions of Ki67 representing cell proliferation, AFP as a liver cancer marker, F4/80 representing the distribution of macrophages in the liver tissue, inflammation-related CCL2 and CCL20, and laminin γ2 related to fibrosis and liver cancer were significantly lower.

Accordingly, it was intended to confirm the TM4SF5 expression-dependent liver cancer formation inhibitory efficacy of isoxazole compounds that underwent the SAR process of ST-1-025 → ST-2-001 → ST-3-006/011 → ST-4-005 → ST-5-001 or ST-5-002. In an animal liver cancer model obtained by directly transplanting TM4SF5-expressing liver cancer cells into the liver, ST-5-002 showed the efficacy of inhibiting liver cancer formation, thereby confirming the potential of isoxazole compounds as TM4SF5-specific inhibitors and anti-liver cancer agents.

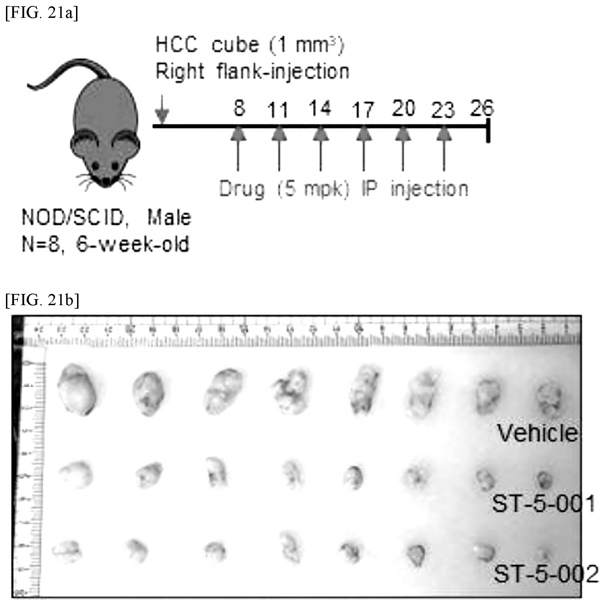

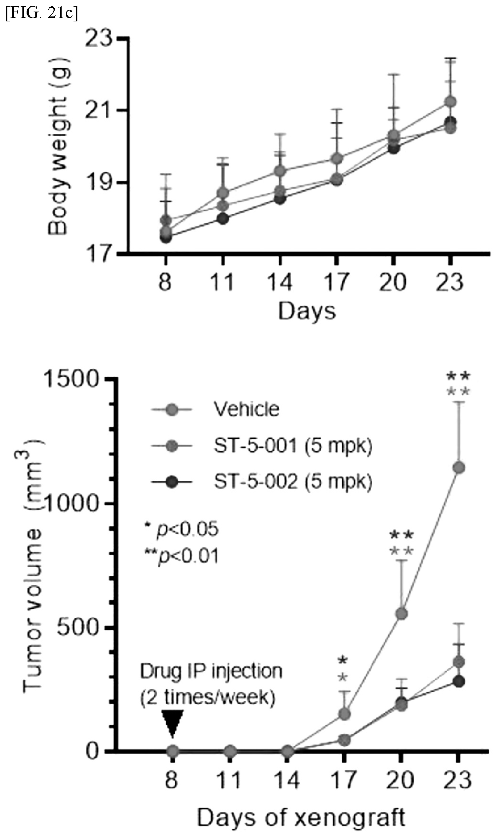

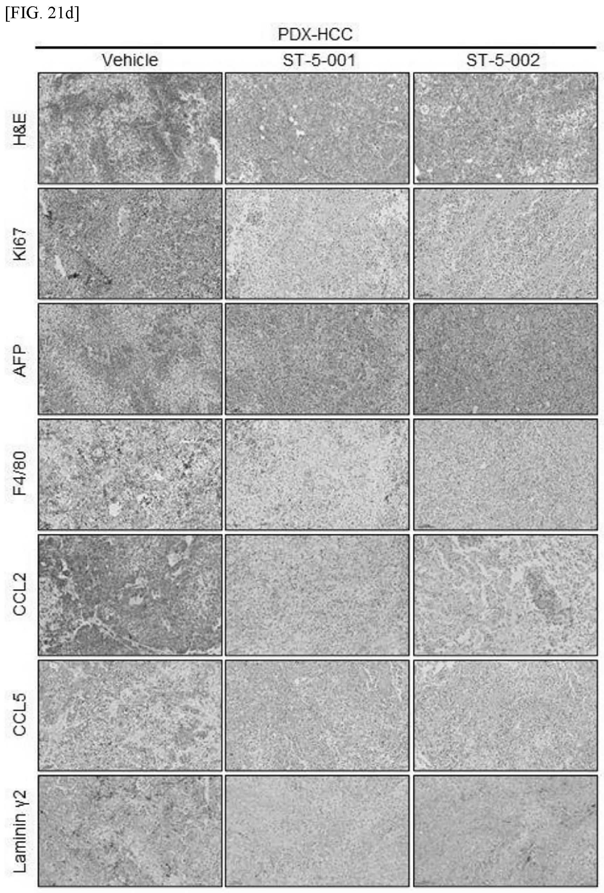

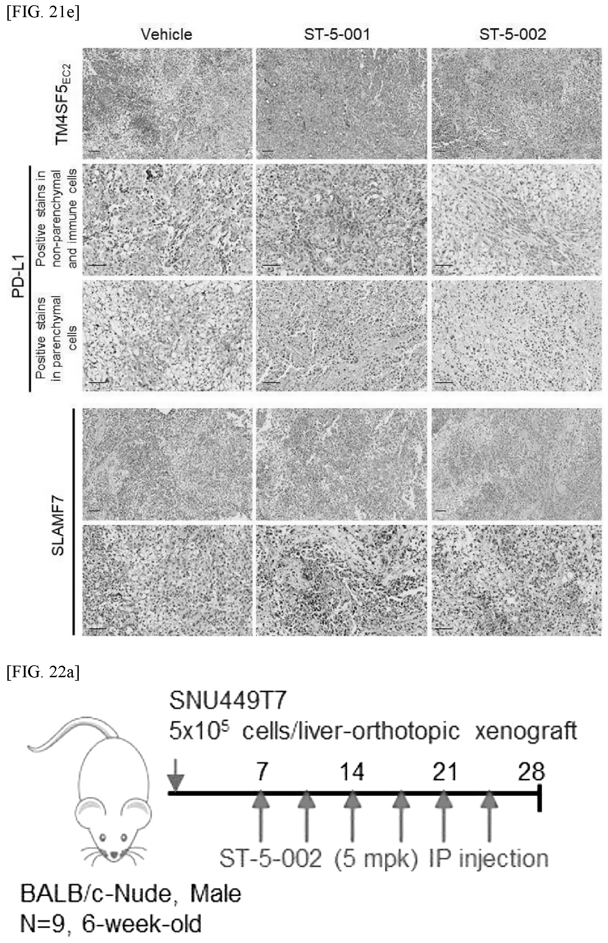

### 2.21. Confirmation of inhibition of liver cancer formation and expression of liver cancer markers by isoxazole compounds in a TM4SF5-expressing PDX model (FIG. 21)

(a-d) A TM4SF5-expressing HCC-PDX model was established by implanting liver cancer patient-derived cancer fragments (HCC cube, 1 mm³), confirmed to express TM4SF5, into the right thigh of 6-week-old male NOD/SCID mice (n=8) deficient in T, B, and NK immune cells. Starting from the 8th day after the tissue implantation, vehicle, drug ST-5-001, or ST-5-002 was intraperitoneally injected at 5 mg/kg twice a week on the indicated dates (a). After 6 injections, the animals were sacrificed on the 28th day after the cancer tissue implantation, liver tissues were obtained, and images of the livers were taken (b). From the 8th day after the implantation, body weight and tumor volume were measured and recorded on each drug injection day (c). Meanwhile, for a portion of the liver tissues, the expression level, distribution, and localization of each factor, as well as the degree of damage to the liver cancer tissue, were confirmed through immunohistochemistry or H&E staining for the given protein factors (d).

As a result, (a) is a schematic diagram of the PDX experimental method for creating a liver cancer model by implanting liver cancer patient-derived cancer fragments confirmed to express TM4SF5. (b) When checking the images of the obtained cancer tissues after the end of the experiment, it was confirmed that the inhibitory effect on liver cancer formation was distinct when treated with ST-5-002 compared to the tumor tissues of the control group injected with the vehicle. (c) When measuring body weight every week after the injection of cells, the body weight was not affected by the drug treatment, and it was confirmed that the tumor volume was significantly reduced in cases where each drug was treated compared to the case of vehicle treatment. (d) As a result of performing immunohistochemistry or H&E staining for the given protein factors using a portion of the liver tissue, cancer formation was inhibited when treated with drug ST-5-001 or ST-5-002 compared to the case of vehicle treatment, and it was confirmed that the expressions of Ki67 representing cell proliferation, AFP as a liver cancer marker, F4/80 representing the distribution of macrophages inside the liver tissue, inflammation-related CCL2 and CCL5, and laminin γ2 related to fibrosis and liver cancer were significantly inhibited and lowered. (e) The expression of TM4SF5 was not significantly different depending on the drug treatment, being high in both the vehicle and drug-treated groups. When staining for PD-L1, based on the cell size, morphology, and staining pattern, the PD-L1 expression in non-parenchymal cells and immune cells, as well as in parenchymal cells such as hepatocytes, was high in the vehicle-treated case, but was confirmed to decrease when the drugs were treated. Meanwhile, in the case of SLAMF7, the expression of SLAMF7 was higher in the non-cancerous area surrounding the cancer site than in the cancer site itself in the vehicle-treated case, but in the drug-treated case, the staining level showed a pattern of SLAMF7 staining infiltrating into the cancer site, even though it was a small cancer mass.

Accordingly, to analyze the TM4SF5 expression-dependent liver cancer formation inhibitory efficacy of isoxazole compounds that underwent the SAR process of ST-1-025 → ST-2-001 → ST-3-006/011 → ST-4-005 → ST-5-001 or ST-5-002, a liver cancer model obtained by transplanting patient-derived liver cancer tissues was established using NOD/SCID mice in which the functions of T, B, and NK immune cells were inhibited. In the liver cancer model obtained by directly transplanting patient-derived liver cancer tissues expressing TM4SF5 into the liver, ST-5-001 or ST-5-002 showed efficacy in significantly inhibiting liver cancer formation. This suggests that the expression of PD-L1, which is expressed not only in hepatocytes as the source of liver cancer but also in various other types of cells constituting liver tissue and inhibits the killing function of T and NK cells, remained high before drug treatment, potentially leading to immune evasion; however, the expression decreased after drug treatment, which could have led to the activation of T/NK cells. That is, by inhibiting the distribution and number of non-parenchymal and immune cells as well as PD-L1-expressing hepatic epithelial cells, the potential for anti-liver cancer inhibition through immune checkpoint inhibition by the isoxazole compound as a TM4SF5-specific inhibitor was confirmed. Meanwhile, in the case of SLAMF7, the expression decreased in the cancer site, and upon drug treatment, the overall expression increased or showed a certain degree of staining even inside the cancer site, which means that the up-regulation of SLAMF7 expression in hepatic epithelial cells by the drug can trigger the activity of immune cells such as NK cells, serving as a fundamental mechanism of action for seeking anti-cancer effects. However, since the animal model in this experiment lacks or has minimal T, B, and NK cells, the anti-cancer effect obtained in the drug-treated cases might not have been entirely due to the activation of those immune cells; rather, it was presumed that oncogenesis, cancer cell survival, and proliferation could have been inhibited by suppressing TM4SF5 signaling activity and protein-protein binding by the drug.

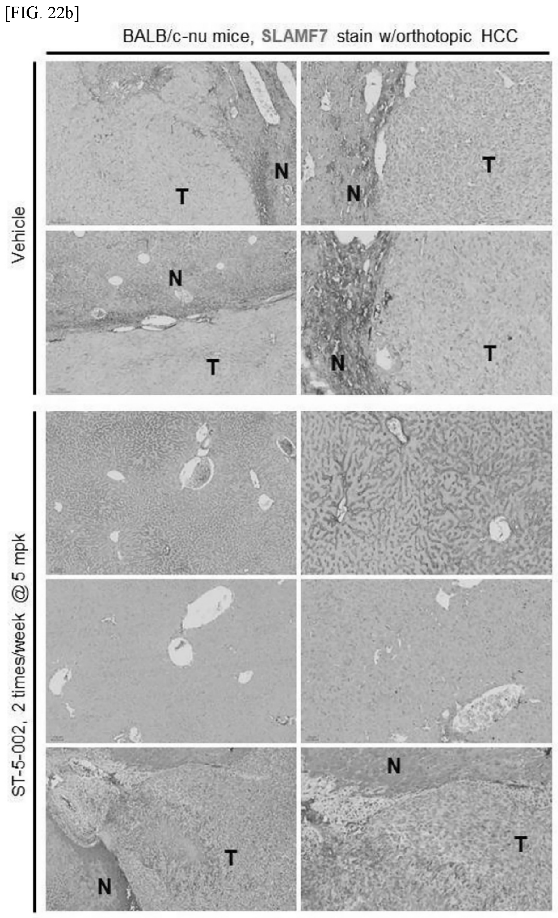

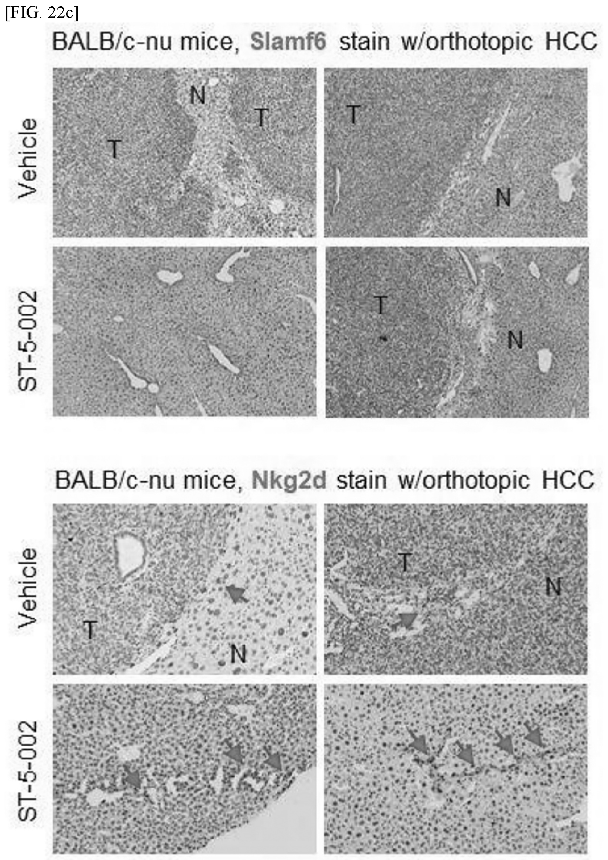

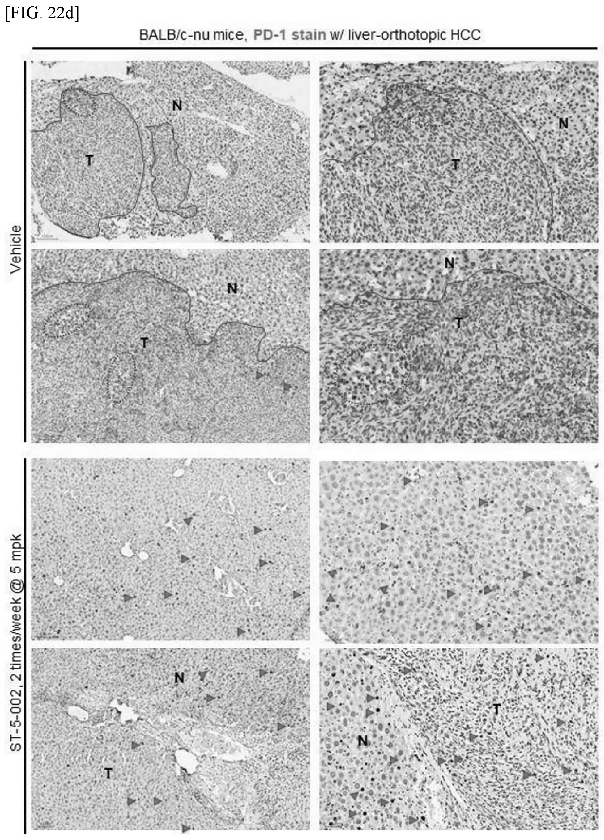

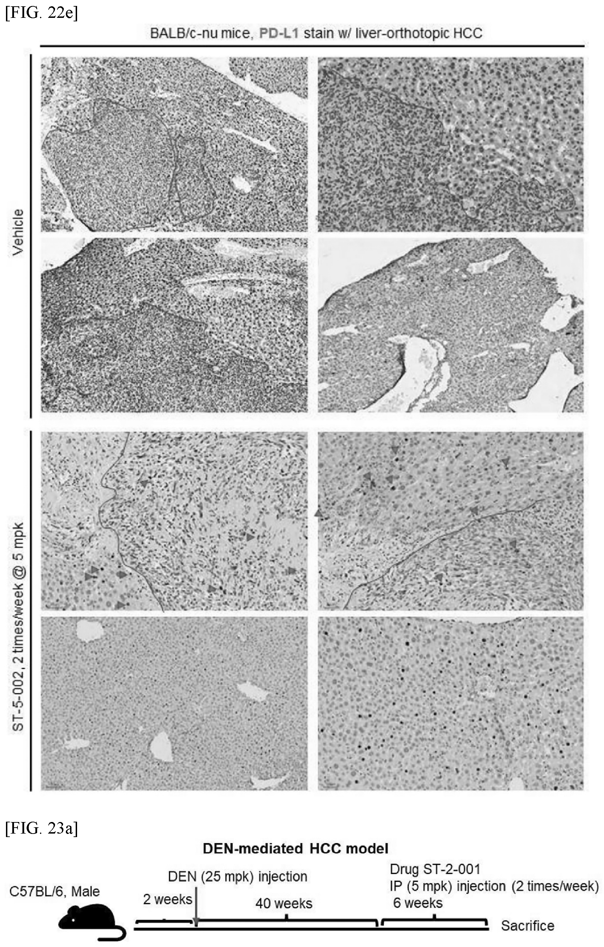

### 2.22. Confirmation of inhibition of liver cancer formation, regulation of expression of ligands related to natural killer function of NK cells, and infiltration of NK cells into cancer tissue in a liver-orthotopic xenograft model of a TM4SF5-expressing cell line of isoxazole compounds (FIG. 22)

(a-b) As in FIG. 20, a liver-orthotopic xenograft model was established by directly injecting 5x10⁵ SNU449_{T7} cells into the livers of 6-week-old male BALB/c-Nude mice (n=9), which are deficient in T and B cells but still capable of activating NK cell functions. Starting one week after the cell injection, the drug ST-5-002 was intraperitoneally injected at 5 mg/kg twice a week. (b-d) After the end of the experiment, for a portion of the obtained liver tissues, the expression level, distribution, and localization of SLAMF7, SLAMF6, NKG2D, PD-L1, and PD-1 factors, as well as the degree of liver cancer tissue, were confirmed through immunohistochemistry.

As a result, (a) is a schematic diagram of the liver-orthotopic xenograft experimental method for the TM4SF5-expressing SNU449_{T7} cell line. (b) As a result of performing immunohistochemistry for SLAMF7 on a portion of the recovered liver tissues after the end of the experiment, the expression of SLAMF7 was high in non-tumor areas and relatively low in tumor areas in the case where vehicle was treated. Meanwhile, when the drug ST-5-002 was treated, the expression of SLAMF7 was uniformly high or low across the tissue in areas where tumors did not form properly, and when the interface of small tumors that did form was observed, it was confirmed that cells highly expressing SLAMF7 infiltrated and were distributed or localized inside the tumor. It was presumed that the distribution (infiltration) of SLAMF7-expressing cells inside the tumor due to such drug treatment suggests the possibility of inducing activation of the natural killer ability of NK cells located inside the tumor. (c) Meanwhile, in the case of staining for SLAMF6 or NKG2D, the expression appeared higher in the non-tumor areas than in the tumor areas in the control group (vehicle treatment), but when ST-5-002 was treated, it was confirmed that the expression generally appeared spread out, or in the case of small cancerous areas, the degree of staining for SLAMF6 and NKG2D was further increased inside the tumor area. (d) Meanwhile, in the vehicle-treated control group, PD-1 stained cells (including NK cells) (red arrow heads) were seen within limited categories or regions (dotted circles) in the tumor area, but in the liver cancer tissue of the mouse treated with the drug ST-5-002, the distribution of PD-1-expressing cells was generally uniform in non-cancerous areas, and even at the boundary between tumor and non-tumor areas or in tumor areas, the infiltration of PD-1-expressing cells showed a degree of staining where they were scattered and distributed without restriction, and not confined to limited regions. (e) When performing staining for PD-L1 in liver-orthotopic liver cancer tissue, the staining of cells showing PD-L1 expression was localized only to limited partial regions (dotted red circles) inside the cancer tissue; however, in the case where the drug ST-5-002 was treated, when observing areas where tumors did not form properly or the interface of small tumors that did form, it was confirmed that cells highly expressing PD-L1 (red arrow heads) were scattered and distributed at a low level inside the tumor. That is, these results allow for the presumption that when carcinogenesis is induced because the drug is not treated, carcinogenesis occurs accompanied by a phenomenon of inhibiting the natural killer effect of NK cells through the action of TM4SF5-expressing hepatic epithelial cells; however, when the function of TM4SF5 is inhibited because the drug is treated, activation of NK cells is induced so that SLAMF7 cells can well infiltrate into the cancer site to cause inhibition of cancer, while cells expressing PD-L1 and PD-1 are located at a low level even throughout the tissue or inside small cancer sites, thereby weakening the significance of immune evasion.

Accordingly, these experimental results showed the efficacy of ST-5-002 in significantly inhibiting liver cancer formation in a liver cancer model obtained by directly transplanting cell-derived liver cancer tissues expressing TM4SF5 into the liver (FIG. 20). That is, these results indicate that when the drug is not treated, carcinogenesis is induced, and carcinogenesis occurs accompanied by a phenomenon of inhibiting the natural killer effect of NK cells (inhibition of expression or inactivation of killing efficacy activation ligands/receptors) through the action of TM4SF5-expressing hepatic epithelial cells; however, when the function of TM4SF5 is inhibited because the drug is treated, activation of NK cells is induced so that cells expressing SLAMF7, SLAMF6, and NKG2D infiltrate inside the cancer site to cause inhibition of cancer, and although cells expressing PD-L1 and PD-1 are distributed and located even inside the cancer site in small numbers, it could be presumed that the significance of immune evasion was weakened.

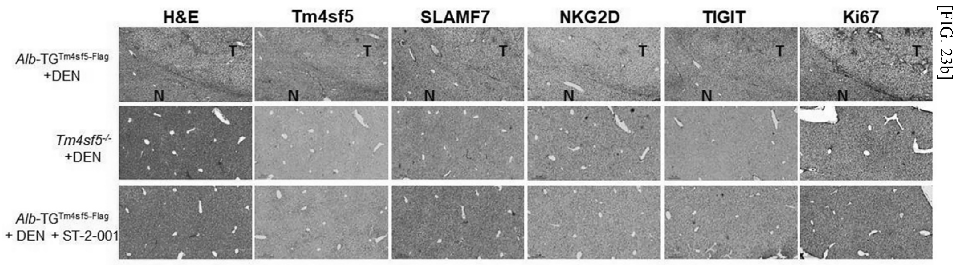

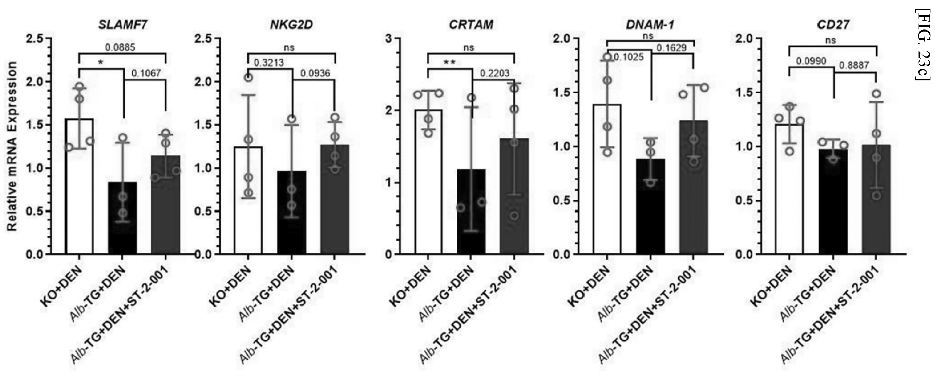

### 2.23. Confirmation of inhibition of liver cancer formation and regulation of expression of ligands related to natural killer function of NK cells upon DEN treatment in TM4SF5 transgenic animals by isoxazole compounds (FIG. 23)

In cases where a liver cancer model is induced by treating animals with diethylnitrosamine (DEN), liver cancer formation was induced by a single intraperitoneal injection (IP injection) of DEN into 2-week-old animals, followed by raising them for 46 weeks. Two-week-old male C57BL/6 wild-type, *Alb*-TG^{Tm4sf5-Flag} [animals in which Tm4sf5-Flag is excessively expressed only in hepatocytes by creating a transgenic animal linking Tm4sf5-Flag to the promoter region of the hepatocyte-specific *albumin* gene], or *Tm4sf5*^{*-*/*-*} Knockout (KO) mice were given a single IP injection of DEN (25 mg/kg body weight). After 40 weeks, the drug ST-2-001 treatment group was given IP injections at a dose of 5 mg/kg body weight (in 40% DMSO) twice a week for 6 weeks (n=5). Thereafter, the animals were sacrificed, and liver tissues were obtained and analyzed. A portion of the tissues was subjected to immunohistochemistry for the given factors, and another portion was subjected to qRT-PCR for measuring mRNA levels of the given factors.

As a result, (a) is a schematic diagram of inducing a liver cancer model by treating animals with DEN. (b) DEN was treated to *Alb*-TG^{Tm4sf5-Flag} or *Tm4sf5*^{*-*/*-*} KO mice, and some *Alb*-TG^{Tm4sf5-Flag} mice were treated with the drug ST-2-001 at a concentration of 5 mg/kg twice a week for the final 6 weeks. Upon obtaining and checking the liver tissues, cancer formation was significantly enhanced in the case of *Alb*-TG^{Tm4sf5-Flag} mice treated with DEN; however, in the groups where DEN and the drug ST-2-001 were treated to KO mice or *Alb*-TG^{Tm4sf5-Flag} mice, cancer formation was minimal or did not occur. When performing H&E staining or immunohistochemistry for TM4SF5, Ki67, and factors capable of regulating the natural killer function of NK cells (activators: SLAMF7 and NKG2D; inhibitor: TIGIT) on the liver cancer tissues, the expression of Tm4sf5 was high in both non-tumor and tumor areas, and the expressions of Ki67 and TIGIT in the tumor area were higher compared to the neighboring non-tumor area. However, the expressions of SLAMF7 and NKG2D were higher in the non-tumor area compared to the tumor area. (c) When checking the mRNA expression levels of ligands/receptors capable of regulating the natural killer function of NK cells by performing qRT-PCR using some tissues, the expressions of NK cell activating receptor factors such as SLAMF7, NKG2D, CRTAM, DNAM-1, and CD27 tended to decrease in *Alb*-TG^{Tm4sf5-Flag} mice compared to DEN-treated KO mice; however, a pattern was confirmed in which the decrease was partially recovered and increased by the treatment with the TM4SF5-specific inhibitor drug ST-2-001. This phenomenon was identified as a case where immune evasion occurred due to the induction of liver cancer expressing TM4SF5 by DEN treatment, accompanied by inactivation through inhibition of the expression of activating ligands/receptors such as SLAMF7 and NKG2D in the cancer site, as well as inhibition of the natural killer efficacy of NK cells according to the enhancement of TIGIT expression.

Accordingly, in the C57BL/6 liver cancer model in which liver cancer is caused by treating young mice with DEN to cause liver damage over a long period (46 weeks), the expression of ligand factors that can induce anti-cancer effects by increasing the activity of NK cells was low in the tumor, which allowed cancer to occur due to low NK cell activity; however, in cases where the isoxazole drug ST-2-001 was treated or in mice where the TM4SF5 gene was not expressed, the expression of such NK cell ligands was overall uniform (higher than the tumor area of the liver tissue not treated with the drug) without being distinguished by regions to the extent that tumors did not form. Therefore, the potential for anti-liver cancer inhibition through immune checkpoint inhibition, such as increasing the activity and uniform distribution of NK cells by the isoxazole drug, was presumed.

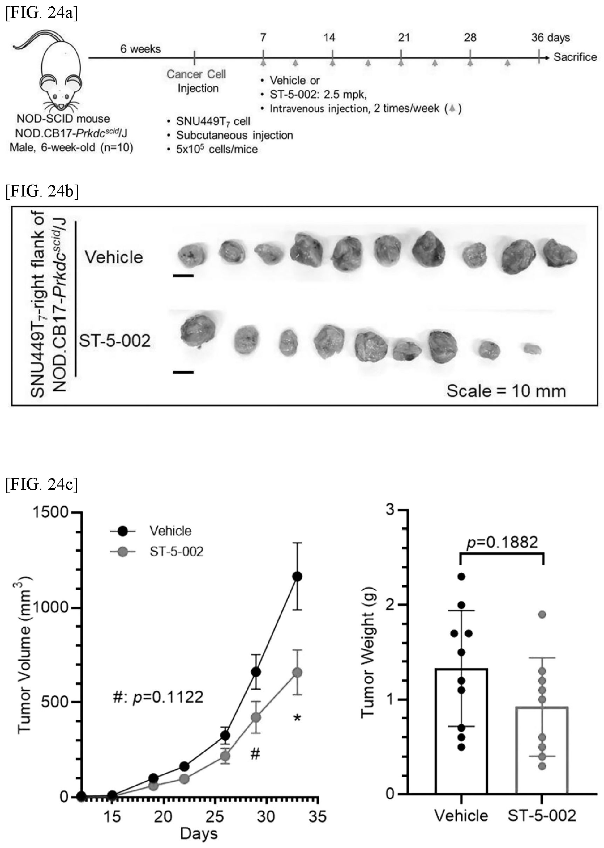

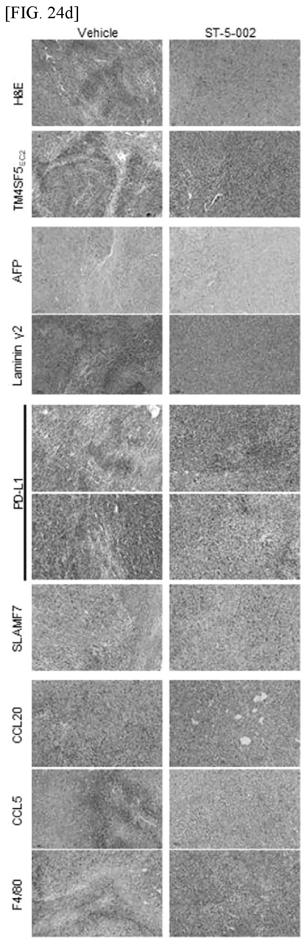

### 2.24. Confirmation of inhibition of liver cancer formation and regulation of expression of ligands related to natural killer function of NK cells in subcutaneous xenograft liver cancer model in severely combined immunodeficient animals (NOD-SCID mouse) by isoxazole compounds (FIG. 24)

SNU449_{T7} cells were subcutaneously injected into the thighs of 6-week-old male NOD/CB17-Prkdc^{scid}/J mice (NOD-SCID mice lacking or having non-functional immune cells such as T cells, B cells, and NK cells, n=10), at a dose of 5X10⁵ cells. One week later, vehicle or the drug ST-5-002 was intravenously injected at a concentration of 2.5 mg/kg of body weight (mpk) twice a week. Three days after the 8th drug injection, the animals were sacrificed, cancer tissues were recovered and photographed, and immunohistochemistry, Western blot, and qRT-PCR analyses were performed.

As a result, (a) is a schematic diagram of the subcutaneous xenograft liver cancer model construction and evaluation of drug anti-cancer efficacy in severely combined immunodeficient (NOD-SCID) animals. (b) When comparing the sizes after isolating the tumors, it was confirmed that the tumor size decreased when the drug ST-5-002 was treated alone compared to the case where vehicle was treated. (c) When comparing the sizes of the cancer tissues between the vehicle-treated control group and the drug-treated group, the drug treatment showed statistically significant inhibitory efficacy in cancer formation in terms of tumor volume and weight. (d) When H&E staining and immunostaining for TM4SF5 were performed, in the case of vehicle treatment, not only cancer formation but also a disordered arrangement of cells and characteristics of invasive fibrotic/cirrhotic scar formation were observed in the liver tissue; however, in the case where the drug ST-5-002 was treated, while the expression of TM4SF5 did not change significantly, the composition of cells within the tissue appeared more orderly and normal. Meanwhile, high expressions of AFP and laminin γ2, which are known to be highly expressed in liver cancer, were confirmed, but were significantly reduced by the drug treatment. The degree of PD-L1 staining decreased in the case of drug treatment compared to the case of vehicle treatment. In the case of SLAMF7, the drug treatment showed a much more distinctly strong staining level in the small cancer areas that formed compared to the surrounding areas, whereas such a feature was weak in the case of vehicle treatment. Furthermore, the staining level of the macrophage marker F4/80 and the expressions of inflammation-related chemokines CCL5 and CCL20 were lower in the drug-treated group compared to the control group.

Accordingly, as the subcutaneous xenograft liver cancer formation model of the TM4SF5-expressing cell line was implemented in NOD-SCID mice lacking immune cells, it was presumed that the anti-cancer effect could be achieved even without the drug acting on immune cells. This effect is thought to occur solely through mechanisms such as inhibiting the signaling activity, survival, and proliferation of TM4SF5 present in liver cancer cells, inhibiting protein-protein binding with membrane proteins and membrane receptors for such cellular functions, lowering the expression of PD-L1, AFP, and laminin γ2 in cancer cells, and regulating the expression level of SLAMF7 to increase.

Meanwhile, the PCR primer sequence information for the genes used in the present invention can be referred to in the following.

**[Table 2]**

| Gene name | Forward sequence (5'- 3') | Reverse Sequence (5'- 3') |
|---|---|---|
| Mouse gene | | |
| *Trem2* | CAGTTTCTCCTGCTGCTGAT | CAGTGCTTCAAGGCGTCATA |
| *Timd4* | GTGTTATTGTTTCTGGCGTTTCT | TGAGGACGCTGTCACTATCT |
| *Clec4f* | CTATCCAGAGGCTTAGGGACTA T | GCAACTGCACCAGAGAACTA |
| *Acc* | ACATTCCGAGCAAGGGATAAG | GGGATGGCAGTAAGGTCAAA |
| *Dgat2* | GCTGATCTGGTTCCCACTTATT | GGAACTTCTTCTGGACCCATC |
| *Fasn* | AGACCCGAACTCCAAGTTATTC | GCAGCTCCTTGTATACTTCTCC |
| *Slamf7* | CATCGAGTCAGGCTTCCTTAAT | CTTGTTGCTTTGCCGATCTAT G |
| *Nkg2d* | GATGGCTCCTCTCTCTCATACA | GCCTTAAAGCTTGAGCCATA GA |
| *Crtam* | CAGCTACACAACTCTCCATTAG T | ACTCTCACTTGCTTCGTCTTC |
| *Dnam-1(CD226)* | AGACGGAGACAGGTGAGAAT | TAGACTGGATGGGAGAAGTA GG |
| *CD27* | AGAGTGTGACCCTCCTCTAAA | CTTCTCTGTGCCATGAGGTAA G |
| *Tm4sf5* | CGAATTGGACCCAAATGCTTAA T | CGCCTCACACAAATTCCAAA G |
| *Gapdh* | GTGGCAAAGTGGAGATTGTTG | CGTTGAATTTGCCGTGAGTG |
| | | |

| Human gene | | |
|---|---|---|
| *Cadm1* | GCTTCTGCTGTTGCTCTTCT | CTCGATCACTGTCACGTCTTT C |
| *Ulbp1* | TTTCCTTAAAGGGCAACTGCT | AGGAACTGCCAAGATCCTCT |
| *Vcam1* | GGAGCTCTACTCATTCCCTAGA | CTAGGAACCTTGCAGCTTAC A |
| *Afp* | GCTGACCTGGCTACCATATTT | TGTTCATCTCCAGTGGGTTTC |
| *Pd-l1* | CAGCAACCAGACGGACAA | TGACTTCCACATGAGCGTG |
| *Tigit* | TCTATCACACCTACCCTGATGG | AGATTCCATTGCTTGGAGCC |
| *Mica* | CCTTGGCCATGAACGTCAGG | CCTCTGAGGCCTCGCTGCG |
| *Micb* | ACCTTGGCTATGAACGTCACA | CCCTCTGAGACCTCGCTGCA |
| *Tm4sf5* | CTTGCTCAACCGCACTCTAT | ATCCCACACAGTACTATCTCC A |
| *Lamc2* | CTCAGGAGGCCACAAGATTAG | TGAGAGGGCTTGTTTGGAAT AG |
| *Col1a1* | CAGACTGGCAACCTCAAGAA | CAGTGACGCTGTAGGTGAAG |
| *Ccl2* | TCATAGCAGCCACCTTCATTC | CTCTGCACTGAGATCTTCCTA TTG |
| *Ccl5* | GCTGTCATCCTCATTGCTACT | CACTTGCCACTGGTGTAGAA |
| *Ccl20* | TTGATGTCAGTGCTGCTACTC | CCGTGTGAAGCCCACAATA |
| *Tnf-a* | AGAGGGAGAGAAGCAACTACA | GGGTCAGTATGTGAGAGGAA GA |
| *Il-8* | ATGACTTCCAAGCTGGCCGTG GCT | TCTCAGCCCTCTTCAAAAACT TCTC |
| *Cxcl1* | CGAAGTCATAGCCACACTCAA | GATTTGTCACTGTTCAGCATC TT |
| *Cxcl2* | TCACCTCAAGAACATCCAAAG T | CAAGCTTTCTGCCCATTCTTG |
| *Cxcl3* | GCAGGGAATTCACCTCAAGAA | TGTGGCTATGACTTCGGTTTG |
| *Cxcl5* | TCTGCAAGTGTTCGCCATAG | GGGCTTCTGGATCAAGACAA A |
| *Cxcl6* | GCTGCGTTGCACTTGTTT | CAAACTTGCTTCCCGTTCTTC |
| *Cxcl10* | GGCCATAGGGAAGCTTGAAA | CAGACATCTCTGCTCATCATT CT |
| *Gapdh* | GGTGTGAACCATGAGAAGTAT GA | GAGTCCTTCCACGATACCAA AG |

Hereinafter, the present invention will be described in detail with examples to facilitate understanding. However, the following examples are intended to illustrate the present invention, and the scope of the present invention is not limited thereto. The examples of the present invention are provided to more completely explain the present invention to those of skill in the art.

## Claims

1. A compound represented by Chemical Formula 1 below, a pharmaceutically acceptable salt thereof, a hydrate thereof or a solvate thereof: 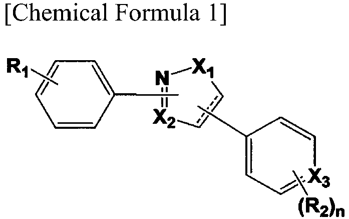 wherein in the Chemical Formula 1,
X₁ is O or N;
X₂ is C or N;
X₃ is C or N;
- - - - - - is single bond or double bond;
R₁ is hydrogen, RₐNH- or RₐO-;
Rₐ is hydrogen, C₁₋₅ alkyl, R_{b}SO₂- or R_{b}CO-;
each R_{b} is independently hydrogen, C₁₋₅ alkyl, C₂₋₅ alkenyl or C₆₋₁₀ aryl unsubstituted or substituted with C₁₋₅ alkyl;
R₂ is hydrogen, halogen, C₁₋₅ alkyl, C₁₋₆ haloalkyl, R_{c}NH- or R_{c}O-;
R_{c} is hydrogen, C₁₋₅ alkyl, R_{d}SO₂- or R_{d}CO-;
each Rₐ is independently hydrogen, C₁₋₅ alkyl or C₆₋₁₀ aryl unsubstituted or substituted with C₁₋₅ alkyl; and
n is 1 or 2.

2. The compound represented by Chemical Formula 1 of claim 1, wherein the compound is represented by any one of Chemical Formulas 2 to 4 below: 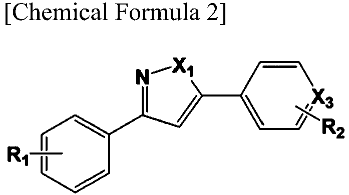 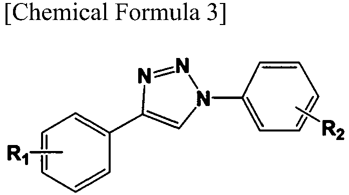 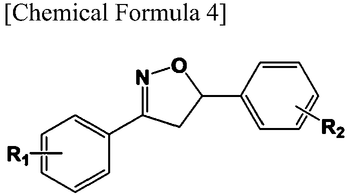 wherein in the Chemical Formulas 2 to 4,
X₁, X₃, R₁, Rₐ, R_{b}, R₂, R_{c}, R_{d} and n are as defined in Chemical Formula 1 of claim 1.

3. The compound of claim 1, wherein the compound is selected from the group consisting of the following compounds:
4-(5-(4-methoxyphenyl)isoxazole-3-yl)aniline;
4-(5-(3-methoxyphenyl)isoxazole-3-yl)aniline;
4-(5-(2-methoxyphenyl)isoxazole-3-yl)aniline;
4-(5-(3,4-dimethoxyphenyl)isoxazole-3-yl)aniline;
4-(5-(4-(tert-butyl)phenyl)isoxazole-3-yl)aniline;
4-(5-(4-fluorophenyl)isoxazole-3-yl)aniline;
4-(5-(4-(trifluoromethyl)phenyl)isoxazole-3-yl)aniline;
4-(5-(4-propoxyphenyl)isoxazole-3-yl)aniline;
3-(5-(4-methoxyphenyl)isoxazole-3-yl)aniline;
*N*-(4-(5-(4-methoxyphenyl)isoxazole-3-yl)phenyl)acetamide;
*N*-(4-(5-(4-methoxyphenyl)isoxazole-3-yl)phenyl)methanesulfonamide;
*N*-(4-(5-(4-methoxyphenyl)isoxazole-3-yl)phenyl)-4-methylbenzenesulfonamide;
*N*-(4-(5-(4-hydroxyphenyl)isoxazole-3-yl)phenyl)-4-methylbenzenesulfonamide;
*N*-(4-(5-(4-methoxyphenyl)isoxazole-3-yl)phenyl)-4-methylbenzamide;
*N*-(4-(5-(4-methoxyphenyl)isoxazole-3-yl)phenyl)acrylamide;
*N*-(4-(5-(3-methoxyphenyl)isoxazole-3-yl)phenyl)acetamide;
*N*-(4-(5-(2-methoxyphenyl)isoxazole-3-yl)phenyl)acetamide;
*N*-(4-(5-(3,4-dimethoxyphenyl)isoxazole-3-yl)phenyl)acetamide;
*N*-(4-(5-(4-propoxyphenyl)isoxazole-3-yl)phenyl)acetamide;
*N*-(4-(5-(4-(trifluoromethyl)phenyl)isoxazole-3-yl)phenyl)acetamide;
*N*-(4-(5-(4-fluorophenyl)isoxazole-3-yl)phenyl)acetamide;
*N*-(4-(5-(4-(tert-butyl)phenyl)isoxazole-3-yl)phenyl)acetamide;
*N*-(4-(5-(pyridine-4-yl)isoxazole-3-yl)phenyl)acetamide;
*N*-(3-(5-(4-methoxyphenyl)isoxazole-3-yl)phenyl)acetamide;
*N*-(3-(5-(4-methoxyphenyl)isoxazole-3-yl)phenyl)-4-methylbenzamide;
*N*-(4-(5-(4-hydroxyphenyl)isoxazole-3-yl)phenyl)acetamide;
4-(3-(4-acetamidophenyl)isoxazole-5-yl)phenyl 4-methylbenzenesulfonate;
*N*-(4-(3-(4-methoxyphenyl)isoxazole-5-yl)phenyl)acetamide;
*N*-(4-(3-(4-methoxyphenyl)isoxazole-5-yl)phenyl)-4-methylbenzenesulfonamide;
*N*-(4-(3-(4-hydroxyphenyl)isoxazole-5-yl)phenyl)-4-methylbenzenesulfonamide;
*N*-(4-(3-(4-hydroxyphenyl)isoxazole-5-yl)phenyl)acetamide;
*N*-(4-(3-(4-hydroxyphenyl)isoxazole-5-yl)phenyl)-4-methylbenzamide;
*N-(4-(1-(4-hydroxyphenyl)-1H-1,2,3-triazole-4-yl)phenyl)-4-*methylbenzenesulfonamide;
*N*-(4-(1-(4-methoxyphenyl)-1*H*-1,2,3-triazole-4-yl)phenyl)acetamide;
*N*-(4-(5-(4-hydroxyphenyl)-1*H*-pyrazol-3-yl)phenyl)-4-methylbenzenesulfonamide;
*N*-(4-(5-(4-hydroxyphenyl)-4,5-dihydroisoxazole-3-yl)phenyl)-4-methylbenzenesulfonamide; and
*N*-(4-(5-(4-methoxyphenyl)-4,5-dihydroisoxazole-3-yl)phenyl)acetamide.

4. A pharmaceutical composition for preventing or treating a liver disease, comprising the compound of any one of claims 1 to 3 or the pharmaceutically acceptable salt thereof.

5. The pharmaceutical composition of claim 4, wherein the liver disease is liver cancer, chronic liver injury, non-alcoholic or alcoholic liver disease or fatty liver disease, hepatitis, liver fibrosis, or liver cirrhosis.

6. The pharmaceutical composition of claim 4, wherein the pharmaceutical composition is for use in treating liver disease by inhibiting expression, protein-protein binding, or signal transduction activation of TM4SF5 protein, or by inhibiting immune checkpoint function by activating a killing activity or controlling the distribution of T and NK immune cells.

7. A pharmaceutical formulation comprising the pharmaceutical composition of claim 4.

8. The pharmaceutical formulation of claim 7, wherein the pharmaceutical formulation is in the form of a patch, ointment, inhalant, nasal drop, tablet, pill, powder, capsule, syrup, or emulsion.

9. The pharmaceutical formulation of claim 7, further comprising at least one selected from the group consisting of a pharmaceutically acceptable carrier, an adjuvant, and an excipient.

10. A method for inhibiting expression, protein-protein binding, or signal transduction activation of TM4SF5 protein, or for inhibiting immune checkpoint function by activating a killing activity or controlling the distribution of T and NK immune cells in a subject or a cell,
comprising administering to the subject a pharmaceutically effective amount of the compound of any one of claims 1 to 3.

11. A method for preventing or treating liver disease in a subject,
comprising administering to the subject a pharmaceutically effective amount of the compound of any one of claims 1 to 3.

12. The method of claim 11, wherein the liver disease is liver cancer, chronic liver injury, non-alcoholic or alcoholic liver disease or fatty liver disease, hepatitis, liver fibrosis, or liver cirrhosis.

13. Use of the compound of any one of claims 1 to 3 or a pharmaceutically acceptable salt thereof for preventing or treating liver disease.

14. The use of claim 13, wherein the liver disease is liver cancer, chronic liver injury, non-alcoholic or alcoholic liver disease or fatty liver disease, hepatitis, liver fibrosis, or liver cirrhosis.
